# EUROPEAN PATENT APPLICATION

(11) **EP 4 781 998 A2**
(43) Date of publication of application: **29.07.2026**
(21) Application number: 25222129.6
(22) Date of filing: 21.02.2023
(51) Int. Cl.: A61K 38/00

(54) **CRF2 RECEPTOR AGONISTS AND THEIR USE IN THERAPY**

(30) Priority: 23.02.2022 EP 22158188
(62) Divisional of application: 23705580.1
(71) Applicant: Corteria Pharmaceuticals, 75008 Paris (FR)
(72) Inventor: JANIAK, Philip, 75008 Paris (FR); OZOUX, Marie-Laure, 75008 Paris (FR); ROVERSI, Daniela, 00071 Pomezia (IT); BIANCHI, Elisabetta, 00071 Pomezia (IT); TRIPEPI, Martina, 00071 Pomezia (IT); SANTOPRETE, Alessia, 00071 Pomezia (IT)
(74) Representative: Arnold & Siedsma

(57) **Abstract**

The present disclosure provides compounds which are peptides comprising the amino acid sequence of the formula (I) disclosed herein or pharmaceutically acceptable salts thereof. The compounds act as agonists of the corticotropin-releasing factor receptor 2 (CRF2) and are useful in therapy, especially in the treatment or prevention of cardiovascular diseases, kidney disease, obesity, diabetes, sarcopenia, particularly obesity-linked sarcopenia, heart failure, cachexia, and pulmonary hypertension.

## Description

### FIELD

The present disclosure relates to compounds which are agonists of the corticotropin-releasing factor receptor 2 (CRF2) and their use in therapy, especially in the treatment or prevention of cardiovascular diseases, obesity, diabetes, sarcopenia, particularly obesity-linked sarcopenia, cachexia, kidney disease, heart failure and pulmonary hypertension.

### BACKGROUND

Urocortins (UCNs) are endogenous peptides which act through corticotropin-releasing factor (CRF) receptors, which are type 2 G protein-coupled receptors (GPCRs). The CRF receptor family comprises CRF1 receptors, which are encoded by the CRHR1 gene, and CRF2 receptors, which are encoded by the CRHR2 gene.

There are three known endogenous urocortins found in mammals: UCN1, UCN2 and UCN3. Despite a high degree of sequence homology, the binding of these peptides to CRF1 and CRF2 is different. CRF1 and CRF2 are activated non-selectively by Corticotropin Releasing Hormone (CRH) and UCN1, whereas UCN2 and UCN3 are CRF2-selective agonists. In particular, UCN2 is a thirty-eight amino acid peptide which selectively activates the CRF2 receptor, including the known isoforms CRF2-alpha (α), - beta (β) and -gamma (y).

Urocortins and their receptors are involved in neurohumoral responses to various stress and pathological situations. In particular, urocortins evoke positive hemodynamic effects in both preclinical experimental heart failure models and patients with heart failure or hypertension. Urocortins and CRF receptors are expressed in the heart, the kidney and in blood vessels, with CRF2 expressed robustly and CRF1 expressed minimally if at all (see Waser et al., Peptides, 2006, 27, 3029-3038). In experimental models, urocortins acting via CRF2 activation have been shown to improve cardiovascular function via the reduction of vascular resistance, improvement in kidney function and cardiac inotropic and lusitropic actions. In clinical studies, UCN2 and UCN3 have been shown to have direct vasorelaxant actions in healthy volunteers and in patients with heart failure (see Stirrat et al., Br. J. Clin. Pharmacol., 2016, 82, 974-982), while UCN2 has also been shown to increase cardiac output and reduce vascular resistance in patients with heart failure (see Davis et al., Eur. Heart J., 2007, 28, 2589-2597; and Chan et al., JACC: Heart Failure, 2013, 1, 433-441). A recombinant acetate salt of UCN3 has been shown to improve cardiac output and reduce vascular resistance in a multicentre study of patients with chronic stable heart failure (see Gheorghiade et al., Eur. J. Heart Fail., 2013, 15, 679-89).

Recent studies have demonstrated that UCN2 and/or UCN3 gene transfer in mice improves not only cardiac function but also glucose disposal (see Giamouridis et al., JACC: Basic to Translational Science, 2018, 3, 2). In addition, subcutaneous delivery of pegylated UCN2 has been shown to improve glucose tolerance and increase glucose uptake in skeletal muscle while reducing body weight (see Borg et al., Diabetes, 2019, 1403-1414). These findings suggest that urocortins may be useful not only in the treatment of cardiovascular diseases but also in the treatment of diseases such as diabetes and obesity.

Nevertheless, the extremely short half-life of urocortins remains a major limitation to their therapeutic use (see Davies et al., JACC, 2007, 49, 461-471). To date, treatment with urocortins cannot be sustained without continuous intravenous infusion. However, the management of chronic diabetic or heart failure patients requires treatment suitable for long-standing and home self-administration.

In an attempt to overcome such limitations, various analogs of urocortins have been proposed. For instance, WO2018013803 (Alsina-Fernandez; Eli Lilly and Company) and WO2022038179 discloses analogs of UCN2 which are taught as having utility in the treatment of diseases such as chronic kidney disease and type II diabetes.

However, there remains a need for improved CRF2 receptor agonists which are useful as therapeutic agents, especially in the treatment and prevention of cardiovascular diseases, obesity and diabetes. In particular, there is a need for selective CRF2 agonists having desirable efficacy, pharmacokinetic properties (e.g., improved half-life) and/or physicochemical properties (e.g., improved stability and/or solubility), extending the possible applications to sarcopenia, kidney disease, particularly obesity-linked sarcopenia, cachexia, heart failure and pulmonary hypertension.

### SUMMARY

In a first aspect, the present disclosure provides a compound which is a peptide comprising the amino acid sequence of formula (I) (SEQ ID NO: 1):

X1-X2-X3-X4-X5-X6-X7-X8-X9-X10-X11-X12-X13-X14-X15-X16-X17-X18-X19- X20-X21-X22-X23-X24-X25-X26-X27-X28-X29-X30-X31-X32-X33-X34-X35- X36-X37-X38 (I)

Wherein
X1 is absent or isoleucine (I); with Isoleucine in L or D configuration
X2 is valine (V); with valine in L or D configuration
X3 is leucine (L)
X4 is serine (S)
X5 is leucine (L)
X6 is aspartate (D)
X7 is valine (V) or D-valine (v)
X8 is proline (P)
X9 is isoleucine (I)
X10 is lysine (K), alanine (A) or glycine (G); in particular Lysine (K)
X11 is leucine (L); isoleucine (I), alpha-methyl-leucine (αMeL) or 2-aminoisobutyric acid (Aib)
X12 is lysine (K), wherein the epsilon-amino group of the lysine side chain is covalently bound to an albumin-binding moiety (herein also denoted as (K*))
X13 is lysine (K), glutamine (Q) or alanine (A); in particular lysine (K) or glutamine (Q); more in particular lysine (K);
X14 is isoleucine (I) or 2-aminoisobutyric acid (Aib)
X15 is leucine (L), alpha-methyl-leucine (αMeL) or 2-aminoisobutyric acid (Aib) X16 is leucine (L)
X17 is glutamate (E) or lysine (K)
X18 is glutamine (Q)
X19 is glutamate (E)
X20 is lysine (K), arginine (R) or alanine; in particular lysine (K) or arginine (R); X21 is glutamine (Q)
X22 is lysine (K) or alanine (A); in particular alanine (A);
X23 is lysine (K), alanine (A), glutamate (E) or 2-aminoisobutyric acid (Aib); in particular lysine (K), alanine (A), or 2-aminoisobutyric acid (Aib);
X24 is glutamine (Q)
X25 is arginine (R), alanine (A), alpha-methyl-leucine (αMeL) or 2-aminoisobutyric acid (Aib); in particular arginine (R), alpha-methyl-leucine (αMeL) or 2-aminoisobutyric acid (Aib);
X26 is glutamate (E), or 2-aminoisobutyric acid (Aib);
X27 is glutamine (Q)
X28 is alanine (A)
X29 is glutamate (E)
X30 is lysine (K) or threonine (T);
X31 asparagine (N), glutamine (Q) or alanine (A);
X32 is lysine (K), valine (V), threonine (T), 2-aminoisobutyric acid (Aib), glutamate (E), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx);
X33 is glutamine (Q)
X34 is isoleucine (I)
X35 is leucine (L)
X36 is alanine (A) or glutamate (E);
X37 is glutamine (Q)
X38 is valine
and wherein
   when X1 is isoleucine, then X7 is D-valine, and X32 is selected from valine (V), threonine (T), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); and said isoleucine is optionally N-terminally acetylated or N-methylated; or a pharmaceutically acceptable salt thereof.

In a second aspect of the disclosure, there is provided a pharmaceutical composition comprising a compound of the disclosure and a pharmaceutically acceptable excipient, diluent or carrier.

The compounds and pharmaceutical compositions disclosed herein are useful in therapy and may be used in the treatment or prevention of various diseases through agonism of the CRF2 receptor. Thus, in other aspects, the disclosure is directed to the use of the compounds and pharmaceutical compositions in therapy, especially in the treatment or prevention of cardiovascular diseases, obesity, diabetes, kidney disease, sarcopenia, particularly obesity-linked sarcopenia, cachexia, heart failure and pulmonary hypertension.

### DETAILED DESCRIPTION

The present disclosure relates to compounds which are derivatives of UCN2 and which are useful as CRF2 receptor agonists. The compounds exhibit desirable activity and selectivity at the CRF2 receptor, as well as improved pharmacokinetic properties and beneficial *in vivo* effects in relevant animal models. In addition, the compounds exhibit desirable physicochemical properties, such as desirable solubility and stability, making them excellent candidates for solution formulations for subcutaneous delivery. The compounds are useful in therapy, especially in the treatment or prevention of cardiovascular diseases, kidney disease, obesity, diabetes, sarcopenia, particularly obesity-linked sarcopenia, cachexia, heart failure and pulmonary hypertension.

The desired properties of the UCN2 analogues disclosed in WO2022038179, in particular enabling once daily administration, were found to be further improved by N-terminal modification (N-methylation, acetylation or N-terminal truncation with preferably acylation or N-methylation on Val 2) of said UCN2 like peptides when combined with further modifications at position 32 as evident from the data hereinafter, shown to be key for the chemical stability of the peptides. Preferably these N-terminal modifications and modifications at position 32 are further combined with a D-valine at position 7 and further changes to lower the isoelectric point below 7 to improve the solubility of the peptides at neutral pH. In particular In case the isoelectric point of the sequence is close to 7, mutations were introduced at one or more positions where basic amino acids are present, such as in particular the positions 10, 13, 20, 22, 23 and 25 with the benefits of a) lowering the isoelectric point below 6 and improving solubility at neutral pH and b) at the same time surprisingly reduce the undesired mast cell degranulation effect resulting in pseudo-allergic reactions often observed with peptides comprising basic amino acids.

### Compounds

The compounds of the disclosure are peptides comprising the amino acid sequence of the formula (I) recited above or pharmaceutically acceptable salts of said peptides.

In the present disclosure, amino acids are referred to by their name, their commonly known three-letter symbols or by the one-letter symbols recommended by the IUPAC-IUB Biochemical Nomenclature Commission. Generally accepted three-letter codes for other amino acids, such as Aib for 2-aminoisobutyric acid, may also be used. Unless otherwise indicated, all amino acids employed in the compounds of the disclosure are L-amino acids. Thus, for example, L-valine is referred to as "valine", "V" or "Val", whereas D-valine is specifically identified as such, and may be represented "v" or "D-val". In one letter symbols the D-amino acids are represented with small caps, with for example isoleucine being referred to as "i", valine being referred to as "v", serine being referred to as "s", leucine being referred to as "I", etc .

In an embodiment the present invention provides a compound which is a peptide comprising the amino acid sequence of formula (I) above; wherein;
X1 is absent or isoleucine (I); with Isoleucine in L or D configuration
X2 is valine (V); with valine in L or D configuration;
X3 is leucine (L)
X4 is serine (S)
X5 is leucine (L)
X6 is aspartate (D);
X7 is valine (V) or D-valine (v);
X8 is proline (P)
X9 is isoleucine (I)
X10 is lysine (K)
X11 is leucine (L); isoleucine (I), alpha-methyl-leucine (αMeL) or 2-aminoisobutyric acid (Aib);
X12 is lysine (K), wherein the epsilon-amino group of the lysine side chain is covalently bound to an albumin-binding moiety (K*);
X13 is lysine (K) or glutamine (Q);
X14 is isoleucine (I) or 2-aminoisobutyric acid (Aib);
X15 is leucine (L), alpha-methyl-leucine (αMeL) or 2-aminoisobutyric acid (Aib);
X16 is leucine (L)
X17 is glutamate (E) or lysine (K);
X18 is glutamine (Q)
X19 is glutamate (E)
X20 is lysine (K) or arginine (R);
X21 is glutamine (Q)
X22 is lysine (K)
X23 is lysine (K), alanine (A), glutamate (E) or 2-aminoisobutyric acid (Aib);
X24 is glutamine (Q)
X25 is arginine (R), alpha-methyl-leucine (αMeL) or 2-aminoisobutyric acid (Aib);
X26 is glutamate (E), or 2-aminoisobutyric acid (Aib);
X27 is glutamine (Q)
X28 is alanine (A)
X29 is glutamate (E)
X30 is lysine (K) or threonine (T);
X31 asparagine (N), glutamine (Q) or alanine (A);
X32 is lysine (K), valine (V), threonine (T), 2-aminoisobutyric acid (Aib), glutamate (E), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx);
X33 is glutamine (Q)
X34 is isoleucine (I)
X35 is leucine (L)
X36 is alanine (A) or glutamate (E);
X37 is glutamine (Q)
X38 is valine
and wherein;
   when X1 is isoleucine, then X7 is D-valine, and X32 is selected from valine (V), threonine (T), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); and said isoleucine is optionally N-terminally acetylated or N-methylated; in particular N-terminally acetylated; or a pharmaceutically acceptable salt thereof.

The aforementioned proviso on the selection of X32 when X1 is isoleucine, and X7 is D-valine, is applicable throughout the different embodiments herein disclosed, but for the independent embodiments hereinafter. In an embodiment of the aforementioned proviso X1 is an N-terminally acetylated or N-methylated isoleucine. In a particular embodiment of the aforementioned proviso X1 is an N-terminally acetylated isoleucine.

In an embodiment when X1 is absent, X2 is valine with valine in L or D configuration, in particular valine in L configuration (V); with said valine optionally N-terminally acylated or alkylated (e.g N-methylated). In particular when X1 is absent, X2 is valine said valine being N-terminally acylated with a C1-20alkanoyl group; more in particular said valine being N-terminally acylated with a C1-10alkanoyl group; even more in particular said valine being N-terminally acylated with an alkanoyl group selected from ethanoyl, methanoyl, propanoyl, butanoyl, pentanoyl hexanoyl, heptanoyl, decanoyl, dodecanoyl, or tetradecanoyl; more in particular selected from ethanoyl, methanoyl, propanoyl, butanoyl, pentanoyl hexanoyl or heptanoyl.

In a particular embodiment when X1 is absent, X2 is valine with valine in L or D configuration, in particular valine in L configuration (V); with said valine being N-terminally acylated or alkylated (preferably with an alkanoyl group as mentioned herein before); and X32 is selected from valine (V), threonine (T), 2-aminoisobutyric acid (Aib), alanine (A), glutamate (E), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx).

In an embodiment when X1 is absent, X2 is valine with valine in L or D configuration, in particular valine in L configuration (V); with said valine being N-terminally acylated or alkylated (preferably with an alkanoyl group as mentioned herein before); and X32 is selected from valine (V), threonine (T), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx).

In another particular embodiment when X1 is absent, X2 is valine with valine in L or D configuration, in particular valine in L configuration (V); with said valine being N-terminally alkylated or N-terminally acylated with a C1-20alkanoyl group; more in particular said valine being N-terminally acylated with a C1-10alkanoyl group; even more in particular said valine being N-terminally acylated with an alkanoyl group selected from from ethanoyl, methanoyl, propanoyl, butanoyl, pentanoyl hexanoyl or heptanoyl; and X32 is selected from valine (V), threonine (T), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx).

In an embodiment, X7 is D-valine (v).

In an embodiment, X11 alpha-methyl-leucine (αMeL) or 2-aminoisobutyric acid (Aib); in particular alpha-methyl-leucine (αMeL).

In an embodiment, X15 is alpha-methyl-leucine (αMeL) or 2-aminoisobutyric acid (Aib).

In an embodiment, X23 is alanine (A), glutamate (E) or 2-aminoisobutyric acid (Aib).

In an embodiment, X25 is alpha-methyl-leucine (αMeL) or 2-aminoisobutyric acid (Aib); in particular alpha-methyl-leucine (αMeL).

In an embodiment, X31 is glutamine (Q).

In an embodiment, X32 is selected from valine (V), threonine (T), 2-aminoisobutyric acid (Aib), alanine (A), glutamate (E), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); in particular X32 is selected from valine (V), threonine (T), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); with the proviso that when X1 is isoleucine, then X7 is D-valine, and X32 is selected from valine (V), threonine (T), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); in particular 2-aminoisobutyric acid (Aib), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx), and said isoleucine is optionally N-terminally acetylated or N-methylated.

In a more particular embodiment X32 is selected from 2-aminoisobutyric acid (Aib), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); even more in particular X32 is selected from 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex) .
- In an embodiment, the amino acid residue at X38 is amidated.

In an independent embodiment, the present invention provides a peptide comprising the amino acid sequence of formula (I) as defined above, wherein at least one of X10, X11, X13, X15, X20, X22, X23, X25, X31 and X32 is selected as;
- X10 being alanine (A)or glycine (G);
- X11 being isoleucine (I), alpha-methyl-leucine (αMeL) or 2-aminoisobutyric acid (Aib); in particular alpha-methyl-leucine (αMeL) or 2-aminoisobutyric acid (Aib); more in particular alpha-methyl-leucine (αMeL);
- X13 being glutamine (Q) or alanine (A);
- X15 being alpha-methyl-leucine (αMeL) or 2-aminoisobutyric acid (Aib);
- X20 being arginine (R) or alanine (A);
- X22 being alanine (A);
- X23 being alanine (A), glutamate (E) or 2-aminoisobutyric acid (Aib);
- X25 being alanine (A); alpha-methyl-leucine (αMeL) or 2-aminoisobutyric acid (Aib); in particular alpha-methyl-leucine (αMeL) or 2-aminoisobutyric acid (Aib); more in particular alpha-methyl-leucine (αMeL);
- X31 being glutamine (Q) or alanine (A); in particular glutamine (Q) and
- X32 being glutamate (E), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); in particular X32 being selected from 2-aminoisobutyric acid (Aib), 2-aminobutyric acid (Abu); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); and 1-aminocyclohexanecarboxylic acid (Chx).

In a further independent embodiment X7 is D-Valine, X38 is amidated and at least one of X11, X15, X23, X25, X31 and X32 is selected as;
- X11 being alpha-methyl-leucine (αMeL) or 2-aminoisobutyric acid (Aib); in particular alpha-methyl-leucine (αMeL);
- X15 being alpha-methyl-leucine (αMeL) or 2-aminoisobutyric acid (Aib);
- X23 being alanine (A), glutamate (E) or 2-aminoisobutyric acid (Aib);
- X25 being alpha-methyl-leucine (αMeL) or 2-aminoisobutyric acid (Aib); in particular alpha-methyl-leucine (αMeL);
- X31 being glutamine (Q); and
- X32 being glutamate (E), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); in particular X32 being selected from 2-aminoisobutyric acid (Aib), 2-aminobutyric acid (Abu); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); and 1-aminocyclohexanecarboxylic acid (Chx).

In another independent embodiment X1 is and N-methylated or acetylated isoleucine (in particular an acetylated isoleucine) or absent and when absent X2 is acylated Valine, X7 is D-Valine, X38 is amidated and at least one of X11, X15, X23, X25, X31 and X32 is selected as;
- X11 being alpha-methyl-leucine (αMeL) or 2-aminoisobutyric acid (Aib); in particular alpha-methyl-leucine (αMeL);
- X15 being alpha-methyl-leucine (αMeL) or 2-aminoisobutyric acid (Aib);
- X23 being alanine (A), glutamate (E) or 2-aminoisobutyric acid (Aib);
- X25 being alpha-methyl-leucine (αMeL) or 2-aminoisobutyric acid (Aib); in particular alpha-methyl-leucine (αMeL);
- X31 being glutamine (Q); and
- X32 being glutamate (E), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); in particular X32 being selected from 2-aminoisobutyric acid (Aib), 2-aminobutyric acid (Abu); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); and 1-aminocyclohexanecarboxylic acid (Chx).

In an another independent embodiment X7 is D-Valine, X38 is amidated, X32 is selected from glutamate (E), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); in particular X32 being selected from 2-aminoisobutyric acid (Aib), 2-aminobutyric acid (Abu); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); and 1-aminocyclohexanecarboxylic acid (Chx); and at least one of X11, X15, X23, X25, and X31 is selected as;
- X11 being alpha-methyl-leucine (αMeL) or 2-aminoisobutyric acid (Aib); in particular alpha-methyl-leucine (αMeL);
- X15 being alpha-methyl-leucine (αMeL) or 2-aminoisobutyric acid (Aib);
- X23 being alanine (A), glutamate (E) or 2-aminoisobutyric acid (Aib);
- X25 being alpha-methyl-leucine (αMeL) or 2-aminoisobutyric acid (Aib); in particular alpha-methyl-leucine (αMeL); and
- X31 being glutamine (Q).

In another independent embodiment X1 is and N-methylated or acetylated isoleucine (in particular an acetylated isoleucine) or absent and when absent X2 is acylated Valine, X7 is D-Valine, X38 is amidated, X32 is selected from glutamate (E), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); in particular X32 being selected from 2-aminoisobutyric acid (Aib), 2-aminobutyric acid (Abu); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); and 1-aminocyclohexanecarboxylic acid (Chx); and at least one of X11, X15, X23, X25, and X31 is selected as;
- X11 being alpha-methyl-leucine (αMeL) or 2-aminoisobutyric acid (Aib); in particular alpha-methyl-leucine (αMeL);
- X15 being alpha-methyl-leucine (αMeL) or 2-aminoisobutyric acid (Aib);
- X23 being alanine (A), glutamate (E) or 2-aminoisobutyric acid (Aib);
- X25 being alpha-methyl-leucine (αMeL) or 2-aminoisobutyric acid (Aib); in particular alpha-methyl-leucine (αMeL); and
- X31 being glutamine (Q).

In an embodiment:
X1 is absent or an N-terminally acetylated or N-methylated isoleucine (I);
X2 is valine (V), wherein when X1 is absent said valine is N-terminally acylated or alkylated; in particular N- terminally acylated with an alkanoyl group selected from ethanoyl, methanoyl, propanoyl, butanoyl, pentanoyl hexanoyl or heptanoyl; more in particular N- terminally acylated with a pentanoyl group;
X7 is D-valine (v)
X9 is isoleucine (I);
X10 is lysine (K);
X11 is leucine (L) or alpha-methyl-leucine (αMeL);
X14 is isoleucine (I);
X15 is leucine (L);
X23 is lysine (K), alanine (A), glutamate (E) or 2-aminoisobutyric acid (Aib);
X25 is arginine (R);
X26 is glutamate (E);
X31 is asparagine (N);
X32 is glutamate (E), valine (V), threonine (T), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); in particular X32 is selected from 2-aminoisobutyric acid (Aib), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); more in particular X32 is selected from 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex).

In an embodiment:
X1 is absent or an N-terminally acetylated or N-methylated isoleucine (I);
X2 is valine (V), wherein when X1 is absent said valine is N-terminally acetylated or acylated with an alkanoyl group selected from ethanoyl, methanoyl, propanoyl, butanoyl, pentanol hexanoyl or heptanoyl; more in particular N- terminally acylated with a pentanoyl group;
X3 is leucine (L)
X4 is serine (S)
X5 is leucine (L)
X6 is aspartate (D);
X7 is valine (V) or D-valine (v); in particular D-valine (v);
X8 is proline (P)
X9 is isoleucine (I);
X10 is lysine (K);
X11 is leucine (L) or alpha-methyl-leucine (αMeL);
X12 is lysine (K), wherein the epsilon-amino group of the lysine side chain is covalently bound to an albumin-binding moiety (herein also denoted as (K*));
X13 is lysine (K) or glutamine (Q); in particular lysine (K);
X14 is isoleucine (I);
X15 is leucine (L);
X16 is leucine (L) or lysine (K); in particular leucine (L);
X17 is glutamate (E) or arginine (R); in particular glutamate (E);
X18 is glutamine (Q);
X19 is glutamate (E);
X20 is lysine (K) or arginine (R); in particular lysine (K);
X21 is glutamine (Q);
X22 is lysine (K)
X23 is lysine (K), alanine (A), glutamate (E) or 2-aminoisobutyric acid (Aib);
X24 is glutamine (Q);
X25 is arginine (R);
X26 is glutamate (E);
X27 is glutamine (Q);
X28 is alanine (A);
X29 is glutamate (E);
X30 is threonine (T) or lysine (K); in particular threonine (T);
X31 is asparagine (N);
X32 is lysine (K), 2-aminoisobutyric acid (Aib), glutamate (E), alanine (A), 2-aminobutyric acid (Abu); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); or 1-aminocyclopropanecarboxylic acid (Cpx);
X33 is glutamine (Q);
X34 is isoleucine (I)
X35 is leucine (L)
X36 is alanine (A) or glutamate (E); in particular alanine (A);
X37 is glutamine (Q);
X38 is valine (V);
wherein;
when X1 is an N-terminally acetylated or N-methylated isoleucine (I); then X7 is D-valine, and X32 is selected from 2-aminoisobutyric acid (Aib), glutamate (E), alanine (A), 2-aminobutyric acid (Abu); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); or 1-aminocyclopropanecarboxylic acid (Cpx); in particular X32 is then selected from 2-aminoisobutyric acid (Aib), 2-aminobutyric acid (Abu); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); or 1-aminocyclopropanecarboxylic acid (Cpx); even more in particular X32 is then 2-aminoisobutyric acid (Aib), when X1 is an N-terminally acetylated or N-methylated isoleucine (I); and

wherein the albumin binding moiety is selected from the following groups:

   -{AEEA)₂-gGlu-C(O)(CH₂)₁₆COOH;

   -{AEEA)₂-gGlu-C(O)(CH₂)₁₈COOH;

   -{AEEA}-gGlu-C(O)(CH₂)₁₆COOH;

   -{AEEA}-gGlu-C(O)(CH₂)₁₈COOH;

   -gGlu-C(O)(CH₂)₁₄COOH;

   -gGlu-C(O)(CH₂)₁₆COOH;

   -gGlu-C(O)(CH₂)₁₈COOH;

   and

   -{gGlu}₂-C(O)(CH₂)₁₈COOH.
or a pharmaceutically acceptable salt thereof.

In an embodiment:
X1 is absent or an N-terminally acetylated or N-methylated isoleucine (I);
X2 is valine (V), wherein when X1 is absent said valine is N-terminally acylated or alkylated; in particular N- terminally acylated with an alkanoyl group selected from ethanoyl, methanoyl, propanoyl, butanoyl, pentanoyl hexanoyl or heptanoyl; more in particular N- terminally acylated with a pentanoyl group;
X7 is D-valine (v)
X9 is isoleucine (I);
X10 is lysine (K);
X11 is leucine (L) or alpha-methyl-leucine (αMeL);
X14 is isoleucine (I);
X15 is leucine (L);
X23 is lysine (K); glutamate (E) or alanine (A);
X25 is arginine (R);
X26 is glutamate (E);
X31 is asparagine (N);
X32 is glutamate (E), 2-aminoisobutyric acid (Aib), or 4-Amino-piperidine-4-carboxylic acid (4-Pip); and wherein
when X1 is an N-terminally acetylated or N-methylated isoleucine (I), X32 is selected from 2-aminoisobutyric acid (Aib), or 4-Amino-piperidine-4-carboxylic acid (4-Pip).

In an embodiment:
X1 is absent;
X2 is valine (V) N-terminally methylated or acylated with a group selected from a propanoyl group, a butanoyl group, a pentanoyl group or an isopropanoyl group and
X32 is selected from glutamate (E), valine (V), threonine (T), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx).

In an embodiment:
X1 is an N-terminally acetylated or N-methylated isoleucine (I);
X2 is valine (V);
X7 is D-valine (v);
X9 is isoleucine (I);
X10 is lysine (K);
X11 is leucine (L);
X14 is isoleucine (I);
X15 is leucine (L);
X23 is lysine (K), alanine (A), or 2-aminoisobutyric acid (Aib);
X25 is arginine (R);
X26 is glutamate (E);
X31 is asparagine (N);
X32 is glutamate (E), valine (V), threonine (T), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); in particular X32 is selected from 2-aminoisobutyric acid (Aib), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); more in particular X32 is selected from 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex),

In an embodiment:
X1 is absent or isoleucine (I);
X2 is valine (V);
X7 is D-valine (v);
X9 is isoleucine (I);
X10 is lysine (K);
X11 is leucine (L), α-methyl-leucine (αMeL) or 2-aminoisobutyric acid (Aib); in particular X11 is leucine (L), or α-methyl-leucine (αMeL)
X14 is isoleucine (I) or glutamine (Q); in particular X14 is isoleucine (I);
X15 is leucine (L);
X16 is leucine (L);
X17 is glutamate (E) or lysine (K); in particular X17 is glutamate (E)
X23 is lysine (K), alanine (A), or 2-aminoisobutyric acid (Aib); in particular X23 is lysine (K);
X25 is arginine (R);
X26 is glutamate (E);
X30 is lysine (K) or threonine (T); in particular X30 is threonine (T);
X31 is asparagine (N);
X32 is lysine (K), glutamate (E), valine (V), threonine (T), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); in particular X32 is lysine selected from lysine (K), 2-aminoisobutyric acid (Aib), glutamate (E), 2-aminobutyric acid (Abu); α-methyl-lysine (αMeK); 2,3-diaminopropionic acid (Dap); 4-Amino-piperidine-4-carboxylic acid (4-Pip); wherein;
   when X1 is absent, then X2 is optionally N-terminally methylated or acylated with a group selected from an acetyl group, a propanoyl group, a butanoyl group, a pentanoyl group or an isopropanoyl group; and when X1 is isoleucine (I), then X32 is glutamate (E), valine (V), threonine (T), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); in particular X32 is then 2-aminoisobutyric acid (Aib), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); more in particular X32 is 2-aminoisobutyric acid (Aib), glutamate (E), 2-aminobutyric acid (Abu); α-methyl-lysine (αMeK); 2,3-diaminopropionic acid (Dap); 4-Amino-piperidine-4-carboxylic acid (4-Pip); even more in particular X32 is then 2-aminoisobutyric acid (Aib), 2-aminobutyric acid (Abu); α-methyl-lysine (αMeK); 2,3-diaminopropionic acid (Dap); 4-Amino-piperidine-4-carboxylic acid (4-Pip)

As indicated above, the compounds of the disclosure comprise a modified lysine (K) residue at the position denoted by X12 in formula (I), in which an albumin-binding moiety is covalently bound to the epsilon-amino group of the lysine side chain.

The term "albumin-binding moiety" as used herein refers to a moiety which is capable of binding to albumin by covalent or non-covalent binding. In embodiments, the albumin-binding moiety is capable of binding to albumin by non-covalent binding. The albumin-binding moiety may comprise or consist of a group selected from fatty acids, phthalocyanines, coumarins, flavonoids, tetracyclines, naphthalenes, arylcarboxylic acids, heteroarylcarboxylic acids, lipids, alkyl amines, cyclic or linear tetrapyrroles and organometallic compounds thereof, halo-substituted aromatic acid derivatives, organic dyes, and derivatives of tryptophan and thyroxine.

In a particular embodiment, the albumin-binding moiety comprises a C10-C24 fatty acid group which is conjugated to the epsilon-amino group of the lysine side chain either by a direct bond or by a linker. The term "C10-C24 fatty acid" as used herein means a carboxylic acid having from 10 to 24 carbon atoms. The C10-C24 fatty acid can be a saturated monoacid or a saturated diacid. By "saturated" is meant that the fatty acid contains no carbon-carbon double or carbon-carbon triple bonds.

Examples of saturated C10-C24 fatty acids include capric acid (decanoic acid, a C10 monoacid), lauric acid (dodecanoic acid, a C12 monoacid), dodecanedioic acid (DDDA) (a C12 diacid), myristic acid (tetradecanoic acid; a C14 monoacid), tetradecanedioic acid (a C14 diacid), pentadecylic acid (pentadecanoic acid; a C15 monoacid), pentadecanedioic acid (a C15 diacid), palmitic acid (hexadecanoic acid; a C16 monoacid), hexadecanedioic acid (a C16 diacid), margaric acid (heptadecanoic acid; a C17 monoacid), heptadecanedioic acid (a C17 diacid), stearic acid (octadecanoic acid; a C18 monoacid), octadecanedioic acid (a C18 diacid), nonadecylic acid (nonadecanoic acid; a C19 monoacid), nonadecanedioic acid (a C19 diacid), arachadic acid (eicosanoic acid; a C20 monoacid), eicosanedioic acid (a C20 diacid), heneicosylic acid (heneicosanoic acid; a C21 monoacid), heneicosanedioic acid (a C21 diacid), behenic acid (docosanoic acid; a C22 monoacid), docosanedioic acid (a C22 diacid), tricosylic acid (tricosanoic acid, a C23 monoacid), tricosanedioic acid (a C23 diacid), lignoceric acid (tetracosanoic acid; a C24 monoacid), and tetracosanedioic acid (a C24 diacid).

**In** an embodiment, the C10-C24 fatty acid group is a C12-C24 fatty acid; in particular a C12-C20 fatty acid; more in particular a fatty acid selected from the group consisting of lauric acid (dodecanoic acid, a C12 monoacid), dodecanedioic acid (DDDA) (a C12 diacid), myristic acid (tetradecanoic acid; a C14 monoacid), tetradecanedioic acid (a C14 diacid), palmitic acid (hexadecanoic acid; a C16 monoacid), hexadecanedioic acid (a C16 diacid), stearic acid (octadecanoic acid; a C18 monoacid), octadecanedioic acid (a C18 diacid), arachadic acid (eicosanoic acid; a C20 monoacid) and eicosanedioic acid (a C20 diacid).

The C10-C24 fatty acid may be bound directly to the epsilon-amino group of the lysine side chain. Alternatively, the C10-C24 fatty acid may be bound to the epsilon-amino group of the lysine side chain through a linker. The linker may comprise one or more groups selected from [2-(2-aminoethoxy)ethoxy]acetyl (referred to herein as "AEEA"), glycine (Gly), N-methylglycine (N-MeGly) and gamma-glutamate (gGlu).

**In** an embodiment, the albumin-binding moiety is a group of the formula (II):

-Y-Z-C(O)R¹ (II)

wherein
Y is AEEA, {AEEA}₂, {AEEA}s, Gly, {Gly}₂, {Gly}₃, N-MeGly, {N-MeGly}₂, {N-MeGly}₃ or absent;
Z is gGlu, {gGlu}₂ or absent; and
R' is -(CH₂)ₓCOOH or -(CH₂)ₓCH₃, wherein x is an integer from 10 to 22.

**In** an embodiment of the albumin binding moiety of formula (II) Y is {AEEA} or {AEEA}₂ and Z is gGlu or {gGlu}₂.

**In** an embodiment of the albumin binding moiety of formula (II), Y is absent and Z is gGlu.

**In** an embodiment of the albumin binding moiety of formula (II), Y and Z are both absent.

**In** an embodiment of the albumin binding moiety of formula (II), R¹ is -(CH₂)ₓCOOH, wherein x is an integer from 12 to 18.

**In** an embodiment of the albumin binding moiety of formula (II), Y is {AEEA} or {AEEA}₂; Z is gGlu or {gGlu}₂; and R' is -(CH₂)ₓCOOH, wherein x is an integer from 12 to 18

**In** an embodiment, the albumin-binding moiety is selected from the following groups:

-{AEEA}-gGlu-C(O)(CH₂)₁₀COOH;

-{AEEA}-gGlu-C(O)(CH₂)₁₂COOH;

-{AEEA}-gGlu-C(O)(CH₂)₁₄COOH;

-{AEEA}-gGlu-C(O)(CH₂)₁₆COOH;

-{AEEA}-gGlu-C(O)(CH₂)₁₈COOH;

-{AEEA}₂-gGlu-C(O)(CH₂)₁₀COOH;

-{AEEA}₂-gGlu-C(O)(CH₂)₁₂COOH;

-{AEEA}₂-gGlu-C(O)(CH₂)₁₄COOH;

-{AEEA)₂-gGlu-C(O)(CH₂)₁₆COOH;

-{AEEA)₂-gGlu-C(O)(CH₂)₁₈COOH;

-{AEEA}₂-{gGlu}₂-C(O)(CH₂)₁₀COOH;

-{AEEA}₂-{gGlu}₂-C(O)(CH₂)₁₂COOH;

-{AEEA}₂-{gGlu}₂-C(O)(CH₂)₁₄COOH;

-{AEEA}₂-{gGlu}₂-C(O)(CH₂)₁₆COOH;

-{AEEA}₂-{gGlu}₂-C(O)(CH₂)₁₈COOH;

-gGlu-C(O)(CH₂)₁₀COOH;

-gGlu-C(O)(CH₂)₁₂COOH;

-gGlu-C(O)(CH₂)₁₄COOH;

-gGlu-C(O)(CH₂)₁₆COOH;

-gGlu-C(O)(CH₂)₁₈COOH;

-{gGlu}₂-C(O)(CH₂)₁₀COOH;

-{gGlu}₂-C(O)(CH₂)₁₂COOH;

-{gGlu}₁₂-C(O)(CH₂)₁₄COOH;

-{gGlu}₂-C(O)(CH₂)₁₆COOH;

and

-{gGlu}₁₂-C(O)(CH₂)₁₈COOH.

In an embodiment, the albumin-binding moiety is selected from the following groups:

-{AEEA}₂-gGlu-C(O)(CH₂)₁₀COOH;

-{AEEA}₂-gGlu-C(O)(CH₂)₁₂COOH;

-{AEEA}₂-gGlu-C(O)(CH₂)₁₄COOH;

-{AEEA)₂-gGlu-C(O)(CH₂)₁₆COOH;

-{AEEA)₂-gGlu-C(O)(CH₂)₁₈COOH;

-{AEEA}₂-{gGlu}₂-C(O)(CH₂)₁₀COOH;

-{AEEA}₂-{gGlu}₂-C(O)(CH₂)₁₂COOH;

-{AEEA}₂-{gGlu}₁₂-C(O)(CH₂)₁₄COOH;

-{AEEA}₂-{gGlu}₂-C(O)(CH₂)₁₆COOH;

-{AEEA}₂-{gGlu}₂-C(O)(CH₂)₁₈COOH;

-gGlu-C(O)(CH₂)₁₀COOH;

-gGlu-C(O)(CH₂)₁₂COOH;

-gGlu-C(O)(CH₂)₁₄COOH;

-gGlu-C(O)(CH₂)₁₆COOH;

and

-gGlu-C(O)(CH₂)₁₈COOH.

In an embodiment, the albumin-binding moiety is selected from the following groups:

-{AEEA)₂-gGlu-C(O)(CH₂)₁₆COOH;

-{AEEA)₂-gGlu-C(O)(CH₂)₁₈COOH;

-{AEEA}-gGlu-C(O)(CH₂)₁₆COOH;

-{AEEA}-gGlu-C(O)(CH₂)₁₈COOH;

-gGlu-C(O)(CH₂)₁₄COOH;

-gGlu-C(O)(CH₂)₁₆COOH;

-gGlu-C(O)(CH₂)₁₈COOH;

-{gGlu}₂-C(O)(CH₂)₁₆COOH;

and

-{gGlu}₂-C(O)(CH₂)₁₈COOH.

In an embodiment, the albumin-binding moiety is selected from the following groups:

-{AEEA)₂-gGlu-C(O)(CH₂)₁₆COOH;

-{AEEA)₂-gGlu-C(O)(CH₂)₁₈COOH;

-{AEEA}-gGlu-C(O)(CH₂)₁₆COOH;

-{AEEA}-gGlu-C(O)(CH₂)₁₈COOH;

-gGlu-C(O)(CH₂)₁₄COOH;

-gGlu-C(O)(CH₂)₁₆COOH;

-gGlu-C(O)(CH₂)₁₈COOH;

and

-{gGlu}₁₂-C(O)(CH₂)₁₈COOH.

The chemical structures and IUPAC names of some examples of these groups are shown in Table 1 below, in which R denotes the point of attachment of each group to the epsilon-amino group of the lysine residue at X12:

**Table 1**

| **Group** | **Chemical structure / IUPAC name** |
|---|---|
| -{AEEA}₂-gGlu-C(O)(CH₂)₁₆COOH | 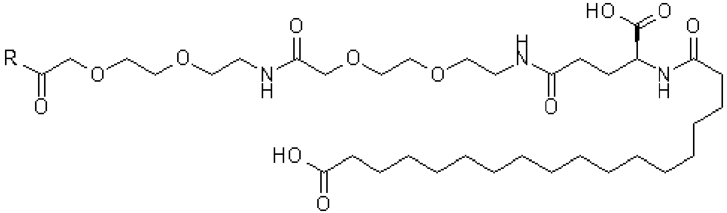 [2-[2-[2-[[2-[2-[2-[[(4S)-4-carboxy-4-(17-carboxyheptadecanoylamino)butanoyl]amino]ethoxy] ethoxy]-acetyl]amino]ethoxy]ethoxy]acetyl- |
| -{AEEA}₂-gGlu-C(O)(CH₂)₁₈COOH | 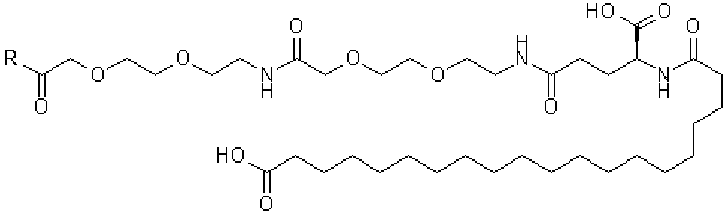 [2-[2-[2-[[2-[2-[2-[[(4S)-4-carboxy-4-(19-carboxynonadecanoylamino)butanoyl]amino]ethoxy] ethoxy]-acetyl]amino]ethoxy]ethoxy]acetyl- |
| -{AEEA}₂-{gGlu}₂-C(O)(CH₂)₁₆COOH | 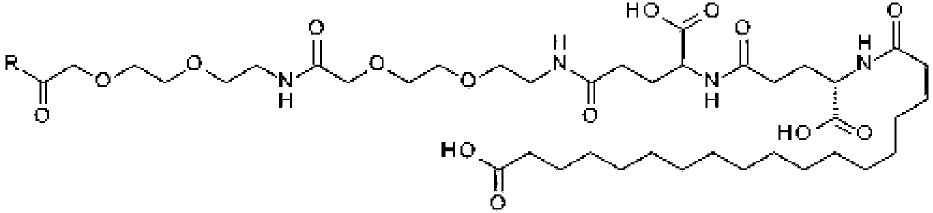 [2-[2-[2-[[2-[2-[2-[[(4S)-4-carboxy-4-[[(4S)-4-carboxy-4-(17-carboxyheptadecanoylamino) butanoyl]amino]butanoyl]amino]ethoxy]-ethoxy]acetyl]amino]ethoxy]ethoxy]acetyl- |
| -{AEEA}₂-{gGlu}₂-C(O)(CH₂)₁₈COOH | 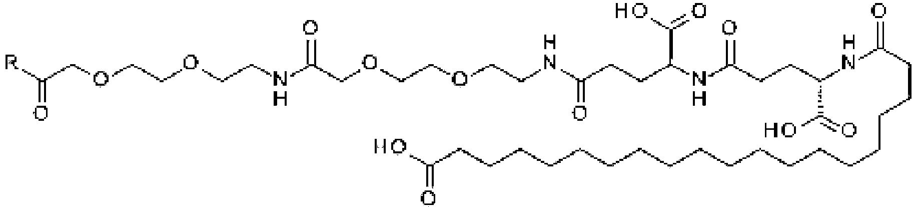 [2-[2-[2-[[2-[2-[2-[[(4S)-4-carboxy-4-[[(4S)-4-carboxy-4-(19-carboxynonadecanoylamino)butanoyl] amino]butanoyl]amino]ethoxy]-ethoxy]acetyl]amino]ethoxy]ethoxy]acetyl- |
| -gGlu-C(O)(CH₂)₁₆COOH | 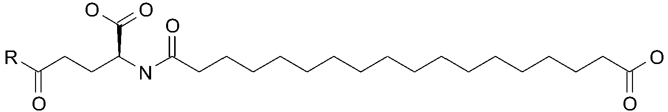 18-[[(1S)-1-carboxy-4-oxo-butyl]amino]-18-oxo-octadecanoic acid |
| -gGlu-C(O)(CH₂)₁₄COOH | 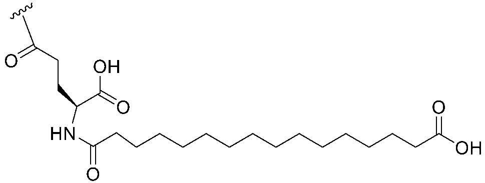 |
| -gGlu-C(O)(CH₂)₁₂COOH | 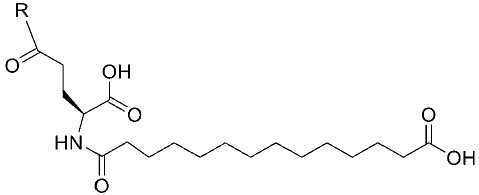 |
| -{AEEA}₂-gGlu-C(O)(CH₂)₁₂COOH | 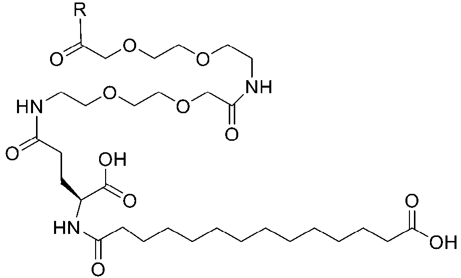 |
| -{AEEA}₂-{gGlu}₂-C(O)(CH₂)₁₂COOH | 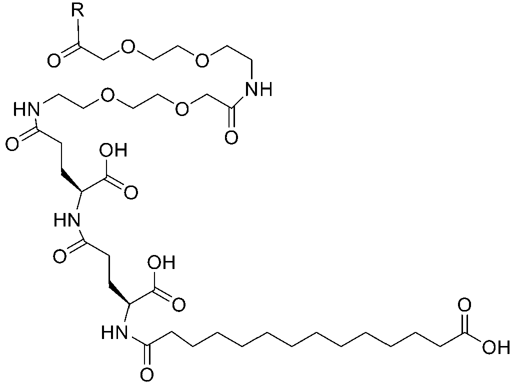 |
| -gGlu-C(O)(CH₂)₁₀COOH | 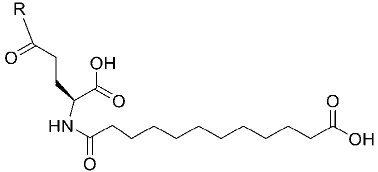 |
| -gGlu-C(O)(CH₂)₁₆COOH | 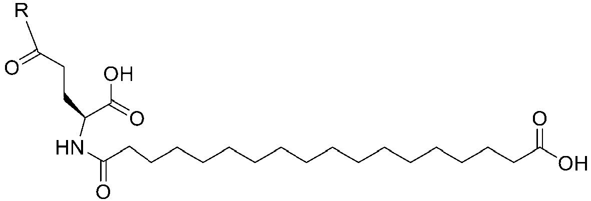 |
| -{AEEA}₂-gGlu-C(O)(CH₂)₁₆COOH | 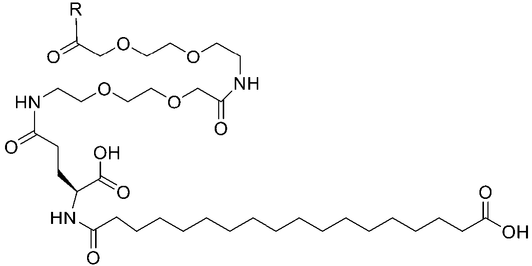 |
| -{AEEA}₂-{gGlu}₂-C(O)(CH₂)₁₆COOH | 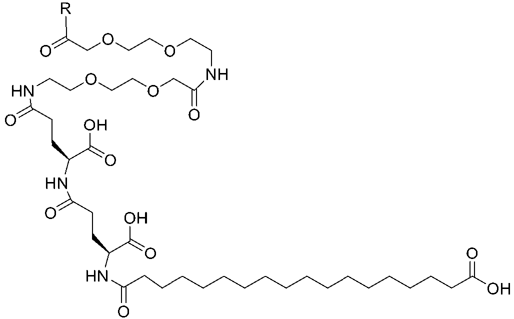 |
| -AEEA-{gGlu}₂-C(O)(CH₂)₁₆COOH | 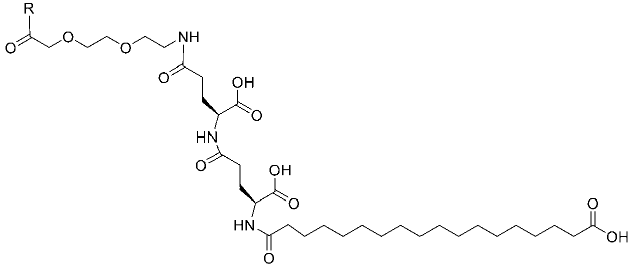 |
| -AEEA-gGlu-C(O)(CH₂)₁₆COOH | 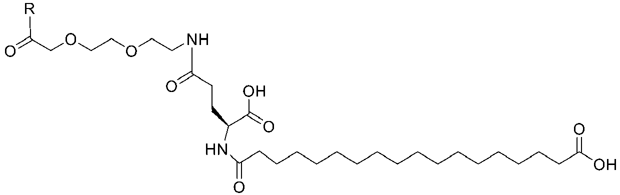 |
| -AEEA-gGlu-AEEA-gGlu-C(O)(CH₂)₁₆COOH | 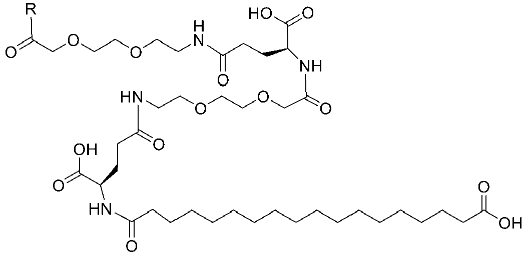 |
| -{AEEA}₃-gGlu-C(O)(CH₂)₁₆COOH | 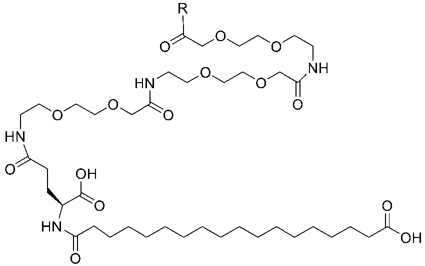 |
| -gGlu-C(O)(CH₂)₁₈COOH | 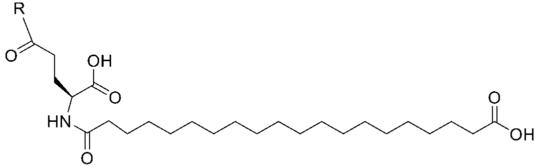 |
| -AEEA-gGlu-C(O)(CH₂)₁₈COOH | 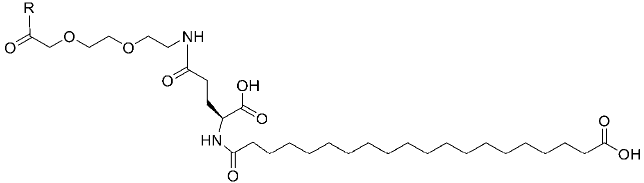 |
| -{AEEA}₂-gGlu-C(O)(CH2)₁₈COOH | 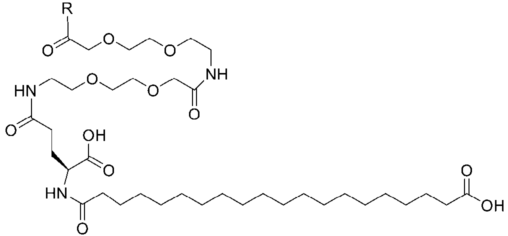 |
| -{gGlu}₂-C(O)(CH2)₁₈COOH | 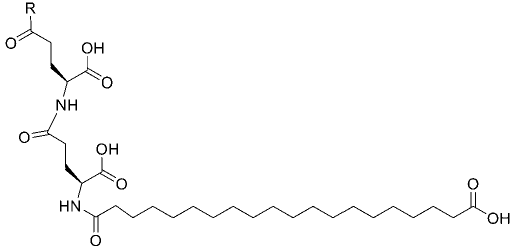 |

In an embodiment, the albumin-binding moiety is selected from the group consisting of -gGlu-C(O)(CH₂)₁₂COOH; -gGlu-C(O)(CH₂)₁₄COOH; -gGlu-C(O)(CH₂)₁₆COOH; -gGlu-C(O)(CH₂)₁₈COOH; -{AEEA}₂-gGlu-C(O)(CH₂)₁₂COOH, - {AEEA}₂-gGlu-C(O)(CH₂)₁₆COOH; -{AEEA}₂-gGlu-C(O)(CH₂)₁₈COOH and -{AEEA}₂-{gGlu}₂-C(O)(CH₂)₁₆COOH.

In a particular embodiment, the albumin-binding moiety is selected from the group consisting of -gGlu-C(O)(CH₂)₁₂COOH; -gGlu-C(O)(CH₂)₁₄COOH; -gGlu-C(O)(CH₂)₁₆COOH; -{AEEA}₂-gGlu-C(O)(CH₂)₁₆COOH and -{AEEA}₂-{gGlu}₂-C(O)(CH₂)₁₆COOH.

In another particular embodiment, the albumin-binding moiety is selected from the group consisting of -gGlu-C(O)(CH₂)₁₄COOH; -gGlu-C(O)(CH₂)₁₆COOH; and - {AEEA}₂-gGlu-C(O)(CH₂)₁₆COOH.

In an embodiment, the compound is a peptide comprising the amino acid sequence of any one of SEQ ID NOs 1 to 244, but for the comparative peptides, and said peptides wherein X1 is isoleucine and said isoleucine is not N-terminally acetylated or N-methylated or a pharmaceutically acceptable salt thereof.

In an embodiment, the compound is a peptide comprising the amino acid sequence of any one of SEQ ID NOs 19, 33, 34, 40, 43, 47, 51, 54, 55, , 57, 64, 80, 92, 93, 96, 98, 100, 104, 110, 113, 118, 125, 131, 132, 136, 144, 145, 189, 190, 193, 203, 206, 210, 212, 213, 215, 217, 218, 219, 222, 230, 231, 233, 234, 235, 236, 237, 238, 241, 242, 243 or a pharmaceutically acceptable salt thereof.

In an embodiment, the compound is a peptide comprising the amino acid sequence of any one of SEQ ID NOs 40, 51, 57, 64, 80, 98, 110, 206, 212, 213, 215, 218, 231, 235, 237, 238, 243 or a pharmaceutically acceptable salt thereof

In an embodiment, the compound is a peptide of the amino acid sequence of formula (I), optionally wherein the amino acid residue at X1 is acetylated and optionally wherein the amino acid residue at X38 is amidated; or a pharmaceutically acceptable salt thereof.

In an embodiment, the compound is a peptide of the amino acid sequence of formula (I) or a pharmaceutically acceptable salt thereof; wherein:
X1 is absent;
X2 is valine (V), wherein when said valine is N-terminally acylated or alkylated; in particular N- terminally acylated with an C1-10alkanoyl group; more in particular selected from ethanoyl, methanoyl, propanoyl, butanoyl, pentanoyl hexanoyl or heptanoyl;
X7 is D-valine (v)
X9 is isoleucine (I);
X10 is lysine (K);
X11 is leucine (L) or alpha-methyl-leucine (αMeL);
X14 is isoleucine (I);
X15 is leucine (L);
X23 is lysine (K), glutamate (E), alanine (A), or 2-aminoisobutyric acid (Aib);
X25 is arginine (R);
X26 is glutamate (E);
X31 is asparagine (N);
X32 is glutamate (E), valine (V), threonine (T), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx);
and optionally wherein the amino acid residue at X38 is amidated.

In an embodiment, the compound is a peptide of the amino acid sequence of formula (I) or a pharmaceutically acceptable salt thereof; wherein:
X1 is an N-terminally acetylated or N-methylated isoleucine (I);
X2 is valine (V);
X7 is D-valine (v);
X9 is isoleucine (I);
X10 is lysine (K);
X11 is leucine (L);
X14 is isoleucine (I);
X15 is leucine (L);
X23 is lysine (K), glutamate (E), alanine (A), or 2-aminoisobutyric acid (Aib);
X25 is arginine (R);
X26 is glutamate (E);
X31 is asparagine (N);
X32 is glutamate (E), valine (V), threonine (T), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); in particular 2-aminoisobutyric acid (Aib), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx);
and optionally wherein the amino acid residue at X38 is amidated.

In an embodiment, the compound is a peptide of any one of SEQ ID NOs 1 to 145 or SEQ ID Nos 152 to 244, or a pharmaceutically acceptable salt thereof. These peptides are listed in Table 2 below, in which K* denotes the modified lysine residue at X12, R^{a} denotes the albumin-binding moiety, Ac- denotes that the N-terminal is acetylated, NMe-denotes that the N-terminal is N-methylated, Propanoyl- denotes that the N-terminal is alkylated with a propanoyl group, Butanoyl- denotes that the N-terminal is alkylated with a butanoyl group, Pentanoyl- denotes that the N-terminal is alkylated with a pentanoyl group, and -NH2 denotes that the C-terminal amino acid residue is amidated as a primary amide.

As used herein, N-terminally acetylated peptide is generally represented as; 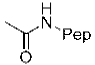 wherein Pep refers to the peptide structure.

As used herein, N-terminally acylated peptide with a propanoyl group is generally represented as; 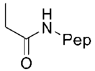 wherein Pep refers to the peptide structure.

As used herein, N-terminally acylated peptide with a butanoyl group is generally represented as; 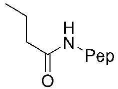 wherein Pep refers to the peptide structure.

As used herein, N-terminally acylated peptide with a pentanoyl group is generally represented as; 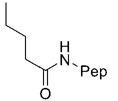 wherein Pep refers to the peptide structure.

As used herein, N-terminally acylated peptide with a isopentanoyl group is generally represented as; 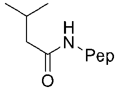 wherein Pep refers to the peptide structure.

**Table 2**

| **SEQ ID NO.** | **Amino acid sequence** | **R^{a}** |
|---|---|---|
| 2 (comp) | Ac-IVLSLDvPIKLK*QILLKQERQKKQREQAEKNKQILEQV-NH2 | -gGlu-C(O)(CH₂)₁₄COOH |
| 3 | Ac-VLSLDvPIKLK*QILLKQERQKKQREQAEKNKQILEQV-NH2 | -gGlu-C(O)(CH₂)₁₄COOH |
| 4 | VLSLDvPIKLK*QILLKQERQKKQREQAEKNKQILEQV-NH2 | -gGlu-C(O)(CH₂)₁₄COOH |
| 5 (comp) | NMeIVLSLDvPIKLK*QILLKQERQKKQREQAEKNKQILEQV-NH2 | -gGlu-C(O)(CH₂)₁₄COOH |
| 6 | Propanoyl-VLSLDvPIKLK*QILLKQERQKKQREQAEKNKQILEQV-NH2 | -gGlu-C(O)(CH₂)₁₄COOH |
| 7 | Butanoyl-VLSLDvPIKLK*QILLKQERQKKQREQAEKNKQILEQV-NH2 | -gGlu-C(O)(CH₂)₁₄COOH |
| 8 | Pentanoyl-VLSLDvPIKLK*QILLKQERQKKQREQAEKNKQILEQV-NH2 | -gGlu-C(O)(CH₂)₁₄COOH |
| 9 | Isopentanoyl-VLSLDvPIKLK*QILLKQERQKKQREQAEKNKQILEQV-NH2 | -gGlu-C(O)(CH₂)₁₄COOH |
| 10 | IVLSLDvPIKLK*QILLKQERQKKQREQAEKNAibQILEQV-NH2 | -gGlu-C(O)(CH₂)₁₄COOH |
| 11 (comp) | IVLSLDvPIKLK*QILLKQERQKKQREQAEKQKQILEQV-NH2 | -gGlu-C(O)(CH₂)₁₄COOH |
| 12 (comp) | IVLSLDvPIKLK*QILLKQERQKKQREQAEKAKQILEQV-NH2 | -gGlu-C(O)(CH₂)₁₄COOH |
| 13 | IVLSLDvPIKLK*QILLKQERQKKQREQAEKNVQILEQV-NH2 | -gGlu-C(O)(CH₂)₁₄COOH |
| 14 | IVLSLDvPIKLK*QILLKQERQKKQREQAEKNTQILEQV-NH2 | -gGlu-C(O)(CH₂)₁₄COOH |
| 15 (comp) | IVLSLDvPIKLK*QILLKQERQKKQREQAETNKQILEQV-NH2 | -gGlu-C(O)(CH₂)₁₄COOH |
| 16 | Ac-IVLSLDvPIKLK*QILLKQERQKKQREQAEKNAibQILEQV-NH2 | -gGlu-C(O)(CH₂)₁₄COOH |
| 17 (comp) | Ac-IVLSLDvPIKLK*QILLKQERQKKQREQAEKNEQILEQV-NH2 | -gGlu-C(O)(CH₂)₁₄COOH |
| 18 | Ac-IVLSLDvPIKLK*QAibLLKQERQKKQREQAEKNVQILEQV-NH2 | -gGlu-C(O)(CH₂)₁₄COOH |
| 19 | Ac-IVLSLDVPIKαMeLK*QILLKQERQKKQREQAEKNAibQILEQV-NH2 | -gGlu-C(O)(CH₂)₁₄COOH |
| 20 | Ac-IVLSLDVPIKαMeLK*QAibLLKQERQKKQREQAEKNAibQILEQV-NH2 | -gGlu-C(O)(CH₂)₁₄COOH |
| 21 | Ac-IVLSLDvPIKLK*KILLKQERQKKQREQAEKNAibQILEQV-NH2 | -gGlu-C(O)(CH₂)₁₄COOH |
| 22 | Pentanoyl-VLSLDvPIKLK*QAibLLKQERQKKQREQAEKNVQILEQV-NH2 | -gGlu-C(O)(CH₂)₁₄COOH |
| 23 | NMeIVLSLDvPIKLK*QILLKQERQKKQREQAEKNAibQILEQV-NH2 | -gGlu-C(O)(CH₂)₁₆COOH |
| 24 | NMelVLSLDvPIKLK*QILLKQERQKKQREQAEKNAQILEQV-NH2 | -gGlu-C(O)(CH₂)₁₆COOH |
| 25 | IVLSLDVPIKLK*QAibLLKQERQKKQREQAEKNAibQILEQV-NH2 | -{AEEA}₂-{gGlu}₂-C(O)(CH₂)₁₆COOH |
| 26 | Ac-IVLSLDVPIKLK*QAibLLKQERQKKQREQAEKNAibQILEQV-NH2 | -{AEEA}₂-{gGlu}₂-C(O)(CH₂)₁₆COOH |
| 27 | Ac-IVLSLDvPIKLK*QAibLLKQERQKKQREQAEKNVQILEQV-NH2 | -{AEEA}₂-{gGlu}₂-C(O)(CH₂)₁₆COOH |
| 28 | NMe-IVLSLDvPIKLK*QAibLLKQERQKKQREQAEKNVQILEQV-NH2 | -{AEEA}₂-{gGlu}₂-C(O)(CH₂)₁₆COOH |
| 29 | Pentanoyl-VLSLDvPIKLK*QAibLLKQERQKKQREQAEKNVQILEQV-NH2 | -{AEEA}₂-{gGlu}₂-C(O)(CH₂)₁₆COOH |
| 30 | Ac-IVLSLDvPIKLK*QILLKQERQKKQREQAEKNAibQILEQV-NH2 | -{AEEA}₂-{gGlu}₂-C(O)(CH₂)₁₆COOH |
| 31 | NMe-IVLSLDvPIKLK*QILLKQERQKKQREQAEKNAibQILEQV-NH2 | -{AEEA}₂-{gGlu}₂-C(O)(CH₂)₁₆COOH |
| 32 | Pentanoyl-VLSLDvPIKLK*QILLKQERQKKQREQAEKNAibQILEQV-NH2 | -{AEEA}₂-{gGlu}₂-C(O)(CH₂)₁₆COOH |
| 33 | Ac-VLSLDvPIKLK*KILLEQEKQKKQREQAETNKQILAQV-NH2 | -gGlu-C(O)(CH₂)₁₄COOH |
| 34 | VLSLDvPIKLK*KILLEQEKQKKQREQAETNKQILAQV-NH2 | -gGlu-C(O)(CH₂)₁₄COOH |
| 35 | Propanoyl-VLSLDvPIKLK*KILLEQEKQKKQREQAETNKQILAQV-NH2 | -gGlu-C(O)(CH₂)₁₄COOH |
| 36 | Butanoyl-VLSLDvPIKLK*KILLEQEKQKKQREQAETNKQILAQV-NH2 | -gGlu-C(O)(CH₂)₁₄COOH |
| 37 | Pentanoyl-VLSLDvPIKLK*KILLEQEKQKKQREQAETNKQILAQV-NH2 | -gGlu-C(O)(CH₂)₁₄COOH |
| 38 | IVLSLDVPIKαMeLK*KILLEQEKQKKQREQAETNAibQILAQV-NH2 | -gGlu-C(O)(CH₂)₁₄COOH |
| 39 | Ac-IVLSLDVPIKLK*KILLEQEKQKKQREQAETNAibQILAQV-NH2 | -gGlu-C(O)(CH₂)₁₄COOH |
| 40 | Ac-IVLSLDvPIKLK*KILLEQEKQKKQREQAETNAibQILAQV-NH2 | -gGlu-C(O)(CH₂)₁₄COOH |
| 41 | Ac-IVLSLDvPIKLK*KILLEQEKQKKQREQAETNAibQILAQV-NH2 | -{gGlu}₂-C(O)(CH₂)₁₄COOH |
| 42 | Ac-IVLSLDvPIKLK*KILLEQEKQKKQREQAETNAibQILAQV-NH2 | -{AEEA}₂-gGlu-C(O)(CH₂)₁₄COOH |
| 43 | Ac-IVLSLDVPIKαMeLK*KILLEQEKQKKQREQAETNAibQILAQV-NH2 | -gGlu-C(O)(CH₂)₁₄COOH |
| 44 | Ac-IVLSLDvPIKαMeLK*KILLEQEKQKKQREQAETNAibQILAQV-NH2 | -gGlu-C(O)(CH₂)₁₄COOH |
| 45 | Ac-VLSLDvPIKLK*KILLEQEKQKKQREQAETNAibQILAQV-NH2 | -gGlu-C(O)(CH₂)₁₄COOH |
| 46 | Ac-VLSLDvPIKαMeLK*KILLEQEKQKKQREQAETNAibQILAQV-NH2 | -gGlu-C(O)(CH₂)₁₄COOH |
| 47 | Ac-IVLSLDvPIKLK*KILLEQEKQKKQREQAETNAQILAQV-NH2 | -gGlu-C(O)(CH₂)₁₄COOH |
| 48 | Ac-IVLSLDvPIKLK*KILLEQEKQKKQREQAETNVQILAQV-NH2 | -gGlu-C(O)(CH₂)₁₄COOH |
| 49 | Ac-IVLSLDVPIKαMeLK*KILLEQEKQKKQREQAETNVQILAQV-NH2 | -gGlu-C(O)(CH₂)₁₄COOH |
| 50 | Ac-IVLSLDvPIKαMeLK*KILLEQEKQKKQREQAETNVQILAQV-NH2 | -gGlu-C(O)(CH₂)₁₄COOH |
| 51 | Ac-IVLSLDvPIKLK*KILLEQEKQKKQREQAETNAbuQILAQV-NH2 | -gGlu-C(O)(CH₂)₁₄COOH |
| 52 | Ac-IVLSLDvPIKLK*KILLEQEKQKKQREQAETNNvaQILAQV-NH2 | -gGlu-C(O)(CH₂)₁₄COOH |
| 53 | Ac-IVLSLDvPIKαMeLK*KILLEQEKQKKQREQAETDKQILAQV-NH2 | -gGlu-C(O)(CH₂)₁₄COOH |
| 54 | Ac-IVLSLDvPIKLK*KILLEQEKQKKQREQAETNαMeKQILAQV-NH2 | -gGlu-C(O)(CH₂)₁₄COOH |
| 55 | Ac-IVLSLDvPIKLK*KILLEQEKQKKQREQAETNDapQILAQV-NH2 | -gGlu-C(O)(CH₂)₁₄COOH |
| 56 (comp) | Ac-IVLSLDvPIKLK*KILLEQEKQKKQREQAETNEQILAQV-NH2 | -gGlu-C(O)(CH₂)₁₄COOH |
| 57 | Ac-IVLSLDvPIKLK*KILLEQEKQKKQREQAETN4-PipQILAQV-NH2 | -gGlu-C(O)(CH₂)₁₄COOH |
| 58 | Ac-IVLSLDvPIKLK*KILLEQEKQKKQREQAETNThPQILAQV-NH2 | -gGlu-C(O)(CH₂)₁₄COOH |
| 59 | Ac-IVLSLDvPIKLK*KILLEQEKQKKQREQAETNChxQILAQV-NH2 | -gGlu-C(O)(CH₂)₁₄COOH |
| 60 | Ac-IVLSLDvPIKLK*KILLEQEKQKKQREQAETNCpexQILAQV-NH2 | -gGlu-C(O)(CH₂)₁₄COOH |
| 61 | Ac-IVLSLDvPIKLK*KILLEQEKQKKQREQAETNCpxQILAQV-NH2 | -gGlu-C(O)(CH₂)₁₄COOH |
| 62 | Ac-IVLSLDvPIKLK*KILLEQEKQKAQREQAETN4PipQILAQV-NH2 | -gGlu-C(O)(CH₂)₁₄COOH |
| 63 | Ac-IVLSLDvPIKLK*KILLEQEKQKAibQREQAETN4PipQILAQV-NH2 | -gGlu-C(O)(CH₂)₁₄COOH |
| 64 | NMeIVLSLDvPIKLK*KILLEQEKQKKQREQAETNAibQILAQV-NH2 | -gGlu-C(O)(CH₂)₁₄COOH |
| 65 | NMeIVLSLDvPIKαMeLK*KILLEQEKQKKQREQAETNAibQILAQV-NH2 | -gGlu-C(O)(CH₂)₁₄COOH |
| 66 | NMelVLSLDvPIKLK*KILLEQEKQKKQREQAETNVQILAQV-NH2 | -gGlu-C(O)(CH₂)₁₄COOH |
| 67 | NMeIVLSLDvPIKαMeLK*KILLEQEKQKKQREQAETNVQILAQV-NH2 | -gGlu-C(O)(CH₂)₁₄COOH |
| 68 | NMelVLSLDvPIKLK*KILLEQEKQKKQREQAETNAQILAQV-NH2 | -gGlu-C(O)(CH₂)₁₄COOH |
| 69 | NMeIVLSLDvPIKLK*KILLEQEKQKKQREQAETNAbuQILAQV-NH2 | -gGlu-C(O)(CH₂)₁₄COOH |
| 70 | NMeIVLSLDvPIKLK*KILLEQEKQKKQREQAETNDapQILAQV-NH2 | -gGlu-C(O)(CH₂)₁₄COOH |
| 71 | NMeIVLSLDvPIKLK*KILLEQEKQKKQREQAETNEQILAQV-NH2 | -gGlu-C(O)(CH₂)₁₄COOH |
| 72 | NMeIVLSLDvPIKLK*KILLEQEKQKKQREQAETN4-PipQILAQV-NH2 | -gGlu-C(O)(CH₂)₁₄COOH |
| 73 | NMeIVLSLDvPIKLK*KILLEQEKQKKQREQAETNThPQILAQV-NH2 | -gGlu-C(O)(CH₂)₁₄COOH |
| 74 | NMeIVLSLDvPIKLK*KILLEQEKQKAQREQAETNAibQILAQV-NH2 | -gGlu-C(O)(CH₂)₁₄COOH |
| 75 | NMeIVLSLDvPIKLK*KILLEQEKQKAibQREQAETNAibQILAQV-NH2 | -gGlu-C(O)(CH₂)₁₄COOH |
| 76 | NMeIVLSLDvPIKLK*KILLEQEKQKAQREQAETN4PipQILAQV-NH2 | -gGlu-C(O)(CH₂)₁₄COOH |
| 77 | NMeIVLSLDvPIKLK*KILLEQEKQKAibQREQAETN4PipQILAQV-NH2 | -gGlu-C(O)(CH₂)₁₄COOH |
| 78 | Propanoyl-VLSLDvPIKLK*KILLEQEKQKKQREQAETNAibQILAQV-NH2 | -gGlu-C(O)(CH₂)₁₄COOH |
| 79 | Butanoyl-VLSLDvPIKLK*KILLEQEKQKKQREQAETNAibQILAQV-NH2 | -gGlu-C(O)(CH₂)₁₄COOH |
| 80 | Pentanoyl-VLSLDvPIKLK*KILLEQEKQKKQREQAETNAibQILAQV-NH2 | -gGlu-C(O)(CH2)14COOH |
| 81 | Pentanoyl-VLSLDvPIKLK*KILLEQEKQKKQREQAETNVQILAQV-NH2 | -gGlu-C(O)(CH2)14COOH |
| 82 | Ac-IVLSLDvPIKLK*KILLEQEKQKKQREQAETNAibQILAQV-NH2 | -gGlu-C(O)(CH₂)₁₂COOH |
| 83 | Ac-IVLSLDvPIKLK*KILLEQEKQKKQREQAETNAbuQILAQV-NH2 | -gGlu-C(O)(CH₂)₁₂COOH |
| 84 | Ac-IVLSLDvPIKLK*KILLEQEKQKKQREQAETN4-PipQILAQV-NH2 | -gGlu-C(O)(CH₂)₁₂COOH |
| 85 (comp) | Ac-IVLSLDvPIKLK*KILLEQEKQKKQREQAETNEQILAQV-NH2 | -gGlu-C(O)(CH₂)₁₂COOH |
| 86 | Pentanoyl-VLSLDvPIKLK*KILLEQEKQKKQREQAETNAibQILAQV-NH2 | -gGlu-C(O)(CH₂)₁₂COOH |
| 87 | NMeIVLSLDvPIKLK*KILLEQEKQKKQREQAETNAibQILAQV-NH2 | -gGlu-C(O)(CH₂)₁₂COOH |
| 88 | NMeIVLSLDvPIKLK*KILLEQEKQKAQREQAETNAibQILAQV-NH2 | -gGlu-C(O)(CH₂)₁₂COOH |
| 89 | NMeIVLSLDvPIKLK*KILLEQEKQKAibQREQAETNAibQILAQV-NH2 | -gGlu-C(O)(CH₂)₁₂COOH |
| 90 | Ac-IVLSLDvPIKLK*KILLEQEKQKAQREQAETN4PipQILAQV-NH2 | -gGlu-C(O)(CH₂)₁₂COOH |
| 91 | Ac-IVLSLDvPIKLK*KILLEQEKQKAibQREQAETN4PipQILAQV-NH2 | -gGlu-C(O)(CH₂)₁₂COOH |
| 92 | Ac-VLSLDvPIKLK*KILLEQEKQKKQREQAETNKQILAQV-NH2 | -{AEEA}₂-gGlu-C(O)(CH₂)₁₆COOH |
| 93 | VLSLDvPIKLK*KILLEQEKQKKQREQAETNKQILAQV-NH2 | -{AEEA}₂-gGlu-C(O)(CH₂)₁₆COOH |
| 94 | Propanoyl-VLSLDVPIKLK*KILLEQEKQKKQREQAETNKQILAQV-NH2 | -{AEEA}₂-gGlu-C(O)(CH₂)₁₆COOH |
| 95 | Butanoyl-VLSLDVPIKLK*KILLEQEKQKKQREQAETNKQILAQV-NH2 | -{AEEA}₂-gGlu-C(O)(CH₂)₁₆COOH |
| 96 | Pentanoyl-VLSLDVPIKLK*KILLEQEKQKKQREQAETNKQILAQV-NH2 | -{AEEA}₂-gGlu-C(O)(CH₂)₁₆COOH |
| 97 | Ac-IVLSLDVPIKLK*KILLEQEKQKKQREQAETNAibQILAQV-NH2 | -{AEEA}₂-gGlu-C(O)(CH₂)₁₆COOH |
| 98 | Ac-IVLSLDvPIKLK*KILLEQEKQKKQREQAETNAibQILAQV-NH2 | -{AEEA}₂-gGlu-C(O)(CH₂)₁₆COOH |
| 99 | Ac-VLSLDvPIKLK*KILLEQEKQKKQREQAETNAibQILAQV-NH2 | -{AEEA}₂-gGlu-C(O)(CH₂)₁₆COOH |
| 100 | Ac-IVLSLDvPIKLK*KILLEQEKQKKQREQAETNAQILAQV-NH2 | -{AEEA}₂-gGlu-C(O)(CH₂)₁₆COOH |
| 101 | Ac-VLSLDvPIKLK*KILLEQEKQKKQREQAETNAQILAQV-NH2 | -{AEEA}₂-gGlu-C(O)(CH₂)₁₆COOH |
| 102 | Ac-IVLSLDVPIKLK*KILLEQEKQKKQREQAETNVQILAQV-NH2 | -{AEEA}₂-gGlu-C(O)(CH₂)₁₆COOH |
| 103 | Ac-IVLSLDvPIKLK*KILLEQEKQKKQREQAETNVQILAQV-NH2 | -{AEEA}₂-gGlu-C(O)(CH₂)₁₆COOH |
| 104 | Ac-IVLSLDvPIKLK*KILLEQEKQKKQREQAETNAbuQILAQV-NH2 | -{AEEA}₂-gGlu-C(O)(CH₂)₁₆COOH |
| 105 | Ac-IVLSLDvPIKLK*KILLEQEKQKKQREQAETNNvaQILAQV-NH2 | -{AEEA}₂-gGlu-C(O)(CH₂)₁₆COOH |
| 106 | Ac-IVLSLDvPIKLK*KILLEQEKQKKQREQAETNaMeKQILAQV-NH2 | -{AEEA}₂-gGlu-C(O)(CH₂)₁₆COOH |
| 107 | Ac-IVLSLDvPIKLK*KILLEQEKQKKQREQAETNDapQILAQV-NH2 | -{AEEA}₂-gGlu-C(O)(CH₂)₁₆COOH |
| 108 | Ac-IVLSLDvPIKLK*KILLEQEKQKKQREQAETNEQILAQV-NH2 | -{AEEA}₂-gGlu-C(O)(CH₂)₁₆COOH |
| 109 | Ac-IVLSLDvPIKLK*KILLEQEKQKKQREQAETN4-PipQILAQV-NH2 | -{AEEA}₂-gGlu-C(O)(CH₂)₁₆COOH |
| 110 | Ac-IVLSLDvPIKαMeLK*KILLEQEKQKKQREQAETNAIbDQILAQV-NH2 | -{AEEA}₂-gGlu-C(O)(CH₂)₁₆COOH |
| 111 | Ac-IVLSLDvPIKαMeLK*KILLEQEKQKKQREQAETNVQILAQV-NH2 | -{AEEA}₂-gGlu-C(O)(CH₂)₁₆COOH |
| 112 | Ac-IVLSLDvPIKαMeLK*KILLEQEKQKKQREQAETNAQILAQV-NH2 | -{AEEA}₂-gGlu-C(O)(CH₂)₁₆COOH |
| 113 | NMeIVLSLDvPIKLK*KILLEQEKQKKQREQAETNAQILAQV-NH2 | -{AEEA}₂-gGlu-C(O)(CH₂)₁₆COOH |
| 114 | NMeIVLSLDvPIKLK*KILLEQEKQKKQREQAETNAbuQILAQV-NH2 | -{AEEA}₂-gGlu-C(O)(CH₂)₁₆COOH |
| 115 | NMeIVLSLDvPIKLK*KILLEQEKQKKQREQAETNDapQILAQV-NH2 | -{AEEA}₂-gGlu-C(O)(CH₂)₁₆COOH |
| 116 (comp) | NMeIVLSLDvPIKLK*KILLEQEKQKKQREQAETNEQILAQV-NH2 | -{AEEA}₂-gGlu-C(O)(CH₂)₁₆COOH |
| 117 | NMeIVLSLDvPIKLK*KILLEQEKQKKQREQAETN4-PipQILAQV-NH2 | -{AEEA}₂-gGlu-C(O)(CH₂)₁₆COOH |
| 118 | NMeIVLSLDvPIKLK*KILLEQEKQKKQREQAETNAibQILAQV-NH2 | -{AEEA}₂-gGlu-C(O)(CH₂)₁₆COOH |
| 119 | NMeIVLSLDvPIKαMeLK*KILLEQEKQKKQREQAETNAibQILAQV-NH2 | -{AEEA}₂-gGlu-C(O)(CH₂)₁₆COOH |
| 120 | Pentanoyl-VLSLDvPIKLK*KILLEQEKQKKQREQAETNAibQILAQV-NH2 | -{AEEA}₂-gGlu-C(O)(CH₂)₁₆COOH |
| 121 | Pentanoyl-VLSLDvPIKLK*KILLEQEKQKKQREQAETNVQILAQV-NH2 | -{AEEA}₂-gGlu-C(O)(CH₂)₁₆COOH |
| 122 | Pentanoyl-VLSLDvPIKLK*KILLEQEKQKKQREQAETNAQILAQV-NH2 | -{AEEA}₂-gGlu-C(O)(CH₂)₁₆COOH |
| 123 | Pentanoyl-VLSLDvPIKαMeLK*KILLEQEKQKKQREQAETNAibQILAQV-NH2 | -{AEEA}₂-gGlu-C(O)(CH₂)₁₆COOH |
| 124 | Pentanoyl-VLSLDvPIKαMeLK*KILLEQEKQKKQREQAETNVQILAQV-NH2 | -{AEEA}₂-gGlu-C(O)(CH₂)₁₆COOH |
| 125 | Ac-IVLSLDvPIKLK*KILLEQEKQKKQREQAETNAibQILAQV-NH2 | -gGlu-C(O)(CH₂)₁₆COOH |
| 126 | Ac-IVLSLDvPIKLK*KILLEQEKQKKQREQAETNAQILAQV-NH2 | -gGlu-C(O)(CH₂)₁₆COOH |
| 127 | Ac-IVLSLDvPIKLK*KILLEQEKQKKQREQAETNAbuQILAQV-NH2 | -gGlu-C(O)(CH₂)₁₆COOH |
| 128 | Ac-IVLSLDvPIKLK*KILLEQEKQKKQREQAETNDapQILAQV-NH2 | -gGlu-C(O)(CH₂)₁₆COOH |
| 129 | Ac-IVLSLDvPIKLK*KILLEQEKQKKQREQAETN4-PipQILAQV-NH2 | -gGlu-C(O)(CH₂)₁₆COOH |
| 130 (comp) | Ac-IVLSLDvPIKLK*KILLEQEKQKKQREQAETNEQILAQV-NH2 | -gGlu-C(O)(CH₂)₁₆COOH |
| 131 | Ac-IVLSLDvPIKLK*KILLEQEKQKAQREQAETN4-PipQILAQV-NH2 | -gGlu-C(O)(CH₂)₁₆COOH |
| 132 | Ac-IVLSLDvPIKLK*KILLEQEKQKAibQREQAETN4-PipQILAQV-NH2 | -gGlu-C(O)(CH₂)₁₆COOH |
| 133 | Ac-IVLSLDdvPIKLK*KILLEQEKQKKQREQAETNThPQILAQV-NH2 | -gGlu-C(O)(CH₂)₁₆COOH |
| 134 | Ac-IVLSLDdvPIKLK*KILLEQEKQKKQREQAETNChxQILAQV-NH2 | -gGlu-C(O)(CH₂)₁₆COOH |
| 135 | Ac-IVLSLDdvPIKLK*KILLEQEKQKKQREQAETNCpexQILAQV-NH2 | -gGlu-C(O)(CH₂)₁₆COOH |
| 136 | Ac-IVLSLDdvPIKLK*KILLEQEKQKKQREQAETNCpxQILAQV-NH2 | -gGlu-C(O)(CH₂)₁₆COOH |
| 137 | NMeIVLSLDvPIKLK*KILLEQEKQKKQREQAETNAibQILAQV-NH2 | -gGlu-C(O)(CH₂)₁₆COOH |
| 138 | NMeIVLSLDvPIKLK*KILLEQEKQKKQREQAETNAQILAQV-NH2 | -gGlu-C(O)(CH₂)₁₆COOH |
| 139 | NMeIVLSLDvPIKLK*KILLEQEKQKKQREQAETNAbuQILAQV-NH2 | -gGlu-C(O)(CH₂)₁₆COOH |
| 140 | NMeIVLSLDvPIKLK*KILLEQEKQKKQREQAETNDapQILAQV-NH2 | -gGlu-C(O)(CH₂)₁₆COOH |
| 141 | NMeIVLSLDvPIKLK*KILLEQEKQKKQREQAETN4-PipQILAQV-NH2 | -gGlu-C(O)(CH₂)₁₆COOH |
| 142 (comp) | NMeIVLSLDvPIKLK*KILLEQEKQKKQREQAETNEQILAQV-NH2 | -gGlu-C(O)(CH₂)₁₆COOH |
| 143 | NMeIVLSLDvPIKLK*KILLEQEKQKKQREQAETNThPQILAQV-NH2 | -gGlu-C(O)(CH₂)₁₆COOH |
| 144 | NMeIVLSLDvPIKLK*KILLEQEKQKAQREQAETNAibQILAQV-NH2 | -gGlu-C(O)(CH₂)₁₆COOH |
| 145 | NMeIVLSLDvPIKLK*KILLEQEKQKAibQREQAETNAibQILAQV-NH2 | -gGlu-C(O)(CH₂)₁₆COOH |
| 146 (comp) | IVLSLDvPIKLK*QILLKQERQKKQREQAEKNKQILEQV-NH2 | -{AEEA}₂-{gGlu}₂-C(O)(CH₂)₁₄COOH |
| 147 (comp) | IVLSLDvPIKLK*QILLKQERQKKQREQAEKNKQILEQV-NH2 | -{gGlu}₂-C(O)(CH₂)₁₄COOH |
| 148 (comp) | IVLSLDvPIKLK*QILLKQERQKKQREQAEKNKQILEQV-NH2 | -{AEEA}₂-gGlu-C(O)(CH₂)₁₄COOH |
| 149 (comp) | IVLSLDVPIKLK*KILLEQEKQKKQREQAETNKQILAQV-NH2 | -{AEEA}₂-gGlu-C(O)(CH₂)₁₆COOH |
| 150 (comp) | IVLSLDvPIKLK*QILLKQERQKKQREQAEKNKQILEQV-NH2 | -{AEEA}₂-gGlu-C(O)(CH₂)₁₆COOH |
| 151 (comp) | IVLSLDvPIKLK*QILLKQERQKKQREQAEKNKQILEQV-NH2 | -gGlu-C(O)(CH₂)₁₄COOH |
| 152 | Ac-IVLSLDvPIKLK*KILLEQEKQKAQREQAETN4-PipQILAQV-NH2 | -gGlu-C(O)(CH₂)₁₄COOH |
| 153 | Ac-IVLSLDvPIKLK*KILLEQEKQKAibQREQAETN4-PipQILAQV-N H2 | -gGlu-C(O)(CH₂)₁₄COOH |
| 154 | NMeIVLSLDvPIKLK*KILLEQEKQKAQREQAETN4-PipQILAQV-NH2 | -gGlu-C(O)(CH₂)₁₄COOH |
| 155 | NMeIVLSLDvPIKLK*KILLEQEKQKAibQREQAETN4-PipQILAQV-NH2 | -gGlu-C(O)(CH₂)₁₄COOH |
| 156 | Hexanoyl-VLSLDvPIKLK*KILLEQEKQKKQREQAETNAibQILAQV-NH2 | -gGlu-C(O)(CH₂)₁₄COOH |
| 157 | Hexanoyl-VLSLDvPIKLK*KILLEQEKQKKQREQAETN4-PipQILAQV-NH2 | -gGlu-C(O)(CH₂)₁₄COOH |
| 158 | Decanoyl-VLSLDvPIKLK*KILLEQEKQKKQREQAETNAibQILAQV-NH2 | -gGlu-C(O)(CH₂)₁₄COOH |
| 159 | NMeVLSLDvPIKLK*KILLEQEKQKKQREQAETNAibQILAQV-NH2 | -gGlu-C(O)(CH₂)₁₄COOH |
| 160 | NMeVLSLDvPIKLK*KILLEQEKQKKQREQAETN4-PipQILAQV-NH2 | -gGlu-C(O)(CH₂)₁₄COOH |
| 161 | Ac-IVLSLDvPIKLK*KILLEQEKQKAQREQAETNAibQILAQV-NH2 | -gGlu-C(O)(CH₂)₁₄COOH |
| 162 | Ac-IVLSLDvPIKLK*KILLEQEKQKAibQREQAETNAibQILAQV-NH2 | -gGlu-C(O)(CH₂)₁₄COOH |
| 163 | Ac-IVLSLDvPIALK*KILLEQEKQKKQREQAETNAibQILAQV-NH2 | -gGlu-C(O)(CH₂)₁₄COOH |
| 164 | Ac-IVLSLDvPIKLK*AILLEQEKQKKQREQAETNAibQILAQV-NH2 | -gGlu-C(O)(CH₂)₁₄COOH |
| 165 | Ac-IVLSLDvPIKLK*KILLEQEAQKKQREQAETNAibQILAQV-NH2 | -gGlu-C(O)(CH₂)₁₄COOH |
| 166 | Ac-IVLSLDvPIKLK*KILLEQEKQAKQREQAETNAibQILAQV-NH2 | -gGlu-C(O)(CH₂)₁₄COOH |
| 167 | Ac-IVLSLDvPIKLK*KILLEQEKQKKQAEQAETNAibQILAQV-NH2 | -gGlu-C(O)(CH₂)₁₄COOH |
| 168 | Ac-IVLSLDvPIKLK*KILLEQEKQKEQREQAETNAibQILAQV-NH2 | -gGlu-C(O)(CH₂)₁₄COOH |
| 169 | Ac-IVLSLDvPIKLK*KILLEQEKQKAQREQAETN4-PipQILAQV-NH2 | -gGlu-C(O)(CH₂)₁₂COOH |
| 170 | Ac-IVLSLDvPIKLK*KILLEQEKQKAibQREQAETN4-PipQILAQV-N H2 | -gGlu-C(O)(CH₂)₁₂COOH |
| 171 | Ac-IVLSLDvPIKLK*KILLEQEKQKKQREQAETNAibQILAQV-NH2 | -{AEEA}₂-gGlu-C(O)(CH₂)₁₂COOH |
| 172 | Ac-IVLSLDvPIKLK*KILLEQEKQKKQREQAETN4-PipQILAQV-NH2 | -{AEEA}₂-gGlu-C(O)(CH₂)₁₂COOH |
| 173 | Ac-IVLSLDvPIKLK*KILLEQEKQKAibQREQAETN4-PipQILAQV-N H2 | -{AEEA}₂-gGlu-C(O)(CH₂)₁₂COOH |
| 174 | Ac-IVLSLDvPIKLK*KILLEQEKQKKQREQAETNAibQILAQV-NH2 | -{AEEA}₂-{gGlu}₂-C(O)(CH₂)₁₂COOH |
| 175 | Ac-IVLSLDvPIKLK*KILLEQEKQKKQREQAETN4-PipQILAQV-NH2 | -{AEEA}₂-{gGlu}₂-C(O)(CH₂)₁₂COOH |
| 176 | NMeIVLSLDvPIKLK*KILLEQEKQKAQREQAETNAibQILAQV-NH2 | -gGlu-C(O)(CH₂)₁₂COOH |
| 177 | NMeIVLSLDvPIKLK*KILLEQEKQKAibQREQAETNAibQILAQV-NH2 | -gGlu-C(O)(CH₂)₁₂COOH |
| 178 | NMeIVLSLDvPIKLK*KILLEQEKQKKQREQAETNAibQILAQV-NH2 | -{AEEA}₂-gGlu-C(O)(CH₂)₁₂COOH |
| 179 | NMeIVLSLDvPIKLK*KILLEQEKQKKQREQAETNAibQILAQV-NH2 | -{AEEA}₂-{gGlu}₂-C(O)(CH₂)₁₂COOH |
| 180 | Pentanoyl-VLSLDvPIKLK*KILLEQEKQKKQREQAETNAibQILAQV-NH2 | -{AEEA}₂-gGlu-C(O)(CH₂)₁₂COOH |
| 181 | Pentanoyl-VLSLDvPIKLK*KILLEQEKQKKQREQAETNAibQILAQV-NH2 | -{AEEA}₂-{gGlu}₂-C(O)(CH₂)₁₂COOH |
| 182 | Ac-IVLSLDvPIKLK*KILLEQEKQKKQREQAETNAibQILAQV-NH2 | -gGlu-C(O)(CH₂)₁₀COOH |
| 183 | Ac-IVLSLDvPIKLK*KILLEQEKQKKQREQAETN4-PipQILAQV-NH2 | -gGlu-C(O)(CH₂)₁₀COOH |
| 184 | Ac-IVLSLDvPIKLK*KILLEQEKQKAibQREQAETN4-PipQILAQV-N H2 | -gGlu-C(O)(CH₂)₁₀COOH |
| 185 | Pentanoyl-VLSLDvPIKLK*KILLEQEKQKKQREQAETNAibQILAQV-NH2 | -gGlu-C(O)(CH₂)₁₀COOH |
| 186 | NMeIVLSLDvPIKLK*KILLEQEKQKKQREQAETNAibQILAQV-NH2 | -gGlu-C(O)(CH₂)₁₀COOH |
| 187 | Ac-IVLSLDvPIKLK*KILLEQEKQKAQREQAETN4-PipQILAQV-NH2 | -gGlu-C(O)(CH₂)₁₆COOH |
| 188 | Ac-IVLSLDvPIKLK*KILLEQEKQKAibQREQAETN4-PipQILAQV-NH2 | -gGlu-C(O)(CH₂)₁₆COOH |
| 189 | Ac-IVLSLDvPIKLK*KILLEQEKQKAQREQAETNAibQILAQV-NH2 | -gGlu-C(O)(CH₂)₁₆COOH |
| 190 | Ac-IVLSLDvPIKLK*KILLEQEKQKAibQREQAETNAibQILAQV-NH2 | -gGlu-C(O)(CH₂)₁₆COOH |
| 191 | Ac-IVLSLDvPIGLK*KILLEQEKQKKQREQAETNAibQILAQV-NH2 | -gGlu-C(O)(CH₂)₁₆COOH |
| 192 | NMeIVLSLDvPIGLK*KILLEQEKQKKQREQAETNAibQILAQV-NH2 | -gGlu-C(O)(CH₂)₁₆COOH |
| 193 | Pentanoyl-VLSLDvPIKLK*KILLEQEKQKKQREQAETNAibQILAQV-NH2 | -gGlu-C(O)(CH₂)₁₆COOH |
| 194 | Pentanoyl-VLSLDvPIKLK*KILLEQEKQKAibQREQAETNAibQILAQV-NH2 | -gGlu-C(O)(CH₂)₁₆COOH |
| 195 | Pentanoyl-VLSLDvPIGLK*KILLEQEKQKKQREQAETNAibQILAQV-NH2 | -gGlu-C(O)(CH₂)₁₆COOH |
| 196 | Ac-IVLSLDvPIKLK*KILLEQEKQKAibQREQAETN4-PipQILAQV-N H2 | -{AEEA}₂-gGlu-C(O)(CH₂)₁₆COOH |
| 197 | Ac-IVLSLDvPIKLK*KILLEQEKQKAibQREQAETNAibQILAQV-NH2 | -{AEEA}₂-gGlu-C(O)(CH₂)₁₆COOH |
| 198 | Ac-IVLSLDvPIKLK*KILLEQEKQKKQREQAETNThpQILAQV-NH2 | -{AEEA}₂-gGlu-C(O)(CH₂)₁₆COOH |
| 199 | Ac-IVLSLDvPIKLK*KILLEQEKQKKQREQAETNThpQILAQV-NH2 | -gGlu-C(O)(CH₂)₁₆COOH |
| 200 | NMeIVLSLDvPIKLK*KILLEQEKQKAibQREQAETNAibQILAQV-NH2 | -{AEEA}₂-gGlu-C(O)(CH₂)₁₆COOH |
| 201 | Pentanoyl-VLSLDvPIKLK*KILLEQEKQKKQREQAETNAibQILAQV-NH2 | -{AEEA}₂-gGlu-C(O)(CH₂)₁₆COOH |
| 202 | Pentanoyl-VLSLDvPIKLK*KILLEQEKQKAibQREQAETNAibQILAQV-NH2 | -{AEEA}₂-gGlu-C(O)(CH₂)₁₆COOH |
| 203 | Ac-IVLSLDvPIKLK*KILLEQEKQKKQREQAETNAibQILAQV-NH2 | -{AEEA}-gGlu-C(O)(CH₂)₁₆COOH |
| 204 | Ac-IVLSLDvPIKLK*KILLEQEKQKKQREQAETNAibQILAQV-NH2 | -{AEEA}₂-{gGlu}₂-C(O)(CH₂)₁₆COOH |
| 205 | Ac-IVLSLDvPIKLK*KILLEQEKQKKQREQAETNAibQILAQV-NH2 | -{AEEA}-{gGlu}₂-C(O)(CH₂)₁₆COOH |
| 206 | Ac-IVLSLDvPIKLK*KILLEQEKQKKQREQAETNAibQILAQV-NH2 | -gGlu-C(O)(CH₂)₁₈COOH |
| 207 | Ac-IVLSLDvPIKLK*KILLEQEKQKKQREQAETN4-PipQILAQV-NH2 | -gGlu-C(O)(CH₂)₁₈COOH |
| 208 | Ac-IVLSLDvPIKLK*KILLEQEKQKAibQREQAETN4-PipQILAQV-N H2 | -gGlu-C(O)(CH₂)₁₈COOH |
| 209 | Ac-IVLSLDvPIKLK*KILLEQEKQKAibQREQAETNAibQILAQV-NH2 | -gGlu-C(O)(CH₂)₁₈COOH |
| 210 | Ac-IVLSLDvPIKLK*KILLEQEKQKKQREQAETNThpQILAQV-NH2 | -gGlu-C(O)(CH₂)₁₈COOH |
| 211 | Ac-IVLSLDvPIKLK*KILLEQEAQKKQREQAETNAibQILAQV-NH2 | -gGlu-C(O)(CH₂)₁₈COOH |
| 212 | Ac-IVLSLDvPIKLK*KILLEQEKQKAQREQAETNAibQILAQV-NH2 | -gGlu-C(O)(CH₂)₁₈COOH |
| 213 | Ac-IVLSLDvPIKLK*KILLEQEKQKEQREQAETNAibQILAQV-NH2 | -gGlu-C(O)(CH₂)₁₈COOH |
| 214 | NMeIVLSLDvPIKLK*KILLEQEKQKKQREQAETNAibQILAQV-NH2 | -gGlu-C(O)(CH₂)₁₈COOH |
| 215 | NMeIVLSLDvPIKLK*KILLEQEKQKAibQREQAETNAibQILAQV-NH2 | -gGlu-C(O)(CH₂)₁₈COOH |
| 216 | NMeIVLSLDvPIKLK*KILLEQEAQKKQREQAETNAibQILAQV-NH2 | -gGlu-C(O)(CH₂)₁₈COOH |
| 217 | NMeIVLSLDvPIKLK*KILLEQEKQKAQREQAETNAibQILAQV-NH2 | -gGlu-C(O)(CH₂)₁₈COOH |
| 218 | NMeIVLSLDvPIKLK*KILLEQEKQKEQREQAETNAibQILAQV-NH2 | -gGlu-C(O)(CH₂)₁₈COOH |
| 219 | Pentanoyl-VLSLDvPIKLK*KILLEQEKQKKQREQAETNAibQILAQV-NH2 | -gGlu-C(O)(CH₂)₁₈COOH |
| 220 | Pentanoyl-VLSLDvPIKLK*KILLEQEKQKAibQREQAETNAibQILAQV-NH2 | -gGlu-C(O)(CH₂)₁₈COOH |
| 221 | Pentanoyl-VLSLDvPIKLK*KILLEQEAQKKQREQAETNAibQILAQV-NH2 | -gGlu-C(O)(CH₂)₁₈COOH |
| 222 | Pentanoyl-VLSLDvPIKLK*KILLEQEKQKAQREQAETNAibQILAQV-NH2 | -gGlu-C(O)(CH₂)₁₈COOH |
| 223 | Decanoyl-VLSLDvPIKLK*KILLEQEKQKKQREQAETNAibQILAQV-NH2 | -gGlu-C(O)(CH₂)₁₈COOH |
| 224 | Dadecanayl-VLSLDvPIKLK*KILLEQEKQKKQREQAETNAibQILAQV-NH2 | -gGlu-C(O)(CH₂)₁₈COOH |
| 225 | Tetradecanoyl-VLSLDvPIKLK*KILLEQEKQKKQREQAETNAibQILAQV-NH2 | -gGlu-C(O)(CH₂)₁₈COOH |
| 226 | Ac-IVLSLDvPIGLK*KILLEQEKQKKQREQAETNAibQILAQV-NH2 | -gGlu-C(O)(CH₂)₁₈COOH |
| 227 | Ac-IVLSLDvPIGLK*KILLEQEKQKKQREQAETN4-PipQILAQV-NH2 | -gGlu-C(O)(CH₂)₁₈COOH |
| 228 | Pentanoyl-VLSLDvPIGLK*KILLEQEKQKKQREQAETNAibQILAQV-NH2 | -gGlu-C(O)(CH₂)₁₈COOH |
| 229 | NMeIVLSLDvPIGLK*KILLEQEKQKKQREQAETNAibQILAQV-NH2 | -gGlu-C(O)(CH₂)₁₈COOH |
| 230 | Ac-IVLSLDvPIKLK*KILLEQEKQKKQREQAETNAibQILAQV-NH2 | -{AEEA}-gGlu-C(O)(CH₂)₁₈COOH |
| 231 | Ac-IVLSLDvPIKLK*KILLEQEKQKEQREQAETNAibQILAQV-NH2 | -{AEEA}-gGlu-C(O)(CH₂)₁₈COOH |
| 232 | Ac-IVLSLDvPIKLK*KILLEQEKQKAQREQAETNAibQILAQV-NH2 | -{AEEA}-gGlu-C(O)(CH₂)₁₈COOH |
| 233 | NMeIVLSLDvPIKLK*KILLEQEKQKEQREQAETNAibQILAQV-NH2 | -{AEEA}-gGlu-C(O)(CH₂)₁₈COOH |
| 234 | NMeIVLSLDvPIKLK*KILLEQEKQKAibQREQAETNAibQILAQV-NH2 | -{AEEA}-gGlu-C(O)(CH₂)₁₈COOH |
| 235 | Pentanoyl-VLSLDvPIKLK*KILLEQEKQKKQREQAETNAibQILAQV-NH2 | -{AEEA}-gGlu-C(O)(CH₂)₁₈COOH |
| 236 | Ac-IVLSLDvPIKLK*KILLEQEKQKEQREQAETNAibQILAQV-NH2 | -{AEEA}₂-gGlu-C(O)(CH₂)₁₈COOH |
| 237 | NMeIVLSLDvPIKLK*KILLEQEKQKEQREQAETNAibQILAQV-NH2 | -{AEEA}₂-gGlu-C(O)(CH₂)₁₈COOH |
| 238 | Ac-IVLSLDvPIKLK*KILLEQEKQKKQREQAETNAibQILAQV-NH2 | -{AEEA}₂-gGlu-C(O)(CH₂)₁₈COOH |
| 239 | NMeIVLSLDvPIKLK*KILLEQEKQKAibQREQAETNAibQILAQV-NH2 | -{AEEA}₂-gGlu-C(O)(CH₂)₁₈COOH |
| 240 | Ac-IVLSLDvPIKLK*KILLEQEKQKAQREQAETNAibQILAQV-NH2 | -{AEEA}₂-gGlu-C(O)(CH₂)₁₈COOH |
| 241 | Ac-IVLSLDvPIKLK*KILLEQEKQKEQREQAETNAibQILAQV-NH2 | -{gGlu}₂-C(O)(CH₂)₁₈COOH |
| 242 | NMeIVLSLDvPIKLK*KILLEQEKQKEQREQAETNAibQILAQV-NH2 | -{gGlu}₂-C(O)(CH₂)₁₈COOH |
| 243 | Ac-IVLSLDvPIKLK*KILLEQEKQKKQREQAETNAibQILAQV-NH2 | -{gGlu}₂-C(O)(CH₂)₁₈COOH |
| 244 | Ac-IVLSLDvPIKLK*KILLEQEKQKAQREQAETNAibQILAQV-NH2 | -{gGlu}₂-C(O)(CH₂)₁₈COOH |

In an embodiment, the compound is a peptide of the following amino acid sequence (SEQ ID NO: 19):
Ac-IVLSLDVPIKαMeLK*QILLKQERQKKQREQAEKNAibQILEQV-NH2
wherein
the K residue at position 12 (denoted as K*) is chemically modified such that the epsilon-amino group of its side chain is covalently bound to -gGlu-C(O)(CH₂)₁₄COOH;
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 33):
Ac-VLSLDvPIKLK*KILLEQEKQKKQREQAETNKQILAQV-NH2
wherein
the K residue at position 11 (denoted as K*) is chemically modified such that the epsilon-amino group of its side chain is covalently bound to -gGlu-C(O)(CH₂)₁₄COOH;
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 34):
VLSLDvPIKLK*KILLEQEKQKKQREQAETNKQILAQV-NH2
wherein
the K residue at position 11 (denoted as K*) is chemically modified such that the epsilon-amino group of its side chain is covalently bound to -gGlu-C(O)(CH₂)₁₄COOH;
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 40):
Ac-IVLSLDvPIKLK*KILLEQEKQKKQREQAETNAibQILAQV-NH2
wherein
the K residue at position 12 (denoted as K*) is chemically modified such that the epsilon-amino group of its side chain is covalently bound to -gGlu-C(O)(CH₂)₁₄COOH;
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 43):
Ac-IVLSLDVPIKαMeLK*KILLEQEKQKKQREQAETNAibQILAQV-NH2
wherein
the K residue at position 12 (denoted as K*) is chemically modified such that the epsilon-amino group of its side chain is covalently bound to -gGlu-C(O)(CH₂)₁₄COOH;
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 47):
Ac-IVLSLDvPIKLK*KILLEQEKQKKQREQAETNAQILAQV-NH2
wherein
the K residue at position 12 (denoted as K*) is chemically modified such that the epsilon-amino group of its side chain is covalently bound to -gGlu-C(O)(CH₂)₁₄COOH;
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 51):
Ac-IVLSLDvPIKLK*KILLEQEKQKKQREQAETNAbuQILAQV-NH2
wherein
the K residue at position 12 (denoted as K*) is chemically modified such that the epsilon-amino group of its side chain is covalently bound to -gGlu-C(O)(CH₂)₁₄COOH;
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 54):
Ac-IVLSLDvPIKLK*KILLEQEKQKKQREQAETNαMeKQILAQV-NH2
wherein
the K residue at position 12 (denoted as K*) is chemically modified such that the epsilon-amino group of its side chain is covalently bound to -gGlu-C(O)(CH₂)₁₄COOH;
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 55):
Ac-IVLSLDvPIKLK*KILLEQEKQKKQREQAETNDapQILAQV-NH2
wherein
the K residue at position 12 (denoted as K*) is chemically modified such that the epsilon-amino group of its side chain is covalently bound to -gGlu-C(O)(CH₂)₁₄COOH;
-NH2 denotes that the C-terminal amino acid residue is amidated as a primary amide; and
Ac- denotes that the N-terminal amino acid residue is acetylated;
or a pharmaceutically acceptable salt thereof.

In a comparative embodiment, the compound is a peptide of the following amino acid sequence (SEQ ID NO: 56):
Ac-IVLSLDvPIKLK*KILLEQEKQKKQREQAETNEQILAQV-NH2
wherein
the K residue at position 12 (denoted as K*) is chemically modified such that the epsilon-amino group of its side chain is covalently bound to -gGlu-C(O)(CH₂)₁₄COOH;
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 57):
Ac-IVLSLDvPIKLK*KILLEQEKQKKQREQAETN4-PipQILAQV-NH2
wherein
the K residue at position 12 (denoted as K*) is chemically modified such that the epsilon-amino group of its side chain is covalently bound to -gGlu-C(O)(CH₂)₁₄COOH;
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 64):
NMeIVLSLDvPIKLK*KILLEQEKQKKQREQAETNAibQILAQV-NH2
wherein
the K residue at position 12 (denoted as K*) is chemically modified such that the epsilon-amino group of its side chain is covalently bound to -gGlu-C(O)(CH₂)₁₄COOH;
-NH2 denotes that the C-terminal amino acid residue is amidated as a primary amide; and
NMe- denotes that the N-terminal amino acid residue is N-methylated;
or a pharmaceutically acceptable salt thereof.

In an embodiment, the compound is a peptide of the following amino acid sequence (SEQ ID NO: 80):
Pentanoyl-VLSLDvPIKLK*KILLEQEKQKKQREQAETNAibQILAQV-NH2
wherein
the K residue at position 11 (denoted as K*) is chemically modified such that the epsilon-amino group of its side chain is covalently bound to -gGlu-C(O)(CH₂)₁₄COOH;
-NH2 denotes that the C-terminal amino acid residue is amidated as a primary amide; and
Pentanoyl- denotes that the N-terminal amino acid residue is acylated with a pentanoyl group;
or a pharmaceutically acceptable salt thereof.

In an embodiment, the compound is a peptide of the following amino acid sequence (SEQ ID NO: 92):
Ac-VLSLDvPIKLK*KILLEQEKQKKQREQAETNKQILAQV-NH2
wherein
the K residue at position 11 (denoted as K*) is chemically modified such that the epsilon-amino group of its side chain is covalently bound to -{AEEA}2-gGlu-C(O)(CH₂)₁₆COOH;
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 93):
VLSLDvPIKLK*KILLEQEKQKKQREQAETNKQILAQV-NH2
wherein
the K residue at position 11 (denoted as K*) is chemically modified such that the epsilon-amino group of its side chain is covalently bound to -{AEEA}2-gGlu-C(O)(CH₂)₁₆COOH;
-NH2 denotes that the C-terminal amino acid residue is amidated as a primary amide;
or a pharmaceutically acceptable salt thereof.

In an embodiment, the compound is a peptide of the following amino acid sequence (SEQ ID NO: 96):
Butanoyl-VLSLDVPIKLK*KILLEQEKQKKQREQAETNKQILAQV-NH2
wherein
the K residue at position 11 (denoted as K*) is chemically modified such that the epsilon-amino group of its side chain is covalently bound to -{AEEA}2-gGlu-C(O)(CH₂)₁₆COOH;
-NH2 denotes that the C-terminal amino acid residue is amidated as a primary amide; and
Butanoyl- denotes that the N-terminal amino acid residue is acylated with a butanoyl group;
or a pharmaceutically acceptable salt thereof.

In an embodiment, the compound is a peptide of the following amino acid sequence (SEQ ID NO: 100):
Ac-IVLSLDvPIKLK*KILLEQEKQKKQREQAETNAQILAQV-NH2
wherein
the K residue at position 12 (denoted as K*) is chemically modified such that the epsilon-amino group of its side chain is covalently bound to -{AEEA}₂-gGlu-C(O)(CH₂)₁₆COOH; and
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 104):
Ac-IVLSLDvPIKLK*KILLEQEKQKKQREQAETNAbuQILAQV-NH2
wherein
the K residue at position 12 (denoted as K*) is chemically modified such that the epsilon-amino group of its side chain is covalently bound to -{AEEA}₂-gGlu-C(O)(CH₂)₁₆COOH; and
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 110):
Ac-IVLSLDvPIKαMeLK*KILLEQEKQKKQREQAETNAibQILAQV-NH2
wherein
the K residue at position 12 (denoted as K*) is chemically modified such that the epsilon-amino group of its side chain is covalently bound to -{AEEA}₂-gGlu-C(O)(CH₂)₁₆COOH; and
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 113):
NMeIVLSLDvPIKLK*KILLEQEKQKKQREQAETNAQILAQV-NH2
wherein
the K residue at position 12 (denoted as K*) is chemically modified such that the epsilon-amino group of its side chain is covalently bound to -{AEEA}2-gGlu-C(O)(CH₂)₁₆COOH; and
-NH2 denotes that the C-terminal amino acid residue is amidated as a primary amide; and
NMe- denotes that the N-terminal amino acid residue is N-methylated;
or a pharmaceutically acceptable salt thereof.

In an embodiment, the compound is a peptide of the following amino acid sequence (SEQ ID NO: 118):
NMeIVLSLDvPIKLK*KILLEQEKQKKQREQAETNAibQILAQV-NH2
wherein
the K residue at position 12 (denoted as K*) is chemically modified such that the epsilon-amino group of its side chain is covalently bound to -{AEEA}₂-gGlu-C(O)(CH₂)₁₆COOH; and
-NH2 denotes that the C-terminal amino acid residue is amidated as a primary amide; and
NMe- denotes that the N-terminal amino acid residue is N-methylated;
or a pharmaceutically acceptable salt thereof.

In an embodiment, the compound is a peptide of the following amino acid sequence (SEQ ID NO: 125):
Ac-IVLSLDvPIKLK*KILLEQEKQKKQREQAETNAibQILAQV-NH2
wherein
the K residue at position 12 (denoted as K*) is chemically modified such that the epsilon-amino group of its side chain is covalently bound to -gGlu-C(O)(CH₂)₁₄COOH;
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 131):
Ac-IVLSLDvPIKLK*KILLEQEKQKAQREQAETN4PipQILAQV-NH2
wherein
the K residue at position 12 (denoted as K*) is chemically modified such that the epsilon-amino group of its side chain is covalently bound to -gGlu-C(O)(CH₂)₁₆COOH;
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 132):
Ac-IVLSLDvPIKLK*KILLEQEKQKAibQREQAETN4PipQILAQV-NH2
wherein
the K residue at position 12 (denoted as K*) is chemically modified such that the epsilon-amino group of its side chain is covalently bound to -gGlu-C(O)(CH₂)₁₆COOH;
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 136):
Ac-IVLSLDdvPIKLK*KILLEQEKQKKQREQAETNCpxQILAQV-NH2
wherein
the K residue at position 12 (denoted as K*) is chemically modified such that the epsilon-amino group of its side chain is covalently bound to -gGlu-C(O)(CH₂)₁₆COOH;
-NH2 denotes that the C-terminal amino acid residue is amidated as a primary amide; and
Ac- denotes that the N-terminal amino acid residue is acetylated;
or a pharmaceutically acceptable salt thereof.

In an embodiment, the compound is a peptide of the following amino acid sequence (SEQ ID NO: 144):
NMeIVLSLDvPIKLK*KILLEQEKQKAQREQAETNAibQILAQV-NH2
wherein
the K residue at position 12 (denoted as K*) is chemically modified such that the epsilon-amino group of its side chain is covalently bound to -gGlu-C(O)(CH₂)₁₆COOH;
-NH2 denotes that the C-terminal amino acid residue is amidated as a primary amide; and
NMe- denotes that the N-terminal amino acid residue is N-methylated;
or a pharmaceutically acceptable salt thereof.

In an embodiment, the compound is a peptide of the following amino acid sequence (SEQ ID NO: 145):
NMeIVLSLDvPIKLK*KILLEQEKQKAibQREQAETNAibQILAQV-NH2
wherein
the K residue at position 12 (denoted as K*) is chemically modified such that the epsilon-amino group of its side chain is covalently bound to -gGlu-C(O)(CH₂)₁₆COOH;
-NH2 denotes that the C-terminal amino acid residue is amidated as a primary amide; and
NMe- denotes that the N-terminal amino acid residue is N-methylated;
or a pharmaceutically acceptable salt thereof.

In an embodiment, the compound is a peptide of the following amino acid sequence (SEQ ID NO: 189):
Ac-IVLSLDvPIKLK*KILLEQEKQKAQREQAETNAibQILAQV-NH2
wherein
the K residue at position 12 (denoted as K*) is chemically modified such that the epsilon-amino group of its side chain is covalently bound to -gGlu-C(O)(CH₂)₁₆COOH;
-NH2 denotes that the C-terminal amino acid residue is amidated as a primary amide; and
Ac- denotes that the N-terminal amino acid residue is Acetylated
or a pharmaceutically acceptable salt thereof.

In an embodiment, the compound is a peptide of the following amino acid sequence (SEQ ID NO: 190):
Ac-IVLSLDvPIKLK*KILLEQEKQKAibQREQAETNAibQILAQV-NH2
wherein
the K residue at position 12 (denoted as K*) is chemically modified such that the epsilon-amino group of its side chain is covalently bound to -gGlu-C(O)(CH₂)₁₆COOH;
-NH2 denotes that the C-terminal amino acid residue is amidated as a primary amide; and
Ac- denotes that the N-terminal amino acid residue is Acetylated
or a pharmaceutically acceptable salt thereof.

In an embodiment, the compound is a peptide of the following amino acid sequence (SEQ ID NO: 193):
Pentanoyl-VLSLDvPIKLK*KILLEQEKQKKQREQAETNAibQILAQV-NH2
wherein
the K residue at position 11 (denoted as K*) is chemically modified such that the epsilon-amino group of its side chain is covalently bound to -gGlu-C(O)(CH₂)₁₆COOH;
-NH2 denotes that the C-terminal amino acid residue is amidated as a primary amide; and
Pentanoyl- denotes that the N-terminal amino acid residue is acylated with a pentanoyl group;
or a pharmaceutically acceptable salt thereof.

In an embodiment, the compound is a peptide of the following amino acid sequence (SEQ ID NO: 203):
Ac-IVLSLDvPIKLK*KILLEQEKQKKQREQAETNAibQILAQV-NH2
wherein
the K residue at position 12 (denoted as K*) is chemically modified such that the epsilon-amino group of its side chain is covalently bound to -{AEEA}-gGlu-C(O)(CH₂)₁₆COOH;
-NH2 denotes that the C-terminal amino acid residue is amidated as a primary amide; and
Ac- denotes that the N-terminal amino acid residue is acetylated
or a pharmaceutically acceptable salt thereof.

In an embodiment, the compound is a peptide of the following amino acid sequence (SEQ ID NO: 206):
Ac-IVLSLDvPIKLK*KILLEQEKQKKQREQAETNAibQILAQV-NH2
wherein
the K residue at position 12 (denoted as K*) is chemically modified such that the epsilon-amino group of its side chain is covalently bound to -gGlu-C(O)(CH₂)₁₈COOH;
-NH2 denotes that the C-terminal amino acid residue is amidated as a primary amide; and
Ac- denotes that the N-terminal amino acid residue is Acetylated;
or a pharmaceutically acceptable salt thereof.

In an embodiment, the compound is a peptide of the following amino acid sequence (SEQ ID NO: 210):
Ac-IVLSLDvPIKLK*KILLEQEKQKKQREQAETNThpQILAQV-NH2
wherein
the K residue at position 12 (denoted as K*) is chemically modified such that the epsilon-amino group of its side chain is covalently bound to -gGlu-C(O)(CH₂)₁₈COOH;
-NH2 denotes that the C-terminal amino acid residue is amidated as a primary amide; and
Ac- denotes that the N-terminal amino acid residue is Acetylated;
or a pharmaceutically acceptable salt thereof.

In an embodiment, the compound is a peptide of the following amino acid sequence (SEQ ID NO: 212):
Ac-IVLSLDvPIKLK*KILLEQEKQKAQREQAETNAibQILAQV-NH2
wherein
the K residue at position 12 (denoted as K*) is chemically modified such that the epsilon-amino group of its side chain is covalently bound to -gGlu-C(O)(CH₂)₁₈COOH;
-NH2 denotes that the C-terminal amino acid residue is amidated as a primary amide; and
Ac- denotes that the N-terminal amino acid residue is Acetylated;
or a pharmaceutically acceptable salt thereof.

In an embodiment, the compound is a peptide of the following amino acid sequence (SEQ ID NO: 213):
Ac-IVLSLDvPIKLK*KILLEQEKQKEQREQAETNAibQILAQV-NH2
wherein
the K residue at position 12 (denoted as K*) is chemically modified such that the epsilon-amino group of its side chain is covalently bound to -gGlu-C(O)(CH₂)₁₈COOH;
-NH2 denotes that the C-terminal amino acid residue is amidated as a primary amide; and
Ac- denotes that the N-terminal amino acid residue is Acetylated;
or a pharmaceutically acceptable salt thereof.

In an embodiment, the compound is a peptide of the following amino acid sequence (SEQ ID NO: 215):
NMeIVLSLDvPIKLK*KILLEQEKQKAibQREQAETNAibQILAQV-NH2
wherein
the K residue at position 12 (denoted as K*) is chemically modified such that the epsilon-amino group of its side chain is covalently bound to -gGlu-C(O)(CH₂)₁₈COOH;
-NH2 denotes that the C-terminal amino acid residue is amidated as a primary amide; and
NMe- denotes that the N-terminal amino acid residue is N-methylated;
or a pharmaceutically acceptable salt thereof.

In an embodiment, the compound is a peptide of the following amino acid sequence (SEQ ID NO: 217):
NMeIVLSLDvPIKLK*KILLEQEKQKAQREQAETNAibQILAQV-NH2
wherein
the K residue at position 12 (denoted as K*) is chemically modified such that the epsilon-amino group of its side chain is covalently bound to -gGlu-C(O)(CH₂)₁₈COOH;
-NH2 denotes that the C-terminal amino acid residue is amidated as a primary amide; and
NMe- denotes that the N-terminal amino acid residue is N-methylated;
or a pharmaceutically acceptable salt thereof.

In an embodiment, the compound is a peptide of the following amino acid sequence (SEQ ID NO: 218):
NMeIVLSLDvPIKLK*KILLEQEKQKEQREQAETNAibQILAQV-NH2
wherein
the K residue at position 12 (denoted as K*) is chemically modified such that the epsilon-amino group of its side chain is covalently bound to -gGlu-C(O)(CH₂)₁₈COOH;
-NH2 denotes that the C-terminal amino acid residue is amidated as a primary amide; and
NMe- denotes that the N-terminal amino acid residue is N-methylated;
or a pharmaceutically acceptable salt thereof.

In an embodiment, the compound is a peptide of the following amino acid sequence (SEQ ID NO: 219):
Pentanoyl-VLSLDvPIKLK*KILLEQEKQKKQREQAETNAibQILAQV-NH2
wherein
the K residue at position 11 (denoted as K*) is chemically modified such that the epsilon-amino group of its side chain is covalently bound to -gGlu-C(O)(CH₂)₁₈COOH;
-NH2 denotes that the C-terminal amino acid residue is amidated as a primary amide; and
Pentanoyl- denotes that the N-terminal amino acid residue is acylated with a pentanoyl group;
or a pharmaceutically acceptable salt thereof.

In an embodiment, the compound is a peptide of the following amino acid sequence (SEQ ID NO: 222):
Pentanoyl-VLSLDvPIKLK*KILLEQEKQKAQREQAETNAibQILAQV-NH2
wherein
the K residue at position 11 (denoted as K*) is chemically modified such that the epsilon-amino group of its side chain is covalently bound to -gGlu-C(O)(CH₂)₁₈COOH;
-NH2 denotes that the C-terminal amino acid residue is amidated as a primary amide; and
Pentanoyl- denotes that the N-terminal amino acid residue is acylated with a pentanoyl group;
or a pharmaceutically acceptable salt thereof.

In an embodiment, the compound is a peptide of the following amino acid sequence (SEQ ID NO: 230):
Ac-IVLSLDvPIKLK*KILLEQEKQKKQREQAETNAibQILAQV-NH2
wherein
the K residue at position 12 (denoted as K*) is chemically modified such that the epsilon-amino group of its side chain is covalently bound to -{AEEA}-gGlu-C(O)(CH₂)₁₈COOH;
-NH2 denotes that the C-terminal amino acid residue is amidated as a primary amide; and
Ac- denotes that the N-terminal amino acid residue is Acetylated;
or a pharmaceutically acceptable salt thereof.

In an embodiment, the compound is a peptide of the following amino acid sequence (SEQ ID NO: 231):
Ac-IVLSLDvPIKLK*KILLEQEKQKEQREQAETNAibQILAQV-NH2
wherein
the K residue at position 12 (denoted as K*) is chemically modified such that the epsilon-amino group of its side chain is covalently bound to -{AEEA}-gGlu-C(O)(CH₂)₁₈COOH;
-NH2 denotes that the C-terminal amino acid residue is amidated as a primary amide; and
Ac- denotes that the N-terminal amino acid residue is Acetylated;
or a pharmaceutically acceptable salt thereof.

In an embodiment, the compound is a peptide of the following amino acid sequence (SEQ ID NO: 233):
NMeIVLSLDvPIKLK*KILLEQEKQKEQREQAETNAibQILAQV-NH2
wherein
the K residue at position 12 (denoted as K*) is chemically modified such that the epsilon-amino group of its side chain is covalently bound to -{AEEA}-gGlu-C(O)(CH₂)₁₈COOH;
-NH2 denotes that the C-terminal amino acid residue is amidated as a primary amide; and
NMe- denotes that the N-terminal amino acid residue is N-methylated;
or a pharmaceutically acceptable salt thereof.

In an embodiment, the compound is a peptide of the following amino acid sequence (SEQ ID NO: 234):
NMeIVLSLDvPIKLK*KILLEQEKQKAibQREQAETNAibQILAQV-NH2
wherein
the K residue at position 12 (denoted as K*) is chemically modified such that the epsilon-amino group of its side chain is covalently bound to -{AEEA}-gGlu-C(O)(CH₂)₁₈COOH;
-NH2 denotes that the C-terminal amino acid residue is amidated as a primary amide; and
NMe- denotes that the N-terminal amino acid residue is N-methylated;
or a pharmaceutically acceptable salt thereof.

In an embodiment, the compound is a peptide of the following amino acid sequence (SEQ ID NO: 235):
Pentanoyl-VLSLDvPIKLK*KILLEQEKQKKQREQAETNAibQILAQV-NH2
wherein
the K residue at position 11 (denoted as K*) is chemically modified such that the epsilon-amino group of its side chain is covalently bound to -{AEEA}-gGlu-C(O)(CH₂)₁₈COOH;
-NH2 denotes that the C-terminal amino acid residue is amidated as a primary amide; and
Pentanoyl- denotes that the N-terminal amino acid residue is acylated with a pentanoyl group;
or a pharmaceutically acceptable salt thereof.

In an embodiment, the compound is a peptide of the following amino acid sequence (SEQ ID NO: 236):
Ac-IVLSLDvPIKLK*KILLEQEKQKEQREQAETNAibQILAQV-NH2
wherein
the K residue at position 12 (denoted as K*) is chemically modified such that the epsilon-amino group of its side chain is covalently bound to -{AEEA}₂-gGlu-C(O)(CH₂)₁₈COOH;
-NH2 denotes that the C-terminal amino acid residue is amidated as a primary amide; and
Ac- denotes that the N-terminal amino acid residue is Acetylated;
or a pharmaceutically acceptable salt thereof.

In an embodiment, the compound is a peptide of the following amino acid sequence (SEQ ID NO: 237):
NMeIVLSLDvPIKLK*KILLEQEKQKEQREQAETNAibQILAQV-NH2
wherein
the K residue at position 12 (denoted as K*) is chemically modified such that the epsilon-amino group of its side chain is covalently bound to -{AEEA}₂-gGlu-C(O)(CH₂)₁₈COOH;
-NH2 denotes that the C-terminal amino acid residue is amidated as a primary amide; and
NMe- denotes that the N-terminal amino acid residue is N-Methylated;
or a pharmaceutically acceptable salt thereof.

In an embodiment, the compound is a peptide of the following amino acid sequence (SEQ ID NO: 238):
Ac-IVLSLDvPIKLK*KILLEQEKQKKQREQAETNAibQILAQV-NH2
wherein
the K residue at position 12 (denoted as K*) is chemically modified such that the epsilon-amino group of its side chain is covalently bound to -{AEEA}₂-gGlu-C(O)(CH₂)₁₈COOH;
-NH2 denotes that the C-terminal amino acid residue is amidated as a primary amide; and
Ac- denotes that the N-terminal amino acid residue is Acetylated;
or a pharmaceutically acceptable salt thereof.

In an embodiment, the compound is a peptide of the following amino acid sequence (SEQ ID NO: 241):
Ac-IVLSLDvPIKLK*KILLEQEKQKEQREQAETNAibQILAQV-NH2
wherein
the K residue at position 12 (denoted as K*) is chemically modified such that the epsilon-amino group of its side chain is covalently bound to -{gGlu}₂-C(O)(CH₂)₁₈COOH;
-NH2 denotes that the C-terminal amino acid residue is amidated as a primary amide; and
Ac- denotes that the N-terminal amino acid residue is Acetylated;
or a pharmaceutically acceptable salt thereof.

In an embodiment, the compound is a peptide of the following amino acid sequence (SEQ ID NO: 242):
NMeIVLSLDvPIKLK*KILLEQEKQKEQREQAETNAibQILAQV-NH2
wherein
the K residue at position 12 (denoted as K*) is chemically modified such that the epsilon-amino group of its side chain is covalently bound to -{gGlu}₂-C(O)(CH₂)₁₈COOH;
-NH2 denotes that the C-terminal amino acid residue is amidated as a primary amide; and
NMe- denotes that the N-terminal amino acid residue is N-Methylated;
or a pharmaceutically acceptable salt thereof.

In an embodiment, the compound is a peptide of the following amino acid sequence (SEQ ID NO: 243):
Ac-IVLSLDvPIKLK*KILLEQEKQKKQREQAETNAibQILAQV-NH2
wherein
the K residue at position 12 (denoted as K*) is chemically modified such that the epsilon-amino group of its side chain is covalently bound to -{gGlu}₂-C(O)(CH₂)₁₈COOH;
-NH2 denotes that the C-terminal amino acid residue is amidated as a primary amide; and
Ac- denotes that the N-terminal amino acid residue is Acetylated;
or a pharmaceutically acceptable salt thereof.

The compounds of the disclosure may be prepared and utilised in the form of pharmaceutically acceptable salts. Pharmaceutically acceptable salts and methods for their preparation are well known in the art (see, e.g., Stahl, et al. "Handbook of Pharmaceutical Salts: Properties, Selection and Use", Second Revised Edition, Wiley-VCH, 2011; and Berge, et al., "Pharmaceutical Salts," Journal of Pharmaceutical Sciences, 1977, 66, 1). Examples of pharmaceutically acceptable salts include trifluoroacetate salts, sodium salts, acetate salts and hydrochloride salts.

### Compound Synthesis

A variety of methods can be used to prepare the compounds of the disclosure. The compounds may be prepared by synthesis in solution or on a solid support, with subsequent isolation and purification. Alternatively, the peptides can be prepared by gene expression in a host cell in which a DNA sequence encoding the peptide has been introduced. Gene expression can also be achieved without utilising a cell system. Combinations of methods may also be used.

In particular, the compounds may be prepared by solid phase synthesis on a suitable resin. Solid phase peptide synthesis is a well-established methodology (see, e.g., Stewart and Young, "Solid Phase Peptide Synthesis", Pierce Chemical Co., Rockford, Ill., 1984; and Atherton and Sheppard, "Solid Phase Peptide Synthesis: A Practical Approach", Oxford-IRL Press, New York, 1989).

Standard manual or automated solid phase synthesis procedures can be used to prepare the compounds. Automated peptide synthesisers are commercially available from, e.g., Applied Biosystems (Foster City, CA) and Protein Technologies Inc. (Tucson, AZ). Reagents for solid phase synthesis are readily available from commercial sources. Solid phase synthesisers can be used according to the manufacturer's instructions for blocking interfering groups, protecting amino acids during reaction, coupling, deprotecting, and capping of unreacted amino acids.

Solid phase synthesis may be initiated by attaching an N-terminally protected amino acid with its carboxy terminus to an inert solid support carrying a cleavable linker. The solid support can be any polymer that allows coupling of the initial amino acid, e.g. a trityl resin, a chlorotrityl resin, a Wang resin or a Rink resin in which the linkage of the carboxy group (or carboxamide group for a Rink resin) to the resin is sensitive to acid (when a Fmoc strategy is used). The support must be one which is stable under the conditions used to deprotect the α-amino group during the peptide synthesis.

After the N-terminally protected first amino acid has been coupled to the solid support, the α-amino protecting group of this amino acid is removed using a reagent such as trifluoroacetic acid (TFA) or piperidine, for example. The remaining protected amino acids are then coupled one after the other or added as a preformed dipeptide, tripeptide or tetrapeptide in the order represented by the peptide sequence using appropriate amide coupling reagents. Examples of coupling reagents include benzotriazol-1-yloxytris (dimethylamino)phosphonium hexafluorophosphate (BOP), hexafluorophosphate benzotriazole tetramethyl uronium (HBTU), hexafluorophosphate azabenzotriazole tetramethyl uronium (HATU), diisopropyl-carbodiimide (DIC), 1-hydroxybenzotriazole (HOBt) and 1-hydroxy-7-azabenzotriazole, and combinations thereof. Typically, couplings are performed at room temperature in an inert solvent such as dimethylformamide (DMF), N-methylpyrrolidone (NMP) or dichloromethane (DCM).

Usually, reactive side chain groups of the amino acids are protected with suitable blocking groups. These protecting groups are removed after the desired peptides have been assembled and they may be removed concomitantly with cleavage of the desired product from the resin under the same conditions. Protecting groups and procedures for their introduction are well known in the art (see, e.g., Greene and Wuts, "Protective Groups in Organic Synthesis", 3rd ed., 1999, Wiley & Sons). Examples of protecting groups include tert-butyloxycarbonyl (tBoc) and fluorenylmethoxycarbonyl (Fmoc).

The albumin-binding moiety may be introduced by selectively functionalising the lysine (K) residue at the position denoted by X12. Thus, the lysine residue may comprise a side chain protecting group which can be selectively removed while other side chain protecting groups remain intact, such that the deprotected lysine residue can be selectively functionalised by the albumin-binding moiety. Conjugation of the albumin-binding moiety to the epsilon-amino group of the lysine side chain may be achieved through an acylation reaction or other suitable reactions known in the art.

By way of illustration, the lysine residue may be protected with a 1-(4,4-dimethyl-2,6-dioxocyclohex-1-ylidene)-3-methylbutyl ("ivDde") protecting group, which is labile to highly nucleophilic bases such as 4% hydrazine in DMF (see Chhabra et al., Tetrahedron Lett. 1998, 39, 1603). Thus, if the N-terminal amino group and all side chain functionalities are protected with acid labile protecting groups, the ivDde group can be selectively removed using a highly nucleophilic base. The resulting free amino group can then be conjugated to the albumin-binding moiety, for example by acylation. Alternatively, the lysine residue may be protected with a (4-methoxyphenyl) diphenylmethyl ("Mmt") protecting group, which is labile to very mild acids such as acetic acid and trifluoroethanol in dichloromethane (see Dubowchik et al., Tetrahedron Lett., 1997, 38(30), 5257). Thus, if the N-terminal amino group and all side chain functionalities are protected with protecting groups only labile to strong acids, the Mmt group can be selectively removed using, e.g., a mixture of acetic acid and trifluoroethanol in dichloromethane (e.g., in a 1:2:7 ratio). The resulting free amino group can then be conjugated to the albumin-binding moiety, for example by acylation.

Alternatively, the albumin binding moiety can be introduced together with the lysine during peptide synthesis by using a prefunctionalized building block as a coupling partner. Examples of such prefunctionalized building blocks include Fmoc-L-Lys[gGlu(OtBu)-C(O)(CH₂)₁₈-C(O)OtBu]-OH, Fmoc-L-Lys[gGlu(OtBu)-C(O)(CH₂)₁₆-C(O)OtBu]-OH, Fmoc-L-Lys[gGlu(OtBu)-C(O)(CH₂)₁₄-C(O)OtBu]-OH and Fmoc-L-Lys[{AEEA}₂-gGlu(OtBu)-C(O)(CH₂)₁₆-C(O)OtBu]-OH.

If desired, the N-terminus of the peptide chain can be modified, for example by acetylation. For the synthesis of C-terminal amide peptides, resins incorporating Rink amide 4-methylbenzhydrylamine (MBHA) or Rink amide AM linkers are typically used with Fmoc synthesis, while MBHA resin is generally used with tBoc synthesis.

After completion of synthesis, peptides are cleaved from the solid-phase support with simultaneous side-chain deprotection using standard treatment methods. This can be achieved by using King's cocktail (King et al., Int. J. Peptide Protein Res., 1990, 36, 255-266) or similar cleavage cocktails known in the art.

The raw material can be purified by chromatography (e.g., by preparative RP-HPLC) if necessary. Crude peptides typically are purified using RP-HPLC on C8 or C18 columns using water-acetonitrile gradients in 0.05 to 0.1% trifluoroacetic acid (TFA). The purity of the peptides can be verified by analytical RP-HPLC. The identity of the peptides can be verified by mass spectrometry. The compounds may be isolated in solid form (e.g., as dry powders) using techniques such as lyophilization.

The present disclosure also relates to intermediate compounds for use in synthesising the present compounds. In particular, there is provided a compound which is a peptide comprising the amino acid sequence of formula (I) disclosed herein, in which residues X1 to X11 and X13 to X38 have the meanings recited in connection with formula (I) and X12 is lysine (K); or a salt thereof. Said compound may be used as an intermediate in the preparation of the compounds of the disclosure, which can be obtained by conjugating the albumin-binding moiety to the epsilon-amino group of the lysine side chain at X12. Addition of the albumin-binding moiety may be performed while the peptide is still attached to the solid phase. After adding the albumin-binding moiety, the peptide may be released from the resin and purified.

Particular processes for preparing the compounds of the disclosure are described in the examples below. The specific synthetic steps for each of the routes described may be combined in different ways to prepare the compounds. The reagents and starting materials are readily available or can be prepared by methods known in the art.

### Pharmaceutical Compositions

Also disclosed herein are pharmaceutical compositions comprising a compound of the disclosure and a pharmaceutically acceptable carrier or excipient.

A pharmaceutical composition may contain from about 0.1% to about 99.9% by weight of a compound of the disclosure and from about 99.9% to about 0.1% by weight of one or more pharmaceutically acceptable carriers, excipients or diluents. In one example, a pharmaceutical composition comprises from about 5% and about 75% by weight of the compound of the disclosure, with the rest being suitable pharmaceutical carriers, diluents or excipients. Methods of preparing pharmaceutical compositions are known, or will be apparent, to those skilled in this art, e.g., from literature such as Remington: The Science and Practice of Pharmacy, 22nd Edition, Pharmaceutical Press.

In an embodiment, the pharmaceutical composition further comprises one or more additional therapeutic agents.

The pharmaceutical composition may be suitable for administration by oral, inhalation or parenteral route, e.g., subcutaneous, intravenous, intraperitoneal, intramuscular or transdermal administration. In particular, the pharmaceutical composition may be suitable for subcutaneous administration. In an embodiment, the pharmaceutical composition is a ready-to-use composition suitable for administration by a pen or autoinjector device.

The compounds may exhibit desirable solubility, chemical stability and/or physical stability, especially in solvents at physiological pH values and solvents containing antimicrobial preservatives such as phenol or meta-cresol. As a consequence, the compounds may be particularly suitable for use in pharmaceutical compositions in solution form.

In a particular embodiment, the pharmaceutical composition is a solution comprising a solvent and, dissolved therein, a compound of the disclosure and an antimicrobial preservative selected from phenol and meta-cresol; wherein the compound of the disclosure is present in an amount of at least 1 mg/ml, at least 5 mg/ml, at least 10 mg/ml or at least 20 mg/ml; and wherein the solution has a pH of from pH 6 to 8 (e.g., pH 7.0 or pH 7.4) measured at 25 °C.

### Use in Therapy

The compounds of the disclosure are useful in therapy and may be used to treat or prevent a variety of diseases. Thus, in other aspects, the disclosure is directed to the use of the compounds in therapy and to therapeutic methods in which an effective amount of a compound of the disclosure is administered to a patient. The disclosure is also directed to the use of the compounds for the manufacture of medicaments for use in therapy. The compounds are especially useful in the therapy of diseases which can be treated or prevented by agonism of the CRF2 receptor.

The term "therapy" as used herein refers to the treatment or prevention of a disease in a patient.

The term "treat" or "treating" as used herein includes prohibiting, restraining, slowing, stopping, or reversing the progression or severity of an existing disease in a patient. Treatment may eliminate a disease; arrest or slow a disease in a patient; inhibit or slow the development of a new disease in a patient; decrease the frequency or severity of symptoms and/or recurrences in a patient who currently has or who previously has had a disease; and/or prolong, i.e., increase, the lifespan of the patient. In particular, treatment of a disease may result in curing, shortening the duration, ameliorating, slowing down or inhibiting progression or worsening of a disease or the symptoms thereof.

The term "prevent" or "preventing" as used herein refers to inhibiting or delaying the onset of a disease or disease in a patient.

The term "disease" as used herein refers to any condition or disorder that damages or interferes with the normal function of a cell, tissue or organ.

The term "patient" as used herein refers to a mammal, such as a human, mouse, guinea pig, rat, dog or cat. In a particular embodiment, the patient is a human patient.

The term "effective amount" as used herein refers to the amount or dose of compound of the disclosure which, upon single or multiple dose administration to the patient, provides the desired effect in the patient. An effective amount can be readily determined by the attending diagnostician by the use of known techniques and by observing results obtained under analogous circumstances. In determining the effective amount for a patient, a number of factors are considered by the attending diagnostician, including, but not limited to: the species of mammal; its size, age, and general health; the specific disease or disease involved; the degree of or involvement or the severity of the disease or disease; the response of the individual patient; the particular compound administered; the mode of administration; the bioavailability characteristics of the preparation administered; the dose regimen selected; the use of concomitant medication; and other relevant circumstances.

The compounds of the disclosure may be effective over a wide dosage range. For example, unitary dosages may fall within the range of about 0.1 µg/kg to about 50 mg/kg of body weight. For example for oral administration the unitary dosages may fall within the range of about 500 µg/kg to about 50 mg/kg. For the parenteral route of administration, e.g., by subcutaneous, intravenous, intraperitoneal, intramuscular or transdermal administration, the unitary dosages may fall within the range of about 0.1 µg/kg to about 300 µg/kg, and for inhalation as the route of administration the unitary dosages may fall within the range of about 1 µg/kg to about 1 mg/kg; in particular within the range of about 30 µg/kg to about 300 µg/kg,

In embodiments, the compounds are administered by once daily administration, once weekly, bi-monthly or monthly administration.

The compounds may be administered in combination with one or more additional therapeutic agents. The term "in combination with" as used herein means administration of the compound of the disclosure either simultaneously, sequentially or in a single combined formulation with the one or more additional therapeutic agents.

The compounds of the disclosure may be administered orally or by a parenteral route, e.g., by subcutaneous, intravenous, intraperitoneal, intramuscular or transdermal administration or by inhalation.

In a particular embodiment, the compounds are administered by subcutaneous administration. The compounds may be administered by a physician, a nurse or self-administered using an injection device. It is understood the gauge size and amount of injection volume is determined by the skilled practitioner. In one embodiment, the amount of injection volume is less than or equal to 2 ml, e.g., less than or equal to 1 ml. In another embodiment, a needle gauge of greater than or equal to 27, e.g., greater than or equal to 29, is used. Administration may be accomplished using an autoinjector or multidose delivery device.

The compounds of the disclosure may be useful in the therapy of diseases which can be treated or prevented by agonism of the CRF2 receptor.

The compounds are particularly useful in the treatment or prevention of cardiovascular diseases, obesity and diabetes. Thus, embodiments of the disclosure relate to the use of the compounds in the treatment or prevention of a cardiovascular diseases, obesity, diabetes, sarcopenia, particularly obesity-linked sarcopenia, cachexia, heart failure and pulmonary hypertension, in a patient.

The disclosure also relates to methods of treating or preventing a cardiovascular diseases, obesity, diabetes, kidney disease, sarcopenia, particularly obesity-linked sarcopenia, cachexia, heart failure and pulmonary hypertension in a patient which comprises administering an effective amount of a compound of the disclosure to the patient. In addition, the disclosure relates to the use of the compounds in the manufacture of a medicament for the treatment or prevention of a cardiovascular diseases, obesity, diabetes, kidney disease, sarcopenia, particularly obesity-linked sarcopenia, cachexia, heart failure and pulmonary hypertension in a patient.

Examples of cardiovascular diseases which may be treated or prevented using the present compounds include heart failure, hypertension, dyslipidaemia, atherosclerosis, arteriosclerosis, coronary heart disease, and stroke. The effect of the compounds in these conditions may be as a result of or associated with their effect on body weight or may be independent thereof. In a particular embodiment, the compounds are used to treat or prevent heart failure alone or on top of standard of care including but not exclusively angiotensin enzyme inhibitors, angiotensin II receptor antagonists, angiotensin receptor/neprilysin inhibitor (ARNi), beta-blockers, mineralocorticoid antagonists, SGLT1 inhibitors, SGLT2 inhibitors, diuretics.

The compounds may also be useful in the treatment or prevention of obesity and other diseases caused or characterised by excess body weight, such as obesity-linked inflammation, obesity-linked sarcopenia, obesity-linked gallbladder disease and obesity-induced sleep apnoea. In terms of a human adult patient, obesity can be defined as a body mass index (BMI) greater than or equal to 30 kg/m². The BMI is a simple index of weight-for-height that is commonly used to classify overweight and obesity in adults. It is defined as a person's weight in kilograms divided by the square of his/her height in meters and hence is expressed in units of kg/m². The compound may be administered in combination with one or more additional therapeutic agents useful in the treatment of obesity. Alternatively or additionally, the treatment may be combined with diet and exercise.

The compounds may also be used in the treatment or prevention of diabetes, especially type II diabetes. The compounds may be administered alone or in combination with one or more additional therapeutic agents useful in the treatment of diabetes, for example one or more agents selected from metformin, thiazolidinediones (TZDs), sulfonylureas (SUs), dipeptidyl peptidase-IV (DPP-IV) inhibitors, glucagon-like peptide-1 (GLP1) agonists, and sodium glucose co-transporters (SGLTs) inhibitors. Alternatively or additionally, treatment may be combined with diet and exercise. The compounds may also be used to treat or prevent hyperglycaemia, type I diabetes and impaired glucose tolerance.

The compounds may also be useful in the treatment or prevention of other diseases such as metabolic syndrome, kidney disease, degenerative diseases (e.g., neurodegenerative diseases) or diseases accompanied by nausea or vomiting. In particular in the treatment or prevention of sarcopenia, particularly obesity-linked sarcopenia, cachexia, heart failure and pulmonary hypertension alone or on top of standard of care.

The present invention is further illustrated by the following examples, which are provided for illustrative purposes only. The examples are not to be construed as limiting the scope or content of the disclosure in any way.

### EXAMPLES

### Abbreviations

Certain abbreviations are used in the examples and elsewhere herein:
"AA" refers to amino acid;
"AEEA" refers to [2-(2-aminoethoxy)ethoxy]acetyl;
"Aib" refers to 2-amino-isobutyric acid;
"AUC" refers to area under the curve;
"cAMP" refers to cyclic adenosine monophosphate;
"Boc" refers to tert-butyloxycarbonyl;
"BOP" refers to (benzotriazol-1-yloxy)tris(dimethylamino)phosphonium hexafluorophosphate;
"BSA" refers to bovine serum albumin;
"tBu" refers to tertiary butyl;
"DCM" refers to dichloromethane;
"Dde" refers to 1-(4,4-dimethyl-2,6-dioxocyclohexylidene)-ethyl;
"IvDde" refers to 1-(4,4-dimethyl-2,6-dioxocyclohexylidene)-3-methyl-butyl;
"DIC" refers to N,N'-diisopropylcarbodiimide;
"DIPEA" refers to N,N-diisopropylethylamine;
"DMEM" refers to Dulbecco's modified Eagle's medium;
"DMF" refers to dimethyl formamide;
"DMSO" refers to dimethyl sulfoxide;
"EDT" refers to ethane dithiol;
"FA" refers to formic acid;
"FBS" refers to fetal bovine serum;
"Fmoc" refers to fluorenylmethyloxycarbonyl;
"gGlu" refers to gamma-glutamate (γE);
"HATU" refers to *O*-(7-azabenzotriazol-1-yl)-*N,N,N',N*'-tetramethyluronium hexafluorophosphate;
"HBSS" refers to Hanks' Balanced Salt Solution;
"HBTU" refers to 2-(1H-benzotriazol-1-yl)-1,1,3,3-tetramethyluronium hexafluorophosphate;
"HEPES" refers to 2-[4-(2-hydroxyethyl)piperazin-1-yl]ethanesulfonic acid;
"HOAt" refers to 1-hydroxy-7-azabenzotriazole;
"HOBt" refers to 1-hydroxybenzotriazole;
"HOSu" refers to N-hydroxysuccinimide;
"HPLC" refers to High Performance Liquid Chromatography;
"hr" refers to hour;
"HTRF" refers to Homogenous Time Resolved Fluorescence;
"IBMX" refers to 3-isobutyl-1-methylxanthine;
"i.v." refers to intravenous;
"kDa" refers to kilodaltons;
"LC/MS" refers to Liquid Chromatography/Mass Spectrometry;
"Mmt" refers to monomethoxy-trityl;
"MS" refers to mass spectrometry;
"OtBu" refers to O-tert-butyl;
"Palm" refers to palmitoyl;
"Pbf" refers to 2,2,4,6,7-pentamethyldihydrobenzofuran-5-sulfonyl;
"PBS" refers to phosphate buffered saline;
"PK" refers to pharmacokinetic;
"RP-HPLC" refers to reversed-phase high performance liquid chromatography;
"s.c." refers to subcutaneous;
"SEM" refers to standard error of the mean;
"Stea" refers to stearyl;
"TIPS" refers to triisopropylsilane;
"TFA" refers to trifluoroacetic acid;
"Trt" refers to trityl; and
"UV" refers to ultraviolet.

### Materials and Methods

The following starting materials and methods were employed in the synthetic procedures described in the examples.

Rink Amide AM Resin LL 0.29 mmol/g (4-(2',4'-Dimethoxyphenyl-Fmoc-aminomethyl)-phenoxyacetamido-norleucylaminomethyl resin, Novabiochem), 100-200 mesh was used for the synthesis of all the peptide amides.

Fmoc-protected natural amino acids were purchased from Novabiochem, Iris Biotech, Bachem or Chem-Impex International. The following standard amino acids were used in the synthesis: Fmoc-L-Ala-OH, Fmoc-L-Arg(Pbf)-OH, Fmoc-L-Asn(Trt)-OH, Fmoc-L-Asp(OMpe)-OH, Fmoc-L-Gln(Trt)-OH, Fmoc-L-Glu(OtBu)-OH, Fmoc-L-Ile-OH, Fmoc-L-Leu-OH, Fmoc-L-Lys(Boc)-OH, Fmoc-L-Pro-OH, Fmoc-L-Ser(tBu)-OH, Fmoc-L-Thr(tBu)-OH, Fmoc-L-Val-OH.

In addition, the following amino acids were purchased from Novabiochem, Iris Biotech, Bachem, Chem-Impex International, FluoroChem, GL Biochem, Alchem Pharmtech or Aurum Pharmatech: Fmoc-L-Lys(Dde)-OH, Fmoc-Aib-OH, Fmoc-D-Val-OH, Fmoc-D-Ile-OH, Fmoc-D-Leu-OH, Fmoc-D-Ser(tBu)-OH, Fmoc-N-methyl-L-Ile, Fmoc-L-Abu-OH, Fmoc-L-Asn(Me2)-OH, Fmoc-L-HSer(Trt)-OH, N-Boc-4-(Fmoc-amino)piperidine-4-carboxylic acid, Fmoc-L-Nva-OH, Fmoc-α-Me-L-Lys(Boc)-OH, Fmoc-α-Me-L-Leu-OH, Fmoc-L-Dap(Boc)-OH, Fmoc-4-amino-tetrahydropyran-4-carboxylic acid, 1-(Fmoc-amino)cyclohexanecarboxylic acid, Fmoc-1-aminocyclopentane-1-carboxylic acid, 1-(Fmoc-amino)cyclopropanecarboxylic acid.

The following side chain building blocks were acquired from Iris Biotech, TCI, Merck, BLD pharm: Fmoc-AEEA-OH, Fmoc-L-Glu-OtBu, Tetradecanedioic acid (HO-C(O)(CH₂)₁₂COOH), 16-(tert-Butoxy)-16-oxohexadecanoic acid (HO-C(O)(CH₂)₁₆COOtBu), 18-(Tert-butoxy)-18-oxooctadecanoic acid (HO-C(O)(CH₂)₁₆COOtBu), Icosanedioic acid (HO-C(O)(CH₂)₁₈COOH).

Propionic acid, butyric acid, valeric acid, 3-methylbutanoic acid, tert-butoxycarbonyl tert-butyl carbonate and acetic anhydride were purchased from TCI, FluoroChem, Fluka or Sigma-Aldrich.

Crude peptides were purified on a preparative HPLC Waters system with a C4 column. Specifically, the following column was used: Reprosil (Dr. Maish) Gold C4 Prep, 250x40 mm, 120 Å, 5 µm.

Acetonitrile + 0.1% TFA and water + 0.1% TFA were employed as eluents. Product containing fractions were collected and lyophilized to obtain the purified product, typically as a TFA salt.

TFA to HCl ion exchange was performed with the following protocol: 10 equivalents of HCl 50mM were added to the peptide. The final concentration of 1mg/ml was reached by diluting the samples with a solution of H2O/ACN 80:20. After lyophilization, peptides were dissolved in H2O/ACN 80:20 and lyophilized again. The completeness of the ion exchange was checked with 19F NMR.

Crude and purified peptides were analyzed by Ultra-high performance liquid chromatography with UV and mass spectrometry detection (UPLC-UV-MS). Analytical UPLC was performed according to one of the following methods:
Method A:
Detection at 214 nm

| | |
|---|---|
| Column: | Acquity Waters BEH C4, 300 Å, 1.7µm (2.1x100 mm) at 45 °C |
| Solvent: | H2O+0.1%TFA: ACN+0.1%TFA (flow 0.4 ml/min) |
| Gradient: | 70:30 (0 min) to 70:30 (1 min) to 50:50 (5 min) to 10:90 (5.2 min) to 10:90 (5.5 min)to 70:30 (5.7 min) to 70:30 (6 min) |
| Mass analyzer: | Waters SQ Detector with electrospray ionization in positive ion detection mode |

Method B:
Detection at 214 nm

| | |
|---|---|
| Column: | Acquity Waters BEH C4, 300 Å, 1.7µm (2.1x100 mm) at 45 °C |
| Solvent: | H2O+0.1%TFA: ACN+0.1%TFA (flow 0.4 ml/min) |
| Gradient: | 65:35 (0 min) to 65:35 (1 min) to 45:55 (5 min) to 10:90 (5.2 min) to 10:90 (5.5 min) to 65:35 (5.7 min) to 65:35 (6 min) |
| Mass analyzer: | Waters SQ Detector with electrospray ionization in positive ion detection mode. |

### Example 1: General synthesis procedure SEQ ID NO: 1-145

The synthesis of all the peptides (SEQ ID NO: 1-145) was performed by standard Fmoc stepwise solid phase synthesis (SPPS) on a Liberty Blue microwave synthesizer (CEM corp.). The assembly was performed using a Rink amide AM Resin LL 0.29 mmol/g on a 0.1 mmol scale, with DIC/Oxyma activation. DMF was used as the solvent. Sequences were assembled with single couplings except for Arginine and for the amino acid introduced after an alpha methylated one, which underwent double coupling. For the modified lysine side chain, Fmoc-L-Lys(Dde)-OH was used at position 12.

The following conditions were employed:
Standard deprotection: 20% piperidine in DMF for 2 x 120 s, 90°C
Washes: 4 x DMF.
Standard Single coupling: 5 eq. AA 0.4 M / 5 eq. DIC 1M/ 5 eq. Oxyma 1M, 120 s, 90°C Washes: 4 x DMF.

At the end of the assembly, N-terminus of the peptide was modified as reported in Examples 2-9 according to the peptide sequence.

The removal of Dde group on Lys12 was achieved by dropping 2% hydrazine monohydrate and washing the resins with DMF/DCM/DMF (6/6/6 time each).

After the removal of the Dde group, the resins were treated as reported in Examples 2-9 according to the peptide sequence side chain derivatization.

Cleavage of the peptides from the resin was performed using the following cleavage cocktail: 87.5% TFA, 5% phenol, 5% water, 2.5% TIPS for 1.5 to 2.5 hours. The resin employed in the synthesis was such that the C-terminal was cleaved from the resin as a primary amide.

The cleavage mixture was collected by filtration, the crude peptides were precipitated in methyl *tert*-butyl ether, centrifuged, the supernatant was removed, fresh diethyl ether was added to the peptides and re-centrifuged, twice; the crude peptides were then lyophilized.

Peptides were analyzed by analytical UPLC and verified by ESI⁺ mass spectrometry. Crude peptides were purified by a conventional preparative RP-HPLC purification procedure.

### Example 2: Synthesis of the peptide with seq ID 125

The compound with seq ID 125, was prepared following the procedure described in Example 1. A Novabiochem Rink amide AM Resin LL 0.29 mmol/g (4-(2',4'-Dimethoxyphenyl-Fmoc-aminomethyl)-phenoxyacetamido-norleucylaminomethyl resin), 100-200 mesh was used. The automated Fmoc-synthesis strategy was applied with DIC/Oxyma-activation. In position 12, Fmoc-L-Lys(Dde)-OH was used in the solid phase synthesis protocol.

At the end of the assembly, N-terminus of the peptide was reacted with acetic anhydride (10 equivalent excess with respect to resin loading, Sigma-Aldrich) in DMF; the mixture was shaken at room temperature for 30 minutes and the reaction was monitored by Kaiser Test.

Dde protecting group on Lys12 was removed as reported in Example 1.

The γ-carboxyl end of glutamic acid was attached to the epsilon-amino group of Lys using a Fmoc-L-Glu-OtBu with DIC/HOAt method (4 equivalent excess with respect to resin loading) in DMF. The mixture was shaken at room temperature for 1 h and the reaction was monitored by Kaiser Test. The resin was filtered and washed with DMF/DCM/DMF (6/6/6 time each). The Fmoc group on the glutamic acid was removed by treating it three times with 20% (v/v) piperidine/DMF solution for 3 minutes. The resin was washed with DMF/DCM/DMF (6/6/6 time each). A Kaiser test on peptide resin aliquot upon completion of Fmoc-deprotection was positive.

Attachment of the albumin-binding moiety was performed using 18-(Tert-butoxy)-18-oxooctadecanoic acid (HO-C(O)(CH₂)₁₆COO*t*Bu) with DIC/HOAt method (5 equivalent excess with respect to resin loading) in DMF. The mixture was shaken at room temperature for 1 h and the reaction was monitored by Kaiser Test. The resin was filtered and washed with DMF/DCM/ DMF (6/6/6 time each).

The peptide was cleaved from the resin as described in the Example 1. The crude product was purified via preparative RP-HPLC on a Reprosil Gold C4 Prep (Dr Maisch), 250x40 mm, 120 Å, 5 µm using an acetonitrile/water gradient (with 0.1% TFA). The purified peptide was analyzed by LC/MS as reported in example 10.

### Example 3: Synthesis of the peptide with seq ID 9

The compound with seq ID 9, was prepared following the procedure described in Example 1. A Novabiochem Rink amide AM Resin LL 0.29 mmol/g (4-(2',4'-Dimethoxyphenyl-Fmoc-aminomethyl)-phenoxyacetamido-norleucylaminomethyl resin), 100-200 mesh was used. The automated Fmoc-synthesis strategy was applied with DIC/Oxyma-activation. In position 12, Fmoc-L-Lys(Dde)-OH was used in the solid phase synthesis protocol.

At the end of the assembly, N-terminus of the peptide was reacted with 3-methylbutanoic acid with DIC/HOAt method (4 equivalent excess with respect to resin loading, Sigma-Aldrich) in DMF; the mixture was shaken at room temperature for 1 h and the reaction was monitored by Kaiser Test.

Dde protecting group on Lys12 was removed as reported in Example 1.

The γ-carboxyl end of glutamic acid was attached to the epsilon-amino group of Lys using a Fmoc-L-Glu-OtBu with DIC/HOAt method (4 equivalent excess with respect to resin loading) in DMF. The mixture was shaken at room temperature for 1 h and the reaction was monitored by Kaiser Test. The resin was filtered and washed with DMF/DCM/DMF (6/6/6 time each). The Fmoc group on the glutamic acid was removed by treating it three times with 20% (v/v) piperidine/DMF solution for 3 minutes. The resin was washed with DMF/DCM/DMF (6/6/6 time each). A Kaiser test on peptide resin aliquot upon completion of Fmoc-deprotection was positive.

Attachment of the albumin-binding moiety was performed using 16-(tert-Butoxy)-16-oxohexadecanoic acid (HO-C(O)(CH₂)₁₆COO*t*Bu) with DIC/HOAt method (5 equivalent excess with respect to resin loading) in DMF. The mixture was shaken at room temperature for 1 h and the reaction was monitored by Kaiser Test. The resin was filtered and washed with DMF/DCM/ DMF (6/6/6 time each).

The peptide was cleaved from the resin as described in the Example 1. The crude product was purified via preparative RP-HPLC on a Reprosil Gold C4 Prep (Dr Maisch), 250x40 mm, 120 Å, 5 µm using an acetonitrile/water gradient (with 0.1% TFA). The purified peptide was analyzed by LC/MS as reported in example 10.

### Example 4: Synthesis of the peptide with seq ID 7

The compound with seq ID 7, was prepared following the procedure described in Example 1. A Novabiochem Rink amide AM Resin LL 0.29 mmol/g (4-(2',4'-Dimethoxyphenyl-Fmoc-aminomethyl)-phenoxyacetamido-norleucylaminomethyl resin), 100-200 mesh was used. The automated Fmoc-synthesis strategy was applied with DIC/Oxyma-activation. In position 12, Fmoc-L-Lys(Dde)-OH was used in the solid phase synthesis protocol.

At the end of the assembly, N-terminus of the peptide was acylated with butyric acid with DIC/HOAt method (4 equivalent excess with respect to resin loading, TCI) in DMF; the mixture was shaken at room temperature for 1 h and the reaction was monitored by Kaiser Test.

Dde protecting group on Lys12 was removed as reported in Example 1.

The γ-carboxyl end of glutamic acid was attached to the epsilon-amino group of Lys using a Fmoc-L-Glu-OtBu with DIC/HOAt method (4 equivalent excess with respect to resin loading) in DMF. The mixture was shaken at room temperature for 1 h and the reaction was monitored by Kaiser Test. The resin was filtered and washed with DMF/DCM/DMF (6/6/6 time each). The Fmoc group on the glutamic acid was removed by treating it three times with 20% (v/v) piperidine/DMF solution for 3 minutes. The resin was washed with DMF/DCM/DMF (6/6/6 time each). A Kaiser test on peptide resin aliquot upon completion of Fmoc-deprotection was positive.

Attachment of the albumin-binding moiety was performed using 16-(tert-Butoxy)-16-oxohexadecanoic acid (HO-C(O)(CH₂)₁₆COO*t*Bu) with DIC/HOAt method (5 equivalent excess with respect to resin loading) in DMF. The mixture was shaken at room temperature for 1 h and the reaction was monitored by Kaiser Test. The resin was filtered and washed with DMF/DCM/ DMF (6/6/6 time each).

The peptide was cleaved from the resin as described in the Example 1. The crude product was purified via preparative RP-HPLC on a Reprosil Gold C4 Prep (Dr Maisch), 250x40 mm, 120 Å, 5 µm using an acetonitrile/water gradient (with 0.1% TFA). The purified peptide was analyzed by LC/MS as reported in example 10.

### Example 5: Synthesis of the peptide with Seq ID 86

The compound with seq ID 86, was prepared following the procedure described in Example 1. A Novabiochem Rink amide AM Resin LL 0.29 mmol/g (4-(2',4'-Dimethoxyphenyl-Fmoc-aminomethyl)-phenoxyacetamido-norleucylaminomethyl resin), 100-200 mesh was used. The automated Fmoc-synthesis strategy was applied with DIC/Oxyma-activation. In position 12, Fmoc-L-Lys(Dde)-OH was used in the solid phase synthesis protocol.

At the end of the assembly N-terminus of the peptide was acylated with valeric acid with DIC/HOAt method (4 equivalent excess with respect to resin loading, Sigma-Aldrich) in DMF; the mixture was shaken at room temperature for 1 h and the reaction was monitored by Kaiser Test.

Dde protecting group on Lys12 was removed as reported in Example 1.

The γ-carboxyl end of glutamic acid was attached to the epsilon-amino group of Lys using a Fmoc-L-Glu-OtBu with DIC/HOAt method (4 equivalent excess with respect to resin loading) in DMF. The mixture was shaken at room temperature for 1 h and the reaction was monitored by Kaiser Test. The resin was filtered and washed with DMF/DCM/DMF (6/6/6 time each). The Fmoc group on the glutamic acid was removed by treating it twice with 20% (v/v) piperidine/DMF solution for 5 minutes (25 ml each). The resin was washed with NMP/DCM/NMP (6/6/6 time each). A Kaiser test on peptide resin aliquot upon completion of Fmoc-deprotection was positive.

Attachment of the albumin-binding moiety was performed using Tetradecanedioic acid (HO-C(O)(CH₂)₁₂COOH) with DIC/HOAt method (5 equivalent excess with respect to resin loading) in NMP. The mixture was shaken at room temperature for 1 h and the reaction was monitored by Kaiser Test. The resin was filtered and washed with NMP/DCM/NMP (6/6/6 time each).

The peptide was cleaved from the resin as described in the Example 1. The crude product was purified via preparative RP-HPLC on a Reprosil Gold C4 Prep (Dr Maisch), 250x40 mm, 120 Å, 5 µm using an acetonitrile/water gradient (with 0.1% TFA). The purified peptide was analyzed by LC/MS as reported in example 10.

### Example 6: Synthesis of the peptide with seq ID 42

The compound with seq ID 42, was prepared following the procedure described in Example 1. A Novabiochem Rink amide AM Resin LL 0.29 mmol/g (4-(2',4'-Dimethoxyphenyl-Fmoc-aminomethyl)-phenoxyacetamido-norleucylaminomethyl resin), 100-200 mesh was used. The automated Fmoc-synthesis strategy was applied with DIC/Oxyma-activation. In position 12, Fmoc-L-Lys(Dde)-OH was used in the solid phase synthesis protocol.

At the end of the assembly, N-terminus of the peptide was reacted with acetic anhydride (10 equivalent excess with respect to resin loading, Sigma-Aldrich) in DMF; the mixture was shaken at room temperature for 30 minutes and the reaction was monitored by Kaiser Test.

Dde protecting group on Lys12 was removed as reported in Example 1.

Fmoc-AEEA-OH was attached to the epsilon-amino group of Lys with DIC/HOAt method (4 equivalent excess with respect to resin loading) in DMF. The mixture was shaken at room temperature for 1 h and the reaction was monitored by Kaiser Test. The resin was filtered and washed with DMF/DCM/DMF (6/6/6 time each). The Fmoc group on AEEA was removed by treating the resin three times with 20% (v/v) piperidine/DMF solution for 3 minutes. The resin was washed with DMF/DCM/DMF (6/6/6 time each). A Kaiser test on peptide resin aliquot upon completion of Fmoc-deprotection was positive.

A second Fmoc-AEEA-OH was attached to the deprotected amino group with DIC/HOAt method in DMF and then deprotected from Fmoc as reported above.

The γ-carboxyl end of glutamic acid was attached to the deprotected amino group using a Fmoc-L-Glu-OtBu with DIC/HOAt method (4 equivalent excess with respect to resin loading) in DMF. The mixture was shaken at room temperature for 1 h and the reaction was monitored by Kaiser Test. The resin was filtered and washed with DMF/DCM/DMF (6/6/6 time each). The Fmoc group on the glutamic acid was removed by treating it three times with 20% (v/v) piperidine/DMF solution for 3 minutes. The resin was washed with DMF/DCM/ DMF (6/6/6 time each). A Kaiser test on peptide resin aliquot upon completion of Fmoc-deprotection was positive.

Attachment of the albumin-binding moiety was performed using 16-(tert-Butoxy)-16-oxohexadecanoic acid (HO-C(O)(CH₂)₁₆COO*t*Bu) with DIC/HOAt method (5 equivalent excess with respect to resin loading) in DMF. The mixture was shaken at room temperature for 1 h and the reaction was monitored by Kaiser Test. The resin was filtered and washed with DMF/DCM/ DMF (6/6/6 time each).

The peptide was cleaved from the resin as described in the Example 1. The crude product was purified via preparative RP-HPLC on a Reprosil Gold C4 Prep (Dr Maisch), 250x40 mm, 120 Å, 5 µm using an acetonitrile/water gradient (with 0.1% TFA). The purified peptide was analyzed by LC/MS as reported in example 10.

### Example 7: Synthesis of the peptide with seq ID 94

The compound with seq ID 94, was prepared following the procedure described in Example 1. A Novabiochem Rink amide AM Resin LL 0.29 mmol/g (4-(2',4'-Dimethoxyphenyl-Fmoc-aminomethyl)-phenoxyacetamido-norleucylaminomethyl resin), 100-200 mesh was used. The automated Fmoc-synthesis strategy was applied with DIC/Oxyma-activation. In position 12, Fmoc-L-Lys(Dde)-OH was used in the solid phase synthesis protocol.

At the end of the assembly, N-terminus of the peptide was acylated with propionic acid with DIC/HOAt method (4 equivalent excess with respect to resin loading, Sigma-Aldrich) in DMF; the mixture was shaken at room temperature for 1 h and the reaction was monitored by Kaiser Test.

Dde protecting group on Lys12 was removed as reported in Example 1.

Fmoc-AEEA-OH was attached to the epsilon-amino group of Lys with DIC/HOAt method (4 equivalent excess with respect to resin loading) in DMF. The mixture was shaken at room temperature for 1 h and the reaction was monitored by Kaiser Test. The resin was filtered and washed with DMF/DCM/DMF (6/6/6 time each). The Fmoc group on AEEA was removed by treating the resin three times with 20% (v/v) piperidine/DMF solution for 3 minutes. The resin was washed with DMF/DCM/DMF (6/6/6 time each). A Kaiser test on peptide resin aliquot upon completion of Fmoc-deprotection was positive.

A second Fmoc-AEEA-OH was attached to the deprotected amino group with DIC/HOAt method in DMF and then deprotected from Fmoc as reported above.

The γ-carboxyl end of glutamic acid was attached to the deprotected amino group using a Fmoc-L-Glu-OtBu with DIC/HOAt method (4 equivalent excess with respect to resin loading) in DMF. The mixture was shaken at room temperature for 1 h and the reaction was monitored by Kaiser Test. The resin was filtered and washed with DMF/DCM/DMF (6/6/6 time each). The Fmoc group on the glutamic acid was removed by treating it three times with 20% (v/v) piperidine/DMF solution for 3 minutes. The resin was washed with DMF/DCM/ DMF (6/6/6 time each). A Kaiser test on peptide resin aliquot upon completion of Fmoc-deprotection was positive.

Attachment of the albumin-binding moiety was performed using 18-(Tert-butoxy)-18-oxooctadecanoic acid (HO-C(O)(CH₂)₁₆COOtBu) with DIC/HOAt method (5 equivalent excess with respect to resin loading) in DMF. The mixture was shaken at room temperature for 1 h and the reaction was monitored by Kaiser Test. The resin was filtered and washed with DMF/DCM/ DMF (6/6/6 time each).

The peptide was cleaved from the resin as described in the Example 1. The crude product was purified via preparative RP-HPLC on a Reprosil Gold C4 Prep (Dr Maisch), 250x40 mm, 120 Å, 5 µm using an acetonitrile/water gradient (with 0.1% TFA). The purified peptide was analyzed by LC/MS as reported in example 10.

### Example 8: Synthesis of the comparative peptide comp3 with seq ID 146

A comparative compound (comp3) with the following peptide sequence,
IVLSLDvPIKLK*QILLKQERQKKQREQAEKNKQILEQV-NH2 (SEQ ID 146)
wherein
the K residue at position 12 (denoted as K*) is chemically modified such that the epsilon-amino group of its side chain is covalently bound to -{AEEA}₂-{gGlu}₂-C(O)(CH₂)₁₄COOH;
-NH2 denotes that the C-terminal amino acid residue is amidated as a primary amide;
was prepared following the procedure described in Example 1. A Novabiochem Rink amide AM Resin LL 0.29 mmol/g (4-(2',4'-Dimethoxyphenyl-Fmoc-aminomethyl)-phenoxyacetamido-norleucylaminomethyl resin), 100-200 mesh was used. The automated Fmoc-synthesis strategy was applied with DIC/Oxyma-activation. In position 12, Fmoc-L-Lys(Dde)-OH was used in the solid phase synthesis protocol.

At the end of the assembly, N-terminus of the peptide was protected using tert-butoxycarbonyl tert-butyl carbonate (10 equivalent excess with respect to resin loading, FluoroChem) in DMF; the mixture was shaken at room temperature for 30 minutes and the reaction was monitored by Kaiser Test.

Dde protecting group on Lys12 was removed as reported in Example 1.

Fmoc-AEEA-OH was attached to the epsilon-amino group of Lys with DIC/HOAt method (4 equivalent excess with respect to resin loading) in DMF. The mixture was shaken at room temperature for 1 h and the reaction was monitored by Kaiser Test. The resin was filtered and washed with DMF/DCM/DMF (6/6/6 time each). The Fmoc group on AEEA was removed by treating the resin three times with 20% (v/v) piperidine/DMF solution for 3 minutes. The resin was washed with DMF/DCM/DMF (6/6/6 time each). A Kaiser test on peptide resin aliquot upon completion of Fmoc-deprotection was positive.

A second Fmoc-AEEA-OH was attached to the deprotected amino group with DIC/HOAt method in DMF and then deprotected from Fmoc as reported above.

The γ-carboxyl end of glutamic acid was attached to the deprotected amino group using a Fmoc-L-Glu-OtBu with DIC/HOAt method (4 equivalent excess with respect to resin loading) in DMF. The mixture was shaken at room temperature for 1 h and the reaction was monitored by Kaiser Test. The resin was filtered and washed with DMF/DCM/DMF (6/6/6 time each). The Fmoc group on the glutamic acid was removed by treating it three times with 20% (v/v) piperidine/DMF solution for 3 minutes. The resin was washed with DMF/DCM/DMF (6/6/6 time each). A Kaiser test on peptide resin aliquot upon completion of Fmoc-deprotection was positive.

A second Fmoc-L-Glu-OtBu was attached to the deprotected amino group with DIC/HOAt method in DMF and then deprotected from Fmoc as reported above. The resin was washed with DMF/DCM/ DMF (6/6/6 time each).

Attachment of the albumin-binding moiety was performed using 16-(tert-Butoxy)-16-oxohexadecanoic acid (HO-C(O)(CH₂)₁₆COOtBu) with DIC/HOAt method (5 equivalent excess with respect to resin loading) in DMF. The mixture was shaken at room temperature for 1 h and the reaction was monitored by Kaiser Test. The resin was filtered and washed with DMF/DCM/ DMF (6/6/6 time each).

The peptide was cleaved from the resin as described in the Example 1. The crude product was purified via preparative RP-HPLC on a Reprosil Gold C4 Prep (Dr Maisch), 250x40 mm, 120 Å, 5 µm using an acetonitrile/water gradient (with 0.1% TFA). The purified peptide was analyzed by LC/MS as reported in example 10.

### Example 9: Synthesis of the peptide with seq ID 41

The compound with seq ID 41, was prepared following the procedure described in Example 1. A Novabiochem Rink amide AM Resin LL 0.29 mmol/g (4-(2',4'-Dimethoxyphenyl-Fmoc-aminomethyl)-phenoxyacetamido-norleucylaminomethyl resin), 100-200 mesh was used. The automated Fmoc-synthesis strategy was applied with DIC/Oxyma-activation. In position 12, Fmoc-L-Lys(Dde)-OH was used in the solid phase synthesis protocol.

At the end of the assembly, N-terminus of the peptide was reacted with acetic anhydride (10 equivalent excess with respect to resin loading, Sigma-Aldrich) in DMF; the mixture was shaken at room temperature for 30 minutes and the reaction was monitored by Kaiser Test.

The protecting group on Lys12 was removed as reported in Example 1.

The γ-carboxyl end of glutamic acid was attached to the deprotected amino group using a Fmoc-L-Glu-OtBu with DIC/HOAt method (4 equivalent excess with respect to resin loading) in DMF. The mixture was shaken at room temperature for 1 h and the reaction was monitored by Kaiser Test. The resin was filtered and washed with DMF/DCM/DMF (6/6/6 time each). The Fmoc group on the glutamic acid was removed by treating it three times with 20% (v/v) piperidine/DMF solution for 3 minutes. The resin was washed with DMF/DCM/DMF (6/6/6 time each). A Kaiser test on peptide resin aliquot upon completion of Fmoc-deprotection was positive.

A second Fmoc-L-Glu-OtBu was attached to the deprotected amino group with DIC/HOAt method in DMF and then deprotected from Fmoc as reported above. The resin was washed with DMF/DCM/ DMF (6/6/6 time each).

Attachment of the albumin-binding moiety was performed using 16-(tert-Butoxy)-16-oxohexadecanoic acid (HO-C(O)(CH₂)₁₆COOtBu) with DIC/HOAt method (5 equivalent excess with respect to resin loading) in DMF. The mixture was shaken at room temperature for 1 h and the reaction was monitored by Kaiser Test. The resin was filtered and washed with DMF/DCM/ DMF (6/6/6 time each).

The peptide was cleaved from the resin as described in the Example 1. The crude product was purified via preparative RP-HPLC on a Reprosil Gold C4 Prep (Dr Maisch), 250x40 mm, 120 Å, 5 µm using an acetonitrile/water gradient (with 0.1% TFA). The purified peptide was analyzed by LC/MS as reported in example 10.

### Example 10: Synthesis of further peptides

The following peptides were synthesized following the procedures described in Examples 1-9. In addition to comparative peptide comp3 above, it includes two further comparative peptides, i.e. comp 1 with seq ID 147 and comp2 with Seq ID 148 respectively

| **SEQ ID NO.** | **Peptide** | **Amino acid sequence** | **R^{a}** |
|---|---|---|---|
| **147** | Comp1 | IVLSLDvPIKLK*QILLKQERQKKQREQAEKNKQILEQV-NH2 | -{gGlu}₂-C(O)(CH₂)₁₄COOH |
| **148** | Comp2 | IVLSLDvPIKLK*QILLKQERQKKQREQAEKNKQILEQV-NH2 | -{AEEA}₂-gGlu-C(O)(CH₂)₁₄COOH |
| Table legend: K* = position of side chain derivatization; v = D-Valine; R^{a} = side chain derivatization for K*; R* structures are reported in Figure 1 | | | |

The calculated and found masses and retention times of these peptides are indicated in Table 3 below, along with those of the compounds of Examples 2-9:

**Table 3**

| **SEQ ID NO.** | **Calc. mass (M/4+H)+** | **Found mass (M/4+H)+** | **Retention Time (min)** | **Method** |
|---|---|---|---|---|
| **146** | 1271.0 | 1271.6 | 3.42 | A |
| **147** | 1311.3 | 1312.0 | 3.50 | A |
| **148** | 1343.6 | 1343.8 | 3.39 | A |
| **2** | 1249.3 | 1249.6 | 3.48 | B |
| **3** | 1221.0 | 1221.4 | 4.10 | A |
| **4** | 1210.6 | 1211.0 | 3.47 | A |
| **5** | 1242.2 | 1242.5 | 3.70 | A |
| **6** | 1224.5 | 1224.8 | 3.28 | B |
| **7** | 1228.0 | 1228.8 | 3.35 | B |
| **8** | 1231.5 | 1232.3 | 3.52 | B |
| **9** | 1231.5 | 1231.7 | 3.57 | B |
| **10** | 1228.0 | 1228.2 | 3.76 | A |
| **13** | 1231.5 | 1231.7 | 3.78 | A |
| **14** | 1232.0 | 1232.2 | 3.74 | A |
| **16** | 1238.5 | 1238.5 | 4.53 | A |
| **17** | 1249.5 | 1249.8 | 3.34 | B |
| **18** | 1235.0 | 1235.0 | 3.50 | B |
| **19** | 1242.0 | 1242.0 | 3.34 | B |
| **20** | 1235.0 | 1234.9 | 3.21 | B |
| **21** | 1238.6 | 1238.4 | 3.30 | B |
| **22** | 1217.2 | 1217.3 | 3.63 | B |
| **23** | 1238.5 | 1238.3 | 2.93 | B |
| **24** | 1235.0 | 1234.8 | 3.98 | A |
| **25** | 1300.5 | 1300.6 | 3.62 | A |
| **26** | 1311.1 | 1310.9 | 3.42 | B |
| **27** | 1314.6 | 1314.7 | 3.62 | A |
| **28** | 1307.6 | 1307.6 | 3.89 | A |
| **29** | 1296.8 | 1296.8 | 3.77 | B |
| **30** | 1318.1 | 1317.9 | 3.71 | B |
| **31** | 1311.1 | 1310.9 | 3.94 | A |
| **32** | 1300.3 | 1300.1 | 3.78 | B |
| **33** | 1192.9 | 1193.8 | 3.00 | B |
| **34** | 1182.4 | 1182.5 | 3.46 | A |
| **35** | 1196.4 | 1196.5 | 4.31 | A |
| **36** | 1200.0 | 1200.0 | 4.45 | A |
| **37** | 1203.5 | 1203.5 | 4.62 | A |
| **38** | 1203.4 | 1203.5 | 3.59 | A |
| **39** | 1210.7 | 1210.4 | 3.36 | B |
| **40** | 1210.5 | 1210.5 | 3.57 | B |
| **41** | 1242.7 | 1243.1 | 3.06 | B |
| **42** | 1283.0 | 1283.0 | 3.37 | B |
| **43** | 1214.0 | 1213.9 | 3.37 | B |
| **44** | 1214.0 | 1213.8 | 3.58 | B |
| **45** | 1182.2 | 1182.1 | 4.21 | A |
| **46** | 1185.9 | 1185.6 | 4.30 | A |
| **47** | 1207.0 | 1207.2 | 3.36 | B |
| **48** | 1214.0 | 1213.9 | 4.63 | A |
| **49** | 1217.5 | 1217.5 | 3.44 | B |
| **50** | 1217.5 | 1217.5 | 3.63 | B |
| **51** | 1210.5 | 1210.4 | 3.55 | B |
| **52** | 1214.0 | 1213.9 | 3.60 | B |
| **53** | 1225.0 | 1225.0 | 3.47 | B |
| **54** | 1224.7 | 1224.6 | 3.45 | B |
| **55** | 1210.7 | 1211.1 | 3.25 | B |
| **56** | 1221.5 | 1221.2 | 3.55 | B |
| **57** | 1220.7 | 1221.0 | 3.27 | B |
| **58** | 1221.0 | 1220.8 | 3.45 | B |
| **59** | 1220.5 | 1220.1 | 3.54 | B |
| **60** | 1217.0 | 1216.7 | 3.49 | B |
| **61** | 1210.0 | 1209.7 | 3.42 | B |
| **64** | 1203.5 | 1203.5 | 3.79 | A |
| **65** | 1207.0 | 1206.9 | 3.85 | A |
| **66** | 1207.0 | 1206.9 | 3.85 | A |
| **67** | 1210.5 | 1210.4 | 3.88 | A |
| **68** | 1200.0 | 1199.7 | 3.69 | A |
| **69** | 1203.4 | 1203.3 | 3.69 | A |
| **70** | 1203.7 | 1203.4 | 3.59 | A |
| **71** | 1214.4 | 1214.2 | 3.66 | A |
| **72** | 1213.7 | 1213.9 | 2.44 | B |
| **73** | 1213.9 | 1213.6 | 3.83 | B |
| **74** | 1189.2 | 1188.8 | 3.87 | A |
| **75** | 1192.7 | 1192.4 | 3.89 | A |
| **78** | 1185.7 | 1185.5 | 3.37 | B |
| **79** | 1189.2 | 1189.0 | 3.45 | B |
| **80** | 1192.7 | 1192.9 | 3.68 | B |
| **81** | 1196.2 | 1196.2 | 3.71 | B |
| **82** | 1203.4 | 1203.5 | 4.19 | A |
| **83** | 1203.4 | 1203.5 | 4.19 | A |
| **84** | 1213.7 | 1213.7 | 4.04 | A |
| **85** | 1214.5 | 1214.1 | 3.22 | B |
| **86** | 1185.7 | 1185.3 | 3.40 | B |
| **87** | 1196.4 | 1196.1 | 3.53 | A |
| **92** | 1272.5 | 1273.3 | 3.33 | B |
| **93** | 1262.0 | 1262.5 | 3.67 | A |
| **94** | 1276.0 | 1275.9 | 3.23 | B |
| **95** | 1279.6 | 1279.4 | 3.39 | B |
| **96** | 1283.0 | 1283.0 | 3.58 | B |
| **97** | 1290.0 | 1289.9 | 3.54 | B |
| **98** | 1290.1 | 1289.8 | 3.62 | B |
| **99** | 1261.8 | 1262.1 | 3.15 | B |
| **100** | 1286.5 | 1286.4 | 3.68 | B |
| **101** | 1258.3 | 1258.0 | 3.33 | B |
| **102** | 1293.6 | 1293.5 | 3.62 | B |
| **103** | 1293.6 | 1293.6 | 3.76 | B |
| **104** | 1290.0 | 1290.0 | 3.70 | B |
| **105** | 1293.6 | 1293.5 | 3.73 | B |
| **106** | 1304.3 | 1304.5 | 3.39 | B |
| **107** | 1290.3 | 1290.1 | 3.60 | B |
| **108** | 1301.0 | 1300.9 | 3.66 | B |
| **109** | 1300.3 | 1300.1 | 3.47 | B |
| **110** | 1293.5 | 1293.4 | 3.71 | B |
| **111** | 1297.1 | 1297.1 | 3.80 | B |
| **112** | 1290.0 | 1289.8 | 3.71 | B |
| **113** | 1279.5 | 1279.4 | 3.94 | A |
| **114** | 1283.0 | 1282.8 | 3.90 | A |
| **115** | 1283.3 | 1283.1 | 3.80 | A |
| **116** | 1294.0 | 1293.8 | 3.84 | A |
| **117** | 1293.3 | 1293.1 | 2.64 | B |
| **118** | 1283.0 | 1283.0 | 3.99 | A |
| **119** | 1286.6 | 1286.4 | 4.03 | A |
| **120** | 1272.3 | 1272.3 | 3.87 | B |
| **121** | 1275.8 | 1275.7 | 3.91 | B |
| **122** | 1268.8 | 1268.6 | 3.84 | B |
| **123** | 1275.8 | 1275.7 | 3.88 | B |
| **124** | 1279.3 | 1279.9 | 3.94 | B |
| **125** | 1217.5 | 1217.3 | 3.80 | B |
| **126** | 1214.0 | 1213.8 | 3.76 | B |
| **127** | 1217.5 | 1217.3 | 3.92 | B |
| **128** | 1217.7 | 1217.8 | 3.74 | B |
| **129** | 1227.7 | 1227.5 | 3.66 | B |
| **130** | 1228.5 | 1228.3 | 3.88 | B |
| **131** | 1228 | 1227.6 | 3.77 | B |
| **132** | 1227.5 | 1227.2 | 3.86 | B |
| **133** | 1224.0 | 1223.8 | 3.82 | B |
| **134** | 1217.0 | 1216.8 | 3.75 | B |
| **135** | 1210.4 | 1210.3 | 2.95 | B |
| **136** | 1207.0 | 1207.1 | 2.98 | B |
| **137** | 1210.5 | 1210.2 | 2.96 | B |
| **138** | 1210.7 | 1210.4 | 3.93 | A |
| **139** | 1220.7 | 1220.8 | 2.88 | B |
| **142** | 1221.5 | 1221.3 | 3.98 | A |
| **143** | 1220.9 | 1220.6 | 4.18 | B |
| **144** | 1196.1 | 1196.2 | 3.21 | A |
| **145** | 1199.7 | 1199.7 | 4.27 | A |
| **152** | 1206.4 | 1206.2 | 3.69 | A |
| **153** | 1209.9 | 1209.6 | 3.74 | A |
| **169** | 1199.4 | 1199.2 | 3.34 | A |
| **170** | 1202.9 | 1202.8 | 3.36 | A |
| **187** | 1213.4 | 1213 | 4.09 | A |
| **188** | 1216.9 | 1216.5 | 4.09 | A |
| **154** | 1199.4 | 1199.2 | 3.90 | B |
| **155** | 1202.9 | 1202.7 | 3.93 | B |
| **176** | 1182.2 | 1182.0 | 3.71 | B |
| **177** | 1185.7 | 1185.4 | 3.72 | B |
| **159** | 1175.2 | 1174.7 | 3.66 | B |
| **160** | 1185.4 | 1185 | 3.53 | B |
| **156** | 1196.2 | 1195.7 | 3.99 | A |
| **157** | 1206.4 | 1206 | 3.86 | A |
| **182** | 1196.4 | 1196.1 | 3.98 | B |
| **183** | 1206.7 | 1206.2 | 3.85 | B |
| **185** | 1178.7 | 1178.3 | 3.04 | A |
| **186** | 1189.4 | 1189.9 | 3.14 | B |
| **206** | 1224.4 | 1224.0 | 3.32 | C |
| **207** | 1234.7 | 1234.5 | 4.26 | A |
| **219** | 1206.7 | 1206.3 | 3.53 | C |
| **214** | 1217.4 | 1217.0 | 3.48 | A |
| **171** | 1276.0 | 1275.6 | 3.08 | A |
| **172** | 1286.3 | 1286.1 | 4.00 | B |
| **180** | 1258.2 | 1257.9 | 3.21 | A |
| **178** | 1269.0 | 1268.6 | 3.42 | B |
| **161** | 1196.1 | 1195.6 | 3.86 | A |
| **162** | 1199.7 | 1199.2 | 3.84 | A |
| **189** | 1203.2 | 1202.7 | 4.20 | A |
| **190** | 1206.7 | 1206.3 | 3.16 | C |
| **163** | 1196.2 | 1195.9 | 4.05 | A |
| **164** | 1196.2 | 1195.9 | 3.77 | A |
| **165** | 1196.2 | 1195.7 | 3.77 | A |
| **166** | 1196.1 | 1195.8 | 3.97 | A |
| **167** | 1189.1 | 1188.8 | 3.78 | A |
| **208** | 1223.9 | 1223.6 | 3.44 | A |
| **215** | 1206.7 | 1206.4 | 3.72 | C |
| **200** | 1272.2 | 1272.3 | 4.17 | B |
| **184** | 1195.9 | 1195.8 | 4.02 | B |
| **173** | 1275.5 | 1275.1 | 4.20 | B |
| **196** | 1289.5 | 1289.1 | 3.87 | A |
| **192** | 1192.6 | 1192.3 | 3.54 | A |
| **201** | 1272.3 | 1271.9 | 3.93 | A |
| **203** | 1253.8 | 1253.7 | 3.81 | A |
| **204** | 1322.3 | 1321.9 | 3.63 | A |
| **197** | 1279.2 | 1278.9 | 3.99 | A |
| **202** | 1261.4 | 1261.2 | 3.05 | C |
| **209** | 1213.7 | 1213.2 | 3.58 | C |
| **226** | 1206.7 | 1206.1 | 3.78 | C |
| **227** | 1216.9 | 1216.8 | 3.65 | C |
| **220** | 1195.9 | 1196.0 | 3.66 | C |
| **228** | 1188.9 | 1188.9 | 3.86 | C |
| **229** | 1199.7 | 1199.2 | 3.92 | A |
| **193** | 1199.7 | 1199.7 | 4.11 | A |
| **194** | 1188.9 | 1189.1 | 4.32 | A |
| **195** | 1181.9 | 1181.5 | 3.48 | C |
| **191** | 1199.7 | 1199.1 | 3.34 | C |
| **205** | 1286.0 | 1285.7 | 3.67 | C |
| **174** | 1308.1 | 1307.8 | 3.97 | B |
| **175** | 1318.3 | 1318.7 | 3.82 | B |
| **181** | 1290.3 | 1290.5 | 4.05 | B |
| **179** | 1301.1 | 1301.4 | 3.22 | B |
| **210** | 1234.9 | 1234.8 | 3.35 | C |
| **158** | 1210.0 | 1210.0 | 3.70 | C |
| **198** | 1300.6 | 1300.8 | 3.74 | A |
| **230** | 1260.7 | 1260.5 | 3.19 | C |
| **234** | 1243.0 | 1243.1 | 3.59 | A |
| **235** | 1242.9 | 1242.7 | 3.36 | C |
| **211** | 1210.2 | 1210.5 | 3.43 | C |
| **216** | 1203.2 | 1203.0 | 3.62 | A |
| **221** | 1192.4 | 1192.3 | 3.62 | C |
| **212** | 1210.2 | 1210.0 | 3.54 | C |
| **217** | 1203.2 | 1203.1 | 3.71 | A |
| **222** | 1192.5 | 1192.4 | 3.71 | C |
| **223** | 1224.2 | 1224.2 | 4.44 | C |
| **224** | 1231.3 | 1231.6 | 3.7 | D |
| **225** | 1238.3 | 1238.3 | 4.10 | D |
| **238** | 1297.1 | 1296.9 | 4.16 | A |
| **243** | 1256.8 | 1256.6 | 3.08 | C |
| **218** | 1217.7 | 1217.6 | 4 | A |
| **239** | 1279.3 | 1279.4 | 3.57 | A |
| **213** | 1224.7 | 1224.8 | 3.55 | C |
| **236** | 1297.3 | 1297.6 | 3.28 | C |
| **241** | 1257.0 | 1257.3 | 4.22 | A |
| **237** | 1290.3 | 1290.7 | 3.48 | A |
| **242** | 1250.0 | 1250.3 | 3.39 | A |
| **233** | 1254.0 | 1254.1 | 3.6 | A |
| **231** | 1261.0 | 1260.9 | 3.39 | C |
| **168** | 1210.7 | 1211.2 | 3.77 | A |
| **232** | 1246.5 | 1247.0 | 4.36 | A |
| **240** | 1282.8 | 1283.2 | 4.31 | A |
| **244** | 1242.5 | 1242.9 | 4.21 | A |

### Example 11: Assessment of activity at human CRF2 α receptor

Agonism of compounds for human corticotropin-releasing factor 2α (CRF2α receptor) was determined by a functional assay measuring cAMP modulation upon treatment of TeloHEAC cells stably expressing human CRF2α receptor.

Compounds were dissolved at 0.5 mM in 100% DMSO and serially diluted (1:2) in 100% DMSO for 16 dilutions. 20 nL of each dilution was then transferred to 384-well assay plate using an acoustic droplet ejection instrument. 5 µL of compound buffer (1x HBSS; 20 mM HEPES, ) were then added to each well.

Prior to use, frozen cells were thawed quickly at 37 °C and washed (5 min at 900 rpm) with 20 mL cell buffer (1x HBSS; 20 mM HEPES). Cells were resuspended in cell buffer added with 2 mM IBMX and adjusted to a cell density of 2M cells/ml.

5 µL cells (final dell density: 10000 cells/well) were dispensed in the compound-containing 384-well assay plate and incubated for 30 minutes 37 °C.

The cAMP level in treated cells was determined using the Cisbio 62AM4PEC kit according to manufacturer's instruction. Finally, plates were incubated for 1 h at room temperature before measuring the fluorescence ratio between 665 / 620 nm.

The percent activity value (E%) was calculated by setting 100 nM of urocortin 2 (UCN2) as 100%. The *in vitro* potency of the compounds was quantified by determining the concentrations that caused 50% activation of maximal response (EC50) relative to urocortin 2.

Representative EC50 values are provided in Table 4 below:

**Table 4**

| **SEQ ID No.** | **EC50 [nM]** |
|---|---|
| 149 (Comp Y) | 0.44 |
| 150 (Comp Z) | 0.25 |
| 151 (Comp X) | 0.12 |
| 146 (Comp 1) | 0.1 |
| 147 (Comp 2) | 0.18 |
| 148 (Comp 3) | 0.65 |
| 2 (Comp 4) | 0.07 |
| 5 (Comp 5) | 0.09 |
| 56 (Comp 10) | 0.3 |
| 116 (comp 12) | 0.37 |
| 130 (Comp 13 | 0.08 |
| 142 (Comp 14) | 0.07 |
| 4 | 0.44 |
| 8 | 0.14 |
| 3 | 0.16 |
| 6 | 0.09 |
| 9 | 0.16 |
| 7 | 0.06 |
| 10 | 0.17 |
| 13 | 0.15 |
| 14 | 0.26 |
| 92 | 2.3 |
| 93 | 5.08 |
| 33 | 0.47 |
| 34 | 1.61 |
| 94 | 1.48 |
| 96 | 0.49 |
| 95 | 0.76 |
| 25 | 5.02 |
| 26 | 18.63 |
| 97 | 3.17 |
| 19 | 0.28 |
| 20 | 1.09 |
| 39 | 0.53 |
| 38 | 0.49 |
| 43 | 0.3 |
| 102 | 17.59 |
| 49 | 0.92 |
| 40 | 0.25 |
| 64 | 0.19 |
| 118 | 0.6 |
| 80 | 0.35 |
| 120 | 1.33 |
| 66 | 1.05 |
| 81 | 0.8 |
| 121 | 5.76 |
| 18 | 3.55 |
| 22 | 4.44 |
| 35 | 0.23 |
| 36 | 0.14 |
| 37 | 0.13 |
| 103 | 3.09 |
| 48 | 1.35 |
| 111 | 2.58 |
| 27 | 16.89 |
| 28 | 2.2 |
| 29 | 37.17 |
| 16 | 0.13 |
| 44 | 0.25 |
| 50 | 0.26 |
| 53 | 8.05 |
| 46 | 0.7 |
| 65 | 0.1 |
| 67 | 0.34 |
| 110 | 0.3 |
| 125 | 0.15 |
| 119 | 1.62 |
| 123 | 0.7 |
| 124 | 0.38 |
| 45 | 0.96 |
| 100 | 0.41 |
| 104 | 0.47 |
| 105 | 0.87 |
| 113 | 0.35 |
| 122 | 0.67 |
| 30 | 0.29 |
| 31 | 0.23 |
| 32 | 1.04 |
| 42 | 3.08 |
| 41 | 1.85 |
| 106 | 0.35 |
| 107 | 1.43 |
| 108 | 1.14 |
| 54 | 0.12 |
| 55 | 0.42 |
| 51 | 0.12 |
| 52 | 0.17 |
| 17 | 0.21 |
| 21 | 0.34 |
| 47 | 0.09 |
| 57 | 0.39 |
| 78 | 0.73 |
| 79 | 0.38 |
| 99 | 6.07 |
| 112 | 0.28 |
| 101 | 1.74 |
| 98 | 0.55 |
| 109 | 1 |
| 68 | 0.05 |
| 71 | 0.21 |
| 72 | 0.14 |
| 126 | 0.05 |
| 127 | 0.08 |
| 129 | 0.14 |
| 137 | 0.07 |
| 69 | 0.09 |
| 70 | 0.17 |
| 128 | 0.17 |
| 115 | 0.76 |
| 114 | 0.18 |
| 140 | 0.17 |
| 139 | 0.05 |
| 117 | 0.63 |
| 138 | 0.05 |
| 141 | 0.16 |
| 23 | 0.33 |
| 24 | 0.04 |
| 58 | 0.41 |
| 133 | 0.16 |
| 59 | 0.81 |
| 134 | 0.5 |
| 60 | 0.34 |
| 135 | 0.19 |
| 61 | 0.36 |
| 136 | 0.16 |
| 74 | 0.15 |
| 75 | 0.19 |
| 152 | 0.51 |
| 153 | 0.41 |
| 154 | 0.52 |
| 155 | 0.51 |
| 156 | 0.29 |
| 157 | 0.32 |
| 158 | 0.04) |
| 159 | 2.97 |
| 160 | 2.08 |
| 161 | 0.32 |
| 162 | 0.49 |
| 163 | 0.3 |
| 164 | 2.43 |
| 165 | 0.64 |
| 166 | 0.77 |
| 167 | 1.15 |
| 168 | 0.8 |
| 169 | 1.34 |
| 170 | 1.28 |
| 171 | 5.79 |
| 172 | 16.13 |
| 173 | 28.14 |
| 174 | 16.18 |
| 175 | 18.85 |
| 176 | 0.99 |
| 177 | 0.86 |
| 178 | 6.5 |
| 179 | 15.55 |
| 180 | 10.67 |
| 181 | 24.68 |
| 182 | 1.88 |
| 183 | 2.47 |
| 184 | 4.25 |
| 185 | 3.54 |
| 186 | 0.79 |
| 187 | 0.26 |
| 188 | 0.28 |
| 189 | 0.29 |
| 190 | 0.3 |
| 191 | 0.18 |
| 192 | 0.11 |
| 193 | 0.17 |
| 194 | 0.44 |
| 195 | 0.21 |
| 196 | 2.2 |
| 197 | 1.28 |
| 198 | 1.7 |
| 199 | 0.16 |
| 200 | 0.71 |
| 201 | 1.37 |
| 202 | 1.73 |
| 203 | 0.43 |
| 204 | 1.84 |
| 205 | 1.43 |
| 206 | 0.11 |
| 207 | 0.12 |
| 208 | 0.15 |
| 209 | 0.22 |
| 210 | 0.21 |
| 211 | 0.15 |
| 212 | 0.11 |
| 213 | 0.22 |
| 214 | 0.12 |
| 215 | 0.12 |
| 216 | 0.05 |
| 217 | 0.09 |
| 218 | 0.11 |
| 219 | 0.16 |
| 220 | 0.27 |
| 221 | 0.18 |
| 222 | 0.14 |
| 223 | 0.08 |
| 224 | 0.07 |
| 225 | 0.09 |
| 226 | 0.18 |
| 227 | 0.2 |
| 228 | 0.19 |
| 229 | 0.11 |
| 230 | 0.16 |
| 231 | 0.28 |
| 232 | 0.12 |
| 233 | 0.12 |
| 234 | 0.09 |
| 235 | 0.16 |
| 236 | 0.54 |
| 237 | 0.41 |
| 238 | 0.11 |
| 239 | 0.11 |
| 240 | 0.22 |
| 241 | 0.56 |
| 242 | 0.22 |
| 243 | 0.13 |
| 244 | 0.28 |

### Example 12: Assessment of activity at anti-target CFR1R receptor

Assessment of activity at anti-target CFR1R receptor is done using the same procedure as the one described for the TeloHEAC cell line.

The CRF1R-overexpression CHO-K1 cell line clone 2 was purchased from PerkinElmer.

Cells were grown in a 10 cm dish at 37 °C / 5% CO₂ in medium (F12 (Hams)/ 10% FBS/ 400 µg/ml G418) to near confluence. At that stage, cells were harvested, resuspended to 10 million/mL in culture medium without G418 and with 10% DMSO. 1 mL vial aliquots were slowly frozen to -80 °C in isopropanol and then transferred in liquid nitrogen for storage. These vials were used to run the experiment following the procedure reported in the previous paragraph, except for the compound used in the control well to define the 100% activation (Sauvagine at 100 nM) and the cell density (final cell density: 4000 cells/well)

Representative EC50 values are provided in Table 5 below:

**Table 5**

| **SEQ ID No.** | **EC50 [nM]** | **Emax (%I)** |
|---|---|---|
| 149 (CompY) | >250 | 4 |
| 150 (CompZ) | >250 | 38 |
| 151 (CompX) | 25.3 | 53 |
| 146 (Comp1) | 100 | 27 |
| 147 (Comp2) | 100 | 22 |
| 148 (Comp3) | 100 | 13 |
| 2 (Comp 4) | 95.41 | 63 |
| 5 (Comp 5) | 157.6 | 48 |
| 56 (Comp 10) | >250 | 3 |
| 116 (Comp 12) | >250 | 0 |
| 130 (Comp 13) | >250 | 4 |
| 142 (Comp 14) | >250 | 3 |
| 4 | >250 | 4 |
| 8 | 186.26 | 50 |
| 3 | >250 | 27 |
| 6 | 166.13 | 56 |
| 9 | 28.24 | 77 |
| 7 | 101.16 | 59 |
| 10 | >250 | 26 |
| 13 | >250 | 24 |
| 14 | >250 | 24 |
| 92 | >250 | 5 |
| 93 | >250 | 6 |
| 33 | >250 | 13 |
| 34 | >250 | 0 |
| 94 | >250 | 0 |
| 96 | >250 | 4 |
| 95 | >250 | 5 |
| 25 | >250 | 6 |
| 26 | >250 | 1 |
| 97 | >250 | 0 |
| 19 | >250 | 6 |
| 20 | >250 | 3 |
| 39 | >250 | 2 |
| 38 | >250 | 6 |
| 43 | >250 | 1 |
| 102 | >250 | -5 |
| 49 | >250 | -3 |
| 40 | >250 | 0 |
| 64 | >250 | 4 |
| 118 | >250 | 7 |
| 80 | >250 | 10 |
| 120 | >250 | 3 |
| 66 | >250 | 4 |
| 81 | >250 | 6 |
| 121 | >250 | 6 |
| 18 | >250 | 10 |
| 22 | >250 | 3 |
| 35 | >250 | 13 |
| 36 | >250 | 22 |
| 37 | >250 | 22 |
| 103 | >250 | 3 |
| 48 | >250 | 4 |
| 111 | >250 | -3 |
| 27 | >250 | 7 |
| 28 | >250 | 6 |
| 29 | >250 | 6 |
| 16 | 185.15 | 47 |
| 44 | >250 | 5 |
| 50 | >250 | 9 |
| 53 | >250 | 5 |
| 46 | >250 | 6 |
| 65 | >250 | 10 |
| 67 | >250 | 8 |
| 110 | >250 | 9 |
| 125 | >250 | 18 |
| 119 | >250 | 5 |
| 123 | >250 | 14 |
| 124 | >250 | 14 |
| 45 | >250 | 5 |
| 100 | >250 | 8 |
| 104 | >250 | 5 |
| 105 | >250 | 10 |
| 113 | >250 | 6 |
| 122 | >250 | 5 |
| 30 | >250 | 15 |
| 31 | >250 | 14 |
| 32 | >250 | 5 |
| 42 | >250 | 1 |
| 41 | >250 | 7 |
| 106 | >250 | 6 |
| 107 | >250 | 0 |
| 108 | >250 | 4 |
| 54 | >250 | 20 |
| 55 | >250 | 5 |
| 51 | >250 | 9 |
| 52 | >250 | 7 |
| 17 | >250 | 12 |
| 21 | >250 | 5 |
| 47 | >250 | 10 |
| 57 | >250 | 24 |
| 78 | >250 | 7 |
| 79 | >250 | 10 |
| 99 | >250 | 0 |
| 112 | >250 | 3 |
| 101 | >250 | 0 |
| 98 | >250 | -2 |
| 109 | >250 | 4 |
| 68 | >250 | 8 |
| 71 | >250 | -4 |
| 72 | >250 | 18 |
| 126 | >250 | 20 |
| 127 | >250 | 11 |
| 129 | 235.62 | 39 |
| 137 | >250 | 9 |
| 69 | >250 | 3 |
| 70 | >250 | 0 |
| 128 | >250 | 10 |
| 115 | >250 | 4 |
| 114 | >250 | 6 |
| 140 | >250 | 14 |
| 139 | >250 | 13 |
| 117 | >250 | 3 |
| 138 | >250 | 10 |
| 141 | >250 | 32 |
| 23 | >250 | 35 |
| 24 | >250 | 37 |
| 58 | >250 | 6 |
| 133 | >250 | 11 |
| 59 | >250 | 9 |
| 134 | >250 | 10 |
| 60 | >250 | 5 |
| 135 | >250 | 15 |
| 61 | >250 | 3 |
| 136 | >250 | 5 |
| 74 | >250 | 7 |
| 75 | >250 | 3 |
| 152 | >250 | 25 |
| 153 | >250 | 22 |
| 154 | >250 | 24 |
| 155 | >250 | 26 |
| 156 | >250 | 8 |
| 157 | >250 | 30 |
| 158 | 98.7 | 54 |
| 159 | >250 | 4 |
| 160 | >250 | 2 |
| 161 | >250 | 3 |
| 162 | >250 | 0 |
| 163 | 189.69 | 53 |
| 164 | >250 | 14 |
| 165 | >250 | 14 |
| 166 | >250 | 5 |
| 167 | >250 | 7 |
| 168 | >250 | 3 |
| 169 | >250 | 13 |
| 170 | >250 | 5 |
| 171 | >250 | 0 |
| 172 | >250 | -6 |
| 173 | >250 | -1 |
| 174 | >250 | 11 |
| 175 | >250 | 5 |
| 176 | >250 | 8 |
| 177 | >250 | 10 |
| 178 | >250 | 1 |
| 179 | >250 | -4 |
| 180 | >250 | 2 |
| 181 | >250 | 9 |
| 182 | >250 | 1 |
| 183 | >250 | 11 |
| 184 | >250 | 3 |
| 185 | >250 | 2 |
| 186 | >250 | 2 |
| 187 | 195.24 | 37 |
| 188 | >250 | 32 |
| 189 | >250 | 9 |
| 190 | >250 | 9 |
| 191 | 92.86 | 54 |
| 192 | >250 | 19 |
| 193 | >250 | 20 |
| 194 | >250 | 10 |
| 195 | *180.45 | 42 |
| 196 | >250 | 9 |
| 197 | >250 | 5 |
| 198 | >250 | 8 |
| 199 | >250 | 11 |
| 200 | >250 | 2 |
| 201 | >250 | 5 |
| 202 | >250 | 8 |
| 203 | >250 | 3 |
| 204 | >250 | -2 |
| 205 | >250 | 1 |
| 206 | 224.24 | 42 |
| 207 | 61.36 | 65 |
| 208 | 83.93 | 63 |
| 209 | >250 | 21 |
| 210 | >250 | 35 |
| 211 | 39.91 | 73 |
| 212 | 228.55 | 41 |
| 213 | >250 | 27 |
| 214 | >250 | 34 |
| 215 | >250 | 19 |
| 216 | 80.27 | 61 |
| 217 | >250 | 33 |
| 218 | >250 | 24 |
| 219 | 222.27 | 48 |
| 220 | >250 | 32 |
| 221 | 22.53 | 79 |
| 222 | 196.44 | 50 |
| 223 | 152 | 58 |
| 224 | 39.53 | 66 |
| 225 | 73.42 | 70 |
| 226 | 42.15 | 82 |
| 227 | 1.74 | 97 |
| 228 | 90.55 | 62 |
| 229 | 202.93 | 43 |
| 230 | >250 | 30 |
| 231 | >250 | 10 |
| 232 | >250 | 13 |
| 233 | >250 | 8 |
| 234 | >250 | 15 |
| 235 | >250 | 27 |
| 236 | >250 | 7 |
| 237 | >250 | 6 |
| 238 | >250 | 12 |
| 239 | >250 | 0 |
| 240 | >250 | 4 |
| 241 | >250 | 7 |
| 242 | >250 | 6 |
| 243 | >250 | 8 |
| 244 | >250 | 8 |

### Example 13: Assessment of activity at rat CRF2 α

The A7R5 rat aortic smooth muscle cell line used in this study was purchased from ATCC.

Cells were grown in a 10 cm dish at 37 °C / 5% CO2 in medium (DMEM / 10% FBS) to near confluence. At that stage, cells were harvested, resuspended to 10 million/mL in culture medium and with 5% DMSO. 1 mL vial aliquots were slowly frozen to -80 °C in isopropanol and then transferred in liquid nitrogen for storage.

These vials were used to run the experiment following the procedure reported in the previous paragraph, except for the compound used in the control well to define the 100% activation (Sauvagine at 100 nM) and the cell density (final cell density: 5000 cells/well).

Representative EC50 values compared to comparative peptide 11 (Seq ID 85 in Table 2) are provided in Table 6 below:

**Table 6**

| **SEQ ID** | **EC₅₀ [nM]** |
|---|---|
| | **0 % HSA** |
| 85 (Comp 11) | 1.01 |
| 40 | 0.59 |
| 144 | 0.06 |
| 145 | 0.05 |
| 86 | 0,68 |
| 87 | 0.32 |
| 206 | 0.06 |
| 189 | 0.15 |
| 215 | 0.04 |
| 230 | 0.11 |
| 234 | 0.05 |
| 235 | 0.17 |
| 212 | 0.08 |
| 217 | 0.06 |
| 222 | 0.18 |
| 238 | 0.09 |
| 243 | 0.09 |
| 218 | 0.13 |
| 213 | 0.45 |
| 236 | 2.81 |
| 241 | 3.52 |
| 237 | 0.63 |
| 242 | 0.40 |
| 233 | 0.37 |
| 231 | 1.32 |
| 168 | 3.23 |
| 232 | 0.40 |
| 240 | 2.30 |
| 244 | 1.77 |

### Example 14: Assessment of solubility, chemical, catabolic stability and PK properties of the peptides according to the invention.

The effect on solubility, chemical, catabolic stability and PK properties of the peptides according to the invention were studied by means of solubility, chemical stability, plasma and SC extract stability, and pharmacokinetics studies. As comparative peptides the following peptides (Table 7) were included in the assays;

| **SEQ ID NO.** | **Comp Number** | **Amino acid sequence** | **R^{a}** |
|---|---|---|---|
| **149** | Comp Y | IVLSLDVPIKLK*KILLEQEKQKKQREQAETNKQI LAQV-NH2 | -{AEEA}₂-gGlu-C(O)(CH₂)₁₆COOH |
| **150** | Comp Z | IVLSLDvPIKLK*QILLKQERQKKQREQAEKNKQI LEQV-NH2 | -{AEEA}₂-gGlu-C(O)(CH₂)₁₆COOH |
| **151** | Comp X | IVLSLDvPIKLK*QILLKQERQKKQREQAEKNKQI LEQV-NH2 | -gGlu-C(O)(CH₂)₁₄COOH |
| Legend: v= D-Valine; K* = position of side chain derivatization | | | |

### In vitro plasma stability

*In vitro* plasma metabolic stability was studied in male Sprague Dawley rats and human. Test compounds were incubated at 3 µM for 2 hours at 37 °C. At each time point (0, 0.25, 1 and 2 h), samples were prepared for analysis by means of a single step protein precipitation technique by adding 150 µL of ethanol, 0.1% formic acid to 50 µL aliquots of plasma. Samples were mixed by vortex for homogeneity and then subjected to centrifugation at 14000 rpm for 15 min. The supernatant (100 µL) was collected, diluted with 100 µL of water, 0.1% formic acid and analyzed by LC-HRMS (TripleTOF 6600⁺, AB Sciex). For each test compound, the area ratios at each time point were compared to the 0 h area ratio and converted to a percentage remaining.

Plasma stability was initially included in the ADME screening funnel. However, all compounds showed high stability in human, rat and minipig or dog plasma some representative examples are provided in **(Table 8).** Good stability results indicate that differences in N-terminal protective group or in the linker did not affect stability.

Human, rat and minipig or dog plasma stability data of representative compounds:

**Table 8**

| **SEQ ID No.** | **Species** | **% remaining at 2 hours** |
|---|---|---|
| 118 | Human PLM | 91 |
| | Rat PLM | 128 |
| | Minipig PLM | 126 |
| 80 | Human PLM | 101 |
| | Rat PLM | 98 |
| | Minipig PLM | 104 |
| 110 | Human PLM | 97 |
| | Rat PLM | 95 |
| | Minipig PLM | 106 |
| 125 | Human PLM | 93 |
| | Rat PLM | 108 |
| | Minipig PLM | 110 |
| 51 | Human PLM | 102 |
| | Rat PLM | 73 |
| | Minipig PLM | 97 |
| 57 | Human PLM | 104 |
| | Rat PLM | 96 |
| | Minipig PLM | 81 |
| 218 | Human PLM | 99 |
| | Rat PLM | 109 |
| | Dog PLM | 102 |
| 213 | Human PLM | 99 |
| | Rat PLM | 99 |
| | Dog PLM | 102 |
| 237 | Human PLM | 83 |
| | Rat PLM | 84 |
| | Dog PLM | 86 |
| 233 | Human PLM | 100 |
| | Rat PLM | 106 |
| | Dog PLM | 99 |

### In vitro subcutaneous tissue supernatant stability and metabolite identification

*In vitro* metabolic stability in subcutaneous tissue supernatant (SCts) was studied in male Sprague Dawley rats. Test compounds were incubated at 3 mM for 2 hours at 37 °C. At each time point (0, 0.25, 1 and 2 h), samples were prepared for analysis by means of a single step protein precipitation technique by adding 150 µL of ethanol, 0.1% formic acid to 50 µL aliquots of SCts. Samples were mixed by vortex for homogeneity and then subjected to centrifugation at 14000 rpm for 15 min. The supernatant (100 µL) was collected, diluted with 100 µL of water, 0.1% formic acid and analyzed by LC-HRMS either with a TripleTOF 6600⁺ (Sciex) or Orbitrap Q Exactive (Thermo). For each test compound, the area ratios at each time point were compared to the 0 h area ratio and converted to a percentage remaining.

Software aided *in vitro* metabolite identification on SCts stability samples was performed using either Metabolite Pilot (Sciex) or Biopharma Finder (Thermo). In addition, metabolite identification using Metabolite Pilot was performed on plasma samples of Comp Y following SC administration to rat at 1 mg/kg.

Representative SCts stability data are provided in **Table 9.** The first analogs of the Comparative peptides that were tested showed a poor metabolic stability in rat SCts (< 5% remaining at 2 hours). *In vitro* soft spot identification on Comp X and Comp Y, and *in vivo* metabolite ID of Comp Y identified the N-terminus as the major metabolic soft spot both *in vitro* (rat) and *in vivo.*

Acetylation or N-methylation of Ile1 at the N-terminus resulted in a substantial improvement of the rat SCts stability (> 50% remaining at 2 hours). Finally, N-terminus stabilization translated into better SC pharmacokinetic profiles in rat (see "Pharmacokinetics" section below). All the tested compounds showed a good SCts stability in all the species (human, rat, minipig and dog) investigated confirming that N-terminus modification did improve SCts stability. Otherwise, chemical nature of the Lys12 linker (gEC16OH, gEC20OH, PEG2-gE-C20OH and PEG2-PEG2-gE-C20OH e.g.) did not significantly affect the stability of these peptides.

Representative rat SCts stability data.

**Table 9**

| **SEQ ID N** | **Species** | **% remaining at 2 h** |
|---|---|---|
| 149 (CompY) | Rat | 4 |
| 151 (CompX) | Rat | 0 |
| 40 | Rat | 79 |
| 110 | Rat | 77 |
| 125 | Rat | 79 |
| 98 | Rat | 74 |
| 199 | Rat | 83 |
| 136 | Rat | 78 |
| 144 | Rat | 74 |
| 145 | Rat | 76 |
| 82 | Rat | 82 |
| 187 | Rat | 82 |
| 188 | Rat | 77 |
| 206 | Rat | 73 |
| 219 | Rat | 71 |
| 214 | Rat | 72 |
| 161 | Rat | 59 |
| 189 | Rat | 67 |
| 190 | Rat | 95 |
| 165 | Rat | 72 |
| 215 | Rat | 84 |
| 203 | Rat | 65 |
| 193 | Rat | 56 |
| 210 | Rat | 72 |
| 234 | Rat | 68 |
| 235 | Rat | 66 |
| 121 | Rat | 77 |
| 217 | Rat | 76 |
| 222 | Rat | 62 |
| 223 | Rat | 79 |
| 218 | Rat | 71 |
| 213 | Rat | 74 |
| 236 | Rat | 75 |
| 241 | Rat | 68 |
| 237 | Rat | 60 |
| 233 | Rat | 56 |
| 231 | Rat | 65 |
| 242 | Rat | 68 |

### Solubility and chemical stability

Solubility and chemical stability were studied in 100 mM phosphate buffer (pH 7.4) and 100 mM acetate buffer (pH 4.5). Test compound powders were dissolved in both buffers at a target concentration of 10 mg/mL and incubated for 1 hour at room temperature. After centrifugation at 2500 rcf for 15 minutes, 10 µL of supernatant was diluted with 190 µL of the incubation buffer and analyzed by LC-UV (Acquity UPLC-DAD, Waters). Solubility was calculated by comparing the peak area of the test compound in the buffer sample with the peak area of the same compound dissolved at 0.5 mg/mL in water: acetonitrile 1:1, 0.1% formic acid.

From the centrifuged supernatant, two additional 80 µL aliquots were collected and diluted with 160 µL of incubation buffer. One aliquot was stored at 5 °C, while the other was stored at 40 °C. After 14 or 28 days, the samples were analyzed by LC-UV. Chemical stability was calculated as % loss using the following equations:
- Chemical stability (as % loss) = [(purity after 14 or 28 days at 5°C) - (purity after 28 days at 40°C)] x 100/ (purity after 28 days at 5°C)
- % purity= [(peak area compound) x 100/ (total peak area)]

Representative buffer stability data are provided in **Table 10** below. Solubility was generally good at both pH (data not shown), as most of the peptides showed values close to the target concentration (10 mg/mL). The peptides showed a good chemical stability at pH 4.5 but a relevant degradation at pH 7.4, probably related to isomerization of Asn31 (observed and confirmed at by high-resolution mass spectrometry). To block or reduce this degradation pathway, bulky amino acids were introduced in substitution of Lys32. In particular, the use of Aib, Abu, and 4-Pip provided a good chemical stabilization with no loss of purity observed after 2 weeks.

**Table 10**

| **SEQ ID No.** | **Buffer stability pH 7.4/4.5** |
|---|---|
| | **% purity loss** |
| 149 (CompY) | 17/- |
| 92 | 16.8/0 |
| 93 | 19/0 |
| 33 | 18.8/0 |
| 34 | 4.5/0 |
| 96 | nd/0 |
| 19 | 0/0 |
| 43 | 0/nd |
| 40 | 0/nd |
| 64 | nd/0 |
| 118 | 0/0 |
| 80 | 0/nd |
| 110 | 0/nd |
| 125 | 0/nd |
| 100 | 13.2/0 |
| 104 | 0/0 |
| 113 | 14.7/0 |
| 54 | nd/0 |
| 55 | 73.5/28.9 |
| 51 | 0/0 |
| 47 | 9.0/0 |
| 57 | 0/0 |
| 98 | 0.0/0.0 |
| 137 | Not Tested / Not Tested |
| 141 | Not Tested / Not Tested |
| 199 | 0/Not Tested |
| 135 | Not Tested / Not Tested |
| 136 | 24.3/0.0 |
| 144 | 0.0/Not Tested |
| 145 | 0.0/Not Tested |
| 82 | 0.0/Not Tested |
| 187 | 0.0/0.0 |
| 188 | 0.0/0.0 |
| 206 | 0.0/0.0 |
| 219 | -2.1/ Not Tested |
| 214 | 0.0/0.0 |
| 161 | 0.6/Not Tested |
| 189 | 0.0/Not Tested |
| 190 | 0.0/Not Tested |
| 165 | 0.0/Not Tested |
| 215 | 0.0/Not Tested |
| 203 | 0.0/0.0 |
| 193 | 0.0/Not Tested |
| 210 | 0.0/0.0 |
| 234 | -0.1/0.0 |
| 235 | 0.0/-1.6 |
| 212 | 0.3/Not Tested |
| 217 | 1.8/Not Tested |
| 222 | 0.0/Not Tested |
| 223 | -0.1/Not Tested |
| 218 | 0.0/Not Tested |
| 239 | 0.0/2.6 |
| 213 | -0.1/Not Tested |
| 236 | 3.8/Not Tested |
| 241 | 2.0/Not Tested |
| 237 | 0.0/Not Tested |
| 242 | 0.0/Not Tested |
| 233 | 0.0/Not Tested |
| 231 | 0.0/Not Tested |

### Pharmacokinetics

The pharmacokinetics of UCN-2 derivatives was studied in male Sprague Dawley rats after subcutaneous administration (SC) at 0.05, 0.1, 0.3 or 1 mg/kg. Peptides were formulated as a solution in 10 mM phosphate pH 7.4 buffer + glycerol 85% (2.3% v/v)). Plasma samples obtained from dosed animals were prepared for analysis by means of a single step protein precipitation technique by adding 200 µL of methanol, 0.1% trifluoroacetic acid to 50 µL aliquots of individual subject samples. Samples were mixed by vortex for homogeneity and then subjected to centrifugation at 16000 rpm for 15 min. The supernatant (200 µL) was analyzed by LC-MSMS. Pharmacokinetic parameters were calculated using established non-compartmental methods. The area under the plasma concentration curve *versus* time (AUC) was determined using Watson LIMS (version 7.6), with linear trapezoidal interpolation in the ascending slope and logarithmic trapezoidal interpolation in the descending slope. The portion of the AUC from the last measurable concentration to infinity was estimated from the equation, Ct/kel, where Ct represents the last measurable concentration and kel is the elimination rate constant. The latter was determined from the concentration curve *versus* time by linear regression at the terminal phase of the semi-logarithmic plot.

In line with what has been observed for the chemical stability date, improved PK properties were obtained by stabilization of the N-terminus via acetylation or N-methylation of Ile-1 or in case Ile-1 is removed via acetylation from Val-2 resulting in extended half-life in rat up to 8.6 h (Data not shown).

Further improvements in terms of catabolic stability were obtained introducing substitution of lysine-32 with bulky residues (e.i. Aib, Abu or 4-Pip). Data of some representative peptides are provided in **Table 11.** Compared to Comp Y at much lower dosage half-life significantly increased.

In addition, and consistently with the higher albumin binding generally observed in peptides with a longer fatty acid chain, C20-OH analogues exhibited a prolonged SC half-life compared to C16-OH analogues and C18-OH analogues.

**Table 11. Rat PK results of representative peptides in comparison to Comp Y.**

| **SEQ ID N** | **Dose (mg/kg)** | **Route** | **T_{1/2} (h)** | **Cmax (ng/mL)** | **Tmax (h)** | **AUClast (ng*h/mL)** |
|---|---|---|---|---|---|---|
| 149 (Comp Y) | 0.1 | IV | 4.3 | - | - | 2760 |
| | 1.0 | SC | 4 | 1045 | 2 | 14500 |
| 82 | 0.05 | SC | 1.4 | 64 | 0.75 | 221 |
| 199 | 0.05 | SC | 10.2 | 111 | 6 | 1625 |
| 187 | 0.05 | SC | 9.7 | 137 | 7 | 2100 |
| 188 | 0.05 | SC | 10.6 | 138 | 6 | 2270 |
| 189 | 0.05 | IV | 7.5 | - | - | 5035 |
| 190 | 0.05 | SC | 5.7 | 112 | 5 | 1930 |
| 203 | 0.05 | IV | 6.2 | - | - | 4865 |
| 110 | 0.05 | IV | 4.8 | - | - | 3880 |
| | 0.1 | SC | 5 | 291 | 5 | 4430 |
| 98 | 0.05 | SC | 5.8 | 159 | 5 | 2710 |
| 144 | 0.05 | SC | 12.6 | 105 | 6 | 1710 |
| 145 | 0.05 | IV | 5.8 | - | - | 4105 |
| | 0.1 | SC | NC | 295 | 8 | 4202 |
| 118 | 0.05 | IV | 5.2 | - | - | 3900 |
| | 0.1 | SC | 5.2 | 225 | 8 | 3540 |
| 193 | 0.05 | IV | 4.9 | - | - | 2340 |
| 206 | 0.05 | IV | 8 | - | - | 8105 |
| | 0.1 | SC | 8 | 197 | 8 | 5770 |
| 210 | 0.05 | IV | 8.4 | - | - | 7785 |
| 212 | 0.025 | IV | 8 | - | - | 4840 |
| | 0.05 | SC | 8 | 154 | 14 | 4685 |
| 241 | 0.05 | IV | 15 | - | - | 6210 |
| 236 | 0.05 | IV | 15 | - | - | 8250 |
| 215 | 0.05 | IV | 8 | - | - | 7320 |
| | 0.1 | SC | 10 | 325 | 7 | 9410 |
| 234 | 0.025 | IV | 7 | - | - | 2705 |
| | 0.05 | SC | 7.5 | 95 | 6 | 2730 |
| 217 | 0.025 | IV | 8 | - | - | 4120 |
| | 0.05 | SC | 12 | 138 | 8 | 4035 |
| 218 | 0.05 | IV | 11 | - | - | 6735 |
| | 0.1 | SC | NC | 115 | 24 | 4125 |
| 242 | 0.05 | IV | 14 | - | - | 7970 |
| 233 | 0.05 | IV | 9 | - | - | 4880 |
| 237 | 0.05 | IV | 11 | - | - | 4160 |
| 219 | 0.05 | IV | 6 | - | - | 5325 |
| | 0.1 | SC | 15 | 156 | 8 | 4520 |
| 222 | 0.025 | IV | 7 | - | - | 3315 |
| | 0.05 | SC | 12 | 113 | 8 | 2890 |
| 235 | 0.025 | IV | 8 | - | - | 4510 |
| 223 | 0.025 | IV | 2.8 | - | - | 1925 |
| | 0.05 | SC | 6.5 | 67 | 2 | 698 |

### Example 15: Assessment of degranulation potential of the peptides according to the invention in human LAD2 cells.

Non-allergic (anaphylactoid) reactions are acute adverse drug reactions that can exacerbate a patient's condition and produce effects that may become life-threatening. The main mechanism involves the direct stimulation of mast cells or basophils leading to the release of anaphylactic mediators such as histamine and β-hexosaminidase. A recent study by McNeil et al. (McNeil BD et al., Nature, 12, 519 (2015)) reported that MrgprX2, a specific membrane receptor on human mast cells, plays a major role in non-allergic reactions induced by small molecule drugs, including neuromuscular blocking agents and insect venom, neuropeptides and peptide therapeutics.

LAD2 (Laboratory of Allergic Diseases 2) cell is a human mast cell line commonly employed to study anaphylactic and anaphylactoid reactions, because its biological properties are identical to those of primary human mast cells, including the functional expression of the MrgprX2 receptor.

LAD2 cells are derived from CD34+ cells isolated from patient with aggressive mastocytosis, without any detectable KIT mutation (Kirshenbaum et al., Leukemia Res. 27, 677 (2003)). LAD2 cells stably express IgE receptor type 1 (FceR1), display a granular phenotype and have been reported to be more sensitive than primary human mast cells to degranulating peptides (Kulka et al., Immunology 123, 398 (2008)).

As such, the LAD2 cell line is considered as reasonably resembling normal human mast cells. LAD2 cell degranulation is quantified through the release of anaphylactic mediators such as β-hexosaminidase by fluorometric quantification.

Human LAD2 cells were obtained after Licensing Agreement with NIH and cultured following the NIH's instructions.

LAD2 cells (20000 cells/well, 96-well plates and 10,000 cells/well in 384-well plates) were incubated with compounds for 30 min. The β-hexosaminidase released into the supernatants and in cell lysates was quantified using the fluorogenic substrate 4-methylumbelliferyl-N-acetyl-b-D-glucosaminide.

On the day of the assay, the lyophilized peptides were dissolved in DMSO and/or PBS 1X pH 7.4, to obtain stock solutions. The stock solutions were used to prepare working serial dilutions to have final concentrations in assay ranging from 50, 100 or 300 to 0.1 or 0.001 µM. Each peptide was tested in biological duplicates. When compounds were dissolved in DMSO, the final solvent concentration in assay was 0.5 % (v/v).

The percentage of degranulation, expressed as % of β-hexosaminidase release, was calculated by dividing the fluorescence signal of the supernatant by the sum of the signals from the supernatant and cell pellet (lysed with Triton X-100, final 1%) and multiplying by 100.

Dose-response curves were generated by plotting the % of β-hexosaminidase release versus total release of cells treated with triton (y-axis) against the concentrations of peptides tested (x-axis). The Mast Cell Degranulation (MCD) EC50 values and standard errors were calculated using XLfit 5.5.0.5 based on the following equation:
4 Parameter Sigmoidal Model: f = (A+((B-A)/(1+((C/x)^D))))
where A=Emin, B=Emax, C=EC50 and D=slope

Potency (EC50) and % β-Hexosaminidase release at highest concentration of all peptides tested in LAD2 cells is summarized in Table 12 below.

Substance P and Icatibant were included as positive and GLP-1 as negative controls, respectively. Also the prior art peptides Comp 10 (SEQ ID No 56), Comp Y (SEQ ID No 149) and Comp X (SEQ ID No 151) were included in this study.

**Table 12. MCD data of representative peptides in comparison to Comp Y and Comp X.**

| **Cmpd** | **EC50 (µM)** |
|---|---|
| Substance P | 0.36 |
| Icatibant | 16.44 |

| **SEQ ID NO.** | |
|---|---|
| 56 (comp) | 38.5 |
| 149(comp) | 50.4 |
| 151 (comp) | 32.9 |
| 118 | >100 |
| 125 | >100 |
| 145 | >100 |
| 219 | 88.2 |
| 161 | >50 |
| 162 | >50 |
| 163 | >100 |
| 165 | >100 |
| 166 | >100 |
| 167 | >50 |
| 215 | >100 |
| 234 | 49.2 |
| 212 | >300 |
| 217 | > 300 |
| 223 | >300 |
| 238 | 36.7 |
| 243 | >300 |
| 218 | >300 |
| 213 | >300 |
| 236 | 74.1 |
| 241 | >300 |
| 237 | >300 |
| 242 | >300 |
| 233 | >300 |
| 231 | 47.5 |
| 168 | >300 |

### Example 16: Assessment of effect on blood pressure

The effect of the compounds on systolic blood pressure was determined in telemetry studies in Sprague Dawley rats at a dosage of 0.1 mg/kg SC.

The rats were previously implanted with a telemetry device (DSI, Saint-Paul, USA, HD-S10, HD-S11 or HD S21) and allowed to recover for a minimum period of 2 weeks before treatment with vehicle or the different peptides. Blood pressure (BP) was recorded through a catheter inserted into the abdominal aorta. The device body was placed in the abdomen. The animals were allowed to recover from surgery for at least 10 days before the first administration of test drug or vehicle.

The chronically instrumented rats of the telemetry studies were individually housed immediately after surgery and then housed in pairs with a non-instrumented companion after recovery.

Systolic blood pressure was recorded for one hour before treatment (basal period). Thereafter, the subcutaneous administration of the test compound or its vehicle was performed, under continuous recording of the signal up to 48 hours, analysed and expressed as % change from baseline.

Representative data are presented in Table 13 below, in which "Duration of action" indicates the last time point for which an effect on BP was still observed (n=2 animals/peptide)

**Table 13**

| | Systolic blood pressure (Mean) | | | |
|---|---|---|---|---|
| SEQ ID No | % change from baseline | | | |
| | 8h | 12h | 24h | Duration of action (h) |
| 110 | -19 | -10 | -6 | 20 |
| 100 | -22 | -12 | -9 | >24 |
| 118 | -24 | -17 | -4 | >24 |
| 113 | -31 | -24 | -7 | >24 |
| 203 | -18 | -11 | 2 | 23 |
| 125 | -25 | -16 | 1 | 22 |
| 189 | -27 | -25 | -13 | >24 |
| 145 | -23 | -21 | -14 | >24 |
| 144 | -22 | -17 | 1 | 23 |
| 193 | -20 | -12 | -8 | >24 |
| 194 | -13 | -3 | 18 | 19 |
| 233 | 1 | -8 | -11 | >24 |
| 218 | -13 | -17 | -16 | >24 |
| 213 | -10 | -18 | -13 | >24 |
| 231 | -3 | -3 | -12 | >24 |
| 206 | -12 | -16 | -13 | >24 |
| 219 | -10 | -13 | -10 | >24 |
| 215 | -12 | -14 | -16 | >24 |
| 209 | -18 | -11 | 3 | 23 |
| 220 | -17 | -14 | -11 | >24 |
| 229 | -12 | -9 | -5 | >24 |
| 210 | -13 | -13 | -6 | >24 |
| 230 | -15 | -16 | 3 | 23 |
| 235 | -8 | -7 | -12 | >24 |
| 10 | -24 | -11 | 2 | 18 |
| 33 | -22 | -11 | 16 | 17 |
| 19 | -21 | 0 | 10 | 11 |
| 43 | -18 | -1 | 13 | 12 |
| 40 | -30 | -18 | 7 | 23 |
| 64 | -25 | -13 | 3 | 23 |
| 55 | -31 | -23 | 0 | 23 |
| | | | -4 | |
| 51 | -27 | -19 | -1 | 24 |
| 52 | -28 | -17 | 4 | 23 |
| 47 | -14 | -6 | 2 | 23 |
| 57 | -34 | -25 | -2 | >24 |
| 82 | -33 | -4 | 11 | 14 |
| 165 | -30 | -20 | 2 | 23 |
| 158 | -11 | -7 | 3 | 23 |

All sequences disclosed herein are listed in Table 2 and the appended sequence listing with the albumin binding moieties shown in Table 2, the entire content of which forms a part of this specification.

All publications, patent applications, patents, and other references mentioned herein are incorporated by reference in their entirety. In addition, the materials, methods, and examples given are illustrative only and not intended to be limiting. Unless otherwise defined, all technical and scientific terms used herein have the same meaning as commonly understood by one of ordinary skill in the art to which this invention belongs. It is to be understood that while the disclosure has been described in conjunction with the detailed description thereof, the foregoing description is intended to illustrate and not limit the scope of the invention, which is defined by the scope of the appended claims. Other aspects, advantages, and modifications are within the scope of the claims.

## Claims

1. A compound which is a peptide comprising the amino acid sequence of formula (I):
X1-X2-X3-X4-X5-X6-X7-X8-X9-X10-X11-X12-X13-X14-X15-X16-X17-X18-X19-X20- X21-X22-X23-X24-X25-X26-X27-X28-X29-X30-X31-X32-X33-X34-X35-X36-X37-X38 (I)
Wherein
X1 is absent or a N-methylated or acetylated isoleucine (I)
X2 is an acylated Valine (V) when X1 is absent;
X3 is leucine (L)
X4 is serine (S)
X5 is leucine (L)
X6 is aspartate (D);
X7 is D-valine (v);
X8 is proline (P)
X9 is isoleucine (I)
X10 is lysine (K), alanine (A)or glycine (G);
X11 is leucine (L); isoleucine (I), alpha-methyl-leucine (αMeL) or 2-aminoisobutyric acid (Aib);
X12 is lysine (K), wherein the epsilon-amino group of the lysine side chain is covalently bound to an albumin-binding moiety (herein also denoted as (K*));
X13 is lysine (K) , glutamine (Q) or alanine (A);
X14 is isoleucine (I) or 2-aminoisobutyric acid (Aib);
X15 is leucine (L), alpha-methyl-leucine (αMeL) or 2-aminoisobutyric acid (Aib);
X16 is leucine (L)
X17 is glutamate (E) or lysine (K);
X18 is glutamine (Q)
X19 is glutamate (E)
X20 is lysine (K), arginine (R) or alanine (A);
X21 is glutamine (Q)
X22 is lysine (K) or alanine (A);
X23 is lysine (K), alanine (A), glutamate (E) or 2-aminoisobutyric acid (Aib);
X24 is glutamine (Q)
X25 is arginine (R), alanine (A), alpha-methyl-leucine (αMeL) or 2-aminoisobutyric acid (Aib);
X26 is glutamate (E), or 2-aminoisobutyric acid (Aib);
X27 is glutamine (Q)
X28 is alanine (A)
X29 is glutamate (E)
X30 is lysine (K) or threonine (T);
X31 asparagine (N), glutamine (Q) or alanine (A);
X32 is lysine (K), valine (V), threonine (T), 2-aminoisobutyric acid (Aib), glutamate (E), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx);
X33 is glutamine (Q)
X34 is isoleucine (I)
X35 is leucine (L)
X36 is alanine (A) or glutamate (E);
X37 is glutamine (Q)
X38 is an amidated valine (V)
and wherein at least one of X11, X15, X23, X25, X31 and X32 is selected as;
• X11 being alpha-methyl-leucine (αMeL) or 2-aminoisobutyric acid (Aib); in particular alpha-methyl-leucine (αMeL);
• X15 being alpha-methyl-leucine (αMeL) or 2-aminoisobutyric acid (Aib);
• X23 being alanine (A), glutamate (E) or 2-aminoisobutyric acid (Aib);
• X25 being alpha-methyl-leucine (αMeL) or 2-aminoisobutyric acid (Aib); in particular alpha-methyl-leucine (αMeL);
• X31 being glutamine (Q); and
• X32 being glutamate (E), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); in particular X32 being selected from 2-aminoisobutyric acid (Aib), 2-aminobutyric acid (Abu); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); and 1-aminocyclohexanecarboxylic acid (Chx).

2. The compound of claim 1, wherein:
X1 is absent or an N-terminally acetylated or N-methylated isoleucine (I);
X2 is valine (V), wherein when X1 is absent said valine is N-terminally acylated with an alkanoyl group selected from ethanoyl, methanoyl, propanoyl, butanoyl, pentanoyl, hexanoyl or heptanoyl; more in particular N- terminally acylated with a pentanoyl group ;
X3 is leucine (L)
X4 is serine (S)
X5 is leucine (L)
X6 is aspartate (D);
X7 is D-valine (v);
X8 is proline (P)
X9 is isoleucine (I);
X10 is lysine (K);
X11 is leucine (L) or alpha-methyl-leucine (αMeL);
X12 is lysine (K), wherein the epsilon-amino group of the lysine side chain is covalently bound to an albumin-binding moiety (herein also denoted as (K*));
X13 is lysine (K);
X14 is isoleucine (I);
X15 is leucine (L);
X16 is leucine (L);
X17 is glutamate (E);
X18 is glutamine (Q);
X19 is glutamate (E);
X20 is lysine (K);
X21 is glutamine (Q);
X22 is lysine (K)
X23 is lysine (K), alanine (A), glutamate (E) or 2-aminoisobutyric acid (Aib);
X24 is glutamine (Q);
X25 is arginine (R);
X26 is glutamate (E);
X27 is glutamine (Q);
X28 is alanine (A);
X29 is glutamate (E);
X30 is threonine (T);
X31 is asparagine (N);
X32 is lysine (K), 2-aminoisobutyric acid (Aib), glutamate (E), alanine (A), 2-aminobutyric acid (Abu); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); or 1-aminocyclopropanecarboxylic acid (Cpx);
X33 is glutamine (Q);
X34 is isoleucine (I)
X35 is leucine (L)
X36 is alanine (A) or glutamate (E); in particular alanine (A);
X37 is glutamine (Q);
X38 is valine (V);
wherein;
when X1 is an N-terminally acetylated or N-methylated isoleucine (I); then X32 is selected from 2-aminoisobutyric acid (Aib), 2-aminobutyric acid (Abu); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); or 1-aminocyclopropanecarboxylic acid (Cpx); in particular X32 is then 2-aminoisobutyric acid (Aib), when X1 is an N-terminally acetylated or N-methylated isoleucine (I); and
wherein the albumin binding moiety is selected from the following groups:
-{AEEA}2-gGlu-C(O)(CH2)16COOH;
-{AEEA}2-gGlu-C(O)(CH2)18COOH;
-{AEEA}-gGlu-C(O)(CH2)16COOH;
-{AEEA}-gGlu-C(O)(CH2)18COOH;
-gGlu-C(O)(CH2)14COOH;
-gGlu-C(O)(CH2)16COOH;
-gGlu-C(O)(CH2)18COOH;
and
-{gGlu}2-C(O)(CH2)18COOH.
or a pharmaceutically acceptable salt thereof.

3. The compound of claim 1 or claim 2, wherein at least at least one of X10, X11, X13, X15, X20, X22, X23, X25, X31 and X32 is selected as;
• X10 being alanine (A)or glycine (G);
• X11 being alpha-methyl-leucine (αMeL);
• X13 being glutamine (Q) or alanine (A);
• X15 being alpha-methyl-leucine (αMeL) or 2-aminoisobutyric acid (Aib);
• X20 being arginine (R) or alanine (A);
• X22 being alanine (A);
• X23 being alanine (A), glutamate (E) or 2-aminoisobutyric acid (Aib);
• X25 being alpha-methyl-leucine (αMeL);
• X31 being glutamine (Q) and
• X32 being selected from 2-aminoisobutyric acid (Aib), 2-aminobutyric acid (Abu); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); and 1-aminocyclohexanecarboxylic acid (Chx).

4. The compound of claim 1, wherein:
X1 is absent or an N-terminally acetylated or N-methylated isoleucine (I);
X2 is valine (V), wherein when X1 is absent said valine is N-terminally acylated with an alkanoyl group selected from ethanoyl, methanoyl, propanoyl, butanoyl, pentanoyl hexanoyl or heptanoyl; more in particular N- terminally alkylated with a pentanoyl group ;
X7 is D-valine (v)
X9 is isoleucine (I);
X10 is lysine (K);
X11 is leucine (L) or alpha-methyl-leucine (αMeL);
X14 is isoleucine (I);
X15 is leucine (L);
X20 is lysine (K);
X23 is lysine (K);
X25 is arginine (R);
X26 is glutamate (E);
X31 is asparagine (N);
X32 is glutamate (E), 2-aminoisobutyric acid (Aib), or 4-Amino-piperidine-4-carboxylic acid (4-Pip); and wherein
when X1 is an N-terminally acetylated or N-methylated isoleucine (I), X32 is selected from 2-aminoisobutyric acid (Aib), or 4-Amino-piperidine-4-carboxylic acid (4-Pip)

5. The compound of claim 1, wherein:
X1 is absent;
X2 is valine (V) N-terminally acylated with a group selected from a propanoyl group, a butanoyl group, a pentanoyl group or an isopropanoyl group and
X32 is selected from glutamate (E), valine (V), threonine (T), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); in particular X32 is selected from 2-aminoisobutyric acid (Aib), 2-aminobutyric acid (Abu); 4-amino-tetrahydro-2H-pyran-4-yl-acetic acid (ThP) or 4-Amino-piperidine-4-carboxylic acid (4-Pip); more in particular X32 is selected from from 2-aminoisobutyric acid (Aib) or 4-Amino-piperidine-4-carboxylic acid (4-Pip)..

6. The compound of claim 1, wherein the compound is a peptide of the amino acid sequence of formula (I), wherein when X1 is absent, X2 is valine with valine in L or D configuration, in particular valine in L configuration (V); with said valine being N-terminally acylated with a C1-20alkanoyl; more in particular said valine being N-terminally acylated with a C1-10alkanoyl; even more in particular said valine being N-terminally acylated with an alkanoyl group selected from ethanoyl, methanoyl, propanoyl, butanoyl, pentanoyl hexanoyl, heptanoyl, decanoyl, dodecanoyl, or tetradecanoyl; more in particular selected from ethanoyl, methanoyl, propanoyl, butanoyl, pentanoyl hexanoyl or heptanoyl.

7. The compound of claim 1, wherein the compound is a peptide of the amino acid sequence of formula (I), wherein when X1 is absent, X2 is valine with valine in L or D configuration, in particular valine in L configuration (V); with said valine being N-terminally acylated; and X32 is selected from valine (V), threonine (T), 2-aminoisobutyric acid (Aib), alanine (A), 2-aminobutyric acid (Abu); norvaline (Nva); α-methyl-lysine (αMeK); 2,3-diaminopropionic acid (Dap); 4-Amino-piperidine-4-carboxylic acid (4-Pip); 4-amino-tetrahydro-2H-pyran-4-yl-acetic acid (ThP); 1-aminocyclohexanecarboxylic acid (Chx); 1-aminocyclopentane-1-carboxylic acid (Cpex); or 1-aminocyclopropanecarboxylic acid (Cpx); in particular X32 is selected from 2-aminoisobutyric acid (Aib), 2-aminobutyric acid (Abu); 4-amino-tetrahydro-2H-pyran-4-yl-acetic acid (ThP) or 4-Amino-piperidine-4-carboxylic acid (4-Pip); more in particular X32 is selected from 2-aminoisobutyric acid (Aib) or 4-Amino-piperidine-4-carboxylic acid (4-Pip).

8. The compound of claim 1, wherein the albumin-binding moiety is a group of the formula (II):
-Y-Z-C(O)R¹ (II)
wherein
Y is AEEA, {AEEA}₂, {AEEA}s, Gly, {Gly}₂, {Gly}₃, N-MeGly, {N-MeGly}₂, {N-MeGly}s or absent, wherein AEEA denotes [2-(2-aminoethoxy)ethoxy]-acetyl;
Z is gGlu, {gGlu}₂ or absent; and
R¹ is -(CH₂)ₓCOOH or -(CH₂)ₓCH₃, wherein x is an integer from 10 to 22.

9. The compound of claim 8, wherein the albumin-binding moiety is selected from the following groups:
-{AEEA}₂-gGlu-C(O)(CH₂)₁₆COOH;
-{AEEA}₂-gGlu-C(O)(CH₂)₁₈COOH;
-{AEEA}-gGlu-C(O)(CH₂)₁₆COOH;
-{AEEA}-gGlu-C(O)(CH₂)₁₈COOH;
-gGlu-C(O)(CH₂)₁₄COOH;
-gGlu-C(O)(CH₂)₁₆COOH;
-gGlu-C(O)(CH₂)₁₈COOH;
-{gGlu}₂-C(O)(CH₂)₁₆COOH;
and
-{gGlu}₂-C(O)(CH₂)₁₈COOH.

10. The compound of claim 1, wherein the compound is a peptide of any one of SEQ ID NOs 40, 43, 47, 51, 54, 55, 57, 64, 80, 98, 100, 104, 110, 113, 118, 125, 131, 132, 136, 144, 145, 189, 190, 193, 203, 206, 210, 212, 213, 215, 217, 218, 219, 222, 230, 231, 233, 234, 235, 236, 237, 238, 241, 242, 243 or a pharmaceutically acceptable salt thereof.

11. A pharmaceutical composition comprising a compound of any one of the preceding claims and a pharmaceutically acceptable excipient, diluent or carrier.

12. A compound of any one of claims 1 to 10 or a pharmaceutical composition of claim 11, for use in therapy.

13. A compound of any one of claims 1 to 10 or a pharmaceutical composition of claim 11, for use in a method of treating or preventing a cardiovascular diseases, obesity, diabetes, sarcopenia, particularly obesity-linked sarcopenia, cachexia, heart failure and pulmonary hypertension in a patient.

14. A compound of any one of claims 1 to 10 or a pharmaceutical composition of claim 11, for use as an agonist of the corticotropin-releasing factor receptor 2 (CRF2).
